(19) Europäisches Patentamt
European Patent Office
Office européen des brevets

(11) EP 1 891 234 B1

(12) # EUROPEAN PATENT SPECIFICATION

(45) Date of publication and mention of the grant of the patent:
**10.12.2014 Bulletin 2014/50**

(21) Application number: **06763457.6**

(22) Date of filing: **01.06.2006**

(51) Int Cl.:
*C12Q 1/68* *(2006.01)* *G01N 33/50* *(2006.01)*

(86) International application number:
**PCT/EP2006/062835**

(87) International publication number:
**WO 2006/128902 (07.12.2006 Gazette 2006/49)**

(54) **USE OF SLC39A12 PROTEINS AS TARGET IN DIAGNOSIS AND DRUG SCREENING IN ALZHEIMER'S DISEASE**

VERWENDUNG VON SLC39A12-PROTEINEN ALS ZIELMOLEKÜLE IN DER DIAGNOSE VON UND BEIM SCREENING VON MEDIKAMENTEN ZUR BEHANDLUNG VON ALZHEIMER-KRANKHEIT

L'UTILISATION DES PROTEINES SLC39A12 EN TANT QUE CIBLES DIAGNOSTIQUES ET DANS LE CRIBLAGE POUR LA MALADIE D'ALZHEIMER

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.06.2005 EP 05104753**
**01.06.2005 US 686058 P**

(43) Date of publication of application:
**27.02.2008 Bulletin 2008/09**

(73) Proprietor: **Evotec International GmbH**
**22419 Hamburg (DE)**

(72) Inventors:
- **POHLNER, Johannes**
  **22175 Hamburg (DE)**
- **VON DER KAMMER, Heinz**
  **22607 Hamburg (DE)**

(74) Representative: **Von Kreisler Selting Werner - Partnerschaft**
**von Patentanwälten und Rechtsanwälten mbB**
**Deichmannhaus am Dom**
**Bahnhofsvorplatz 1**
**50667 Köln (DE)**

(56) References cited:
**EP-A- 1 293 569** **WO-A-01/57275**
**WO-A-02/26982** **WO-A-2004/023973**

- **EIDE DAVID J: "The SLC39 family of metal ion transporters." PFLUGERS ARCHIV : EUROPEAN JOURNAL OF PHYSIOLOGY. FEB 2004, vol. 447, no. 5, February 2004 (2004-02), pages 796-800, XP002346535 ISSN: 0031-6768**
- **FREDERICKSON CHRISTOPHER J ET AL: "The neurobiology of zinc in health and disease." NATURE REVIEWS. NEUROSCIENCE. JUN 2005, vol. 6, no. 6, June 2005 (2005-06), pages 449-462, XP009054406 ISSN: 1471-003X**
- **DATABASE UniProt [Online] EBI; 5 June 2005 (2005-06-05), XP002396845 retrieved from SRS Database accession no. Q504Y0**
- **BLY ET AL: "Examination of the zinc transporter gene, SLC39A12" SCHIZOPHRENIA RESEARCH, ELSEVIER, vol. 81, no. 2-3, 31 January 2006 (2006-01-31), pages 321-322, XP005255529 ISSN: 0920-9964**
- **KATSEL PAVEL L ET AL: "Large-scale microarray studies of gene expression in multiple regions of the brain in schizophrenia and Alzheimer's disease", INTERNATIONAL REVIEW OF NEUROBIOLOGY, ACADEMIC PRESS, LONDON, GB, vol. 63, 1 January 2005 (2005-01-01), pages 41-82, XP009110801, ISSN: 0074-7742, DOI: DOI:10.1016/S0074-7742(05) 63003-6**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- **E. M. Blalock: "Incipient Alzheimer's disease: Microarray correlation analyses reveal major transcriptional and tumor suppressor responses", Proceedings of the National Academy of Sciences, vol. 101, no. 7, 9 February 2004 (2004-02-09), pages 2173-2178, XP055073328, ISSN: 0027-8424, DOI: 10.1073/pnas.0308512100**
- **Laura Shaughnessy ET AL: "Novel Approaches to Models of Alzheimer's Disease Pathology for Drug Screening and Development", Journal of Molecular Neuroscience, vol. 24, no. 1, 1 January 2004 (2004-01-01), pages 023-032, XP055073346, ISSN: 0895-8696, DOI: 10.1385/JMN:24:1:023**


Remarks:

The file contains technical information submitted after the application was filed and not included in this specification

## Description

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70 % of all dementia cases, and it is probably the most devastating age-related neurodegenerative condition affecting about 10 % of the population over 65 years of age and up to 45 % over age 85 (Vickers et al., Progress in Neurobiology 2000, 60: 139-165; Walsh and Selkoe, Neuron 2004, 44:181-193). Presently, this amounts to an estimated 12 million cases in the US, Europe, and Japan. This situation will inevitably worsen with the demographic increase in the number of old people in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β protein evolves from the cleavage of the amyloid precursor protein (APP) by different kinds of proteases (Selkoe and Kopan, Annu Rev Neurosci 2003, 26:565-597; Ling et al., Int J Biochem Cell Biol 2003, 35:1505-1535). Two types of plaques, diffuse plaques and neuritic plaques can be detected in the brain of AD patients. They are primarily found in the cerebral cortex and hippocampus. The generation of toxic Aß deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, J Neural Transm 1998, 53: 127-140). NFTs emerge inside neurons and consist of chemically altered tau, which forms paired helical filaments (PHF) twisted around each other. Characteristically for AD the microtubule associated protein tau aggregating in paired helical filaments displays the abnormal phosphorylation of certain amino acid positions, including among others Ser214. The pattern of tau phosphorylation seems to correlate with the loss of neuronal integrity, and along the formation of NFTs, a loss of neurons can be observed (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376). AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the inferior temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92).

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NYAcad Sci 1998, 855: 738-43). Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers.

WO 2004/023973 discloses purified human polynucteotides, among others a SLC39A12 sequence, for diagnostics and therapeutics (dithp). Also encompassed are the polypeptides (DITHP) encoded by dithp. Also disclosed is the use of dithp, or complements, oligonucleotides, or fragments thereof in diagnostic assays. Further disclosed are vectors and host cells containing ditph for the expression of DITHP and additionally the use of isolated and purified DITHP to induce antibodies and to screen libraries of compounds and the use of anti-DITHP antibodies in diagnostic assays. Also disclosed are microarrays containing dithp and methods of use.

EP 1293569 A discloses full-length cDNAs, among others a SLC39A12 sequence. 1639 cDNA derived from human have been isolated. The full-length nucleotide sequences of the cDNA and amino acid sequences encoded by the nucleotide sequences have been determined. Because the cDNA of the present invention are full-length and contain the translation start site, they provide information useful for analyzing the functions of the polypeptide.

Pavel L. Katsel et al. assesses in International Review of Neurobiology, Volume 63, 2005, Pages 41-82, Large-Scale Microarray Studies of Gene Expression in Multiple Regions of the Brain in Schizophrenia and Alzheimer's Disease, the status of microarray technology and data mining strategies as they relate to the analysis of postmortem brain with a focus on schizophrenia (SZ), Alzheimer's disease (AD), and tissue- and donor-quality requirements.

Eric M. Blalock et al report in 2173-2178, doi: 10.1073/pnas.0308512100 that the pathogenesis of incipient Alzheimer's disease (AD) has been resistant to analysis because of the complexity of AD and the overlap of its early-stage markers with normal aging. Analyzed was hippocampal gene expression of nine control and 22 AD subjects of varying severity on 31 separate microarrays and the correlation of each gene's expression with MiniMental Status Examination (MMSE) and neurofibrillary tangle (NFT) scores across all 31 subjects regardless of diagnosis. These well powered tests revealed a major transcriptional response comprising thousands of genes significantly correlated with AD markers. Several hundred of these genes were also correlated with AD markers across only control and incipient AD subjects (MMSE > 20). Laura Shaughnessy et al. disclose in Journal of Molecular Neuroscience, 1-Aug-2004, Volume 24, Issue 1, pp 23-32, novel approaches to models of Alzheimer's disease pathology for drug screening and development. AD research has benefited significantly from the use of genetically engineered cell lines expressing components of the amyloid-generating pathway, as well as from the study of transgenic mice that develop the pathological hallmarks of the disease, mainly neuritic plaques. A universal and efficient gene-delivery system, using lentiviral vectors, that permits the development of relevant cell biological systems using neuronal cells, including primary neurons and animal models in mammalian species best suited for the study of AD is described.

It is therefore an object of the present invention to provide insight into the pathogenesis of neurological diseases and to provide methods, which are suited inter alia for the diagnosis and development of a treatment of these diseases. This object has been solved by the features of the independent claims. The subclaims define preferred embodiments of the present invention.

The present invention is based on the finding of the association of the metal ion transporter solute carrier family 39 member 12, SLC39A12, with neurodegenerative diseases, in particular Alzheimer's disease. Besides a number of biological functions throughout the whole human body zinc plays an important role in the function of neurons either by modulating protein function or as ionic signal (Frederickson et al., Nature Rev Neurosci. 2005, AOP, doi: 10.1038/nrn1671). Specifically synaptic release of $Zn^{2+}$ neurons is described to be synchronistic with the release of glutamate. Therefore, this specific subpopulation of neurons, mainly located with their cell bodies within the cerebral cortex and the limbic structures, is also termed as "gluzinerg" (Slomianka et al., Neuroscience 1990, 38:843-854; Frederickson & Bush, BioMetals 2001, 14:353-366). Active and/or passive transport of zinc required for the regulation of zinc homeostasis is accomplished by zinc transporters which are members of the solute carrier (SLC) gene series (Liuzzi and Cousins, Annu. Rev. Nutr. 2004, 24:151-172). All together the SLC family comprises 43 different SLC transporter subfamilies and covers about 300 different human transporter genes which include passive transporters, ion coupled transporters and exchangers (Hediger et al., Plugers Arch. 2004, 447:465-468). All zinc transporters have transmembrane domains and belong to two different SLC families (SLC30 and SLC39) depending on specific amino acid homology (Eide, Plugers Arch. 2004, 447:796-800).

In human there are at least 9 zinc transporters known as part of the SLC30 proteins (alias ZnT family) and 15 zinc transporters as part of the SLC39 proteins (alias Zip family). Most of the Zip proteins have eight transmembrane domains with extracellular or intravesicular amino and carboxy termini. Their common feature is to have a very short carboxy terminus and a long loop region between transmembrane domain 3 and 4 which harbours a conserved histidine-rich portion $(HX)_{n=3-6}$. The knowledge about structure and function of the Zip proteins is mainly based on studies on the human Zip transporters hZip1-4, whereas information about the exact transport type and the substrates for most of the Zip proteins are mainly derived from sequence homology studies (Liuzzi and Cousins, Annu. Rev. Nutr. 2004, 24:151-172).

The protein of the SLC39A12 gene belongs to the LIV-1 subfamily of ZIP zinc transporters (LZT subfamily). In contrast to other ZIP transporters LZT proteins have an up to sevenfold higher incidence of histidine-rich repeats over the whole sequence, and a unique motif (HEXPHEXGD) with conserved proline and glutamic acid residues, which is unprecedented in other zinc transporters. In addition they have a long N terminus, and conserved transmembrane domains. The restricted set within the consensus sequence present in zinc and PDF metalloproteases, together with the localisation of LZT proteins to lamellipodiae mirrors cellular location of membrane-type matrix metalloproteases. Evidences are described that LZT proteins situated on the plasma membrane of cells can function as zinc influx transporters (Taylor and Nicholson, Biochim. Biophys. Acta 2003, 1611:16-30).

The SLC39A12 (LZT-Hs8, FLJ30499) gene which is located on human chromosome 10p12.33 consists of 12 exons. It is transcribed into an mRNA transcript of 3130 base pairs length. The coding region consists of 2076 base pairs encoding a 690 amino acid long protein with a calculated molecular weight of 76.5 kDa (BC035118; Strausberg et al., Proc. Natl. Acad. Sci. U.S.A. 2002, 99:16899-16903). Among other splice variants, one SLC39A12 protein variant is annotated which consists of 654 amino acids with a calculated molecular weight of 73 kDa (AK055061; Hubbard et al., Ensembl 2005, Nucleic Acids Res. 2005, 33; Database issue:D447-53). According to an electronic Northern analysis of the NCBI's UniGene dataset SLC39A12 mRNA seems to be exclusively expressed in brain tissue whereas all other members of the LZT protein family show ubiquitous expression or tissue-specific expression in tissues different from brain. This is confirmed by Taylor and Nicholson (Biochim. Biophys. Acta 2003, 1611:16-30) who described an expression only in the central nervous system. A relation of SLC39A12 with neurodegenerative diseases, in particular Alzheimer's disease has not been disclosed so far.

Brief description of the drawings

Figure 1 discloses the identification of differences in the levels of SLC39A12 gene derived mRNA in human brain tissue samples from individuals corresponding to different Braak stages as measured and compared by GeneChip analyses. It indicates that the levels of the respective mRNA species correlate quantitatively with AD progression and thus are indicative for AD as measured by the neuropathological staging of brain tissue samples according to Braak and Braak (Braak staging).

Figure 2 lists the data for the verification of differences in the levels of SLC39A12 gene derived mRNA in human brain tissue samples from individuals corresponding to different Braak stages indicative for AD as measured by quantitative RT-PCR analysis.

Figure 3 shows the analysis of absolute levels of SLC39A12 gene derived mRNA in human brain tissue samples from individuals corresponding to different Braak stages indicative for AD as measured by quantitative RT-PCR and using statistical method of the median at 98%-confidence level.

Figure 4A discloses SEQ ID NO: 1, the amino acid sequence of the human SLC39A12 splice variant 1 protein.

Figure 4B discloses SEQ ID NO: 2, the amino acid sequence of the human SLC39A12 splice variant 2 protein.

Figure 4C discloses SEQ ID NO: 3, the amino acid sequence of the human SLC39A12 splice variant 3 protein.

Figure 5A shows SEQ ID NO: 4, the nucleotide sequence of the human SLC39A12 splice variant 1 cDNA.

Figure 5B shows SEQ ID NO: 5, the nucleotide sequence of the human SLC39A12 splice variant 2 cDNA.

Figure 5C shows SEQ ID NO: 6, the nucleotide sequence of the human SLC39A12 splice variant 3 DNA.

Figure 6A depicts SEQ ID NO: 7, the coding sequence (cds) of the human SLC39A12 splice variant 1.

Figure 6B depicts SEQ ID NO: 8, the coding sequence (cds) of the human SLC39A12 splice variant 2.

Figure 6C depicts SEQ ID NO: 9, the coding sequence (cds) of the human SLC39A12 splice variant 3.

Figure 7 depicts the sequence alignment of the primers used for SLC39A12 transcription level profiling by quantitative RT-PCR with the corresponding clippings of SLC39A12 cDNA.

Figure 8 schematically charts the alignment of the SLC39A12 cDNA sequence, the coding sequence and both primer sequences used for SLC39A12 transcription level profiling.

Figure 9 shows an immunoblot (Western blot) analysis of the affinity-purified polyclonal rabbit anti-SLC39A12 antiserum HKQ1.

Figure 10 shows an immunofluorescense analysis of the affinity-purified polyclonal rabbit anti-SLC39A12 antiserum HKQ1.

Figures 11 A exemplifies the increase in the level of SLC39A12 protein in brain cerebral cortex tissue samples from AD patients (Braak 4-6) when compared to the levels observed in respective samples from age-matched controls (Braak 1-3) which have not been diagnosed to suffer from AD signs and symptoms.

Figures 11 B exemplifies the increase in the level of SLC39A12 protein in brain cerebral white matter tissue samples from AD patients (Braak 4 and 5) when compared to the levels observed in respective samples from age-matched controls (Braak 0 and 2) which have not been diagnosed to suffer from AD signs and symptoms.

Figure 12 shows detection of SLC39A12 protein expressed by three independent SLC39A12 transgenic fly lines under the control of gmr-GAL4 by Western blots analysis.

Figure 13 shows a comparison of SLC39A12 mRNA level expressed by three independent SLC39A12 transgenic fly lines under the control of gmr-GAL4 by RT-PCR using SLC39A12 specific primers.

Figure 14 shows that co-expression of SLC39A12 in TauP301 L transgenic Drosophila results in a significant and dosage dependent increase in the phosphorylation of Ser214 of mutant tau protein in comparison to control flies (TauP301L).

Figure 15 shows SLC39A12-expression in H4-neuroglioma cells stably expressing the Swedish Mutant APP by Western blot analysis.

Figure 16 shows SLC39A12 expression in H4-neuroglioma cells stably expressing the Swedish Mutant APP by immunofluorescence analysis.

Figure 17 shows the verification of the function of SLC39A12 as zinc transporter by using a zinc uptake assay. SLC39A12, when overexpressed in H4-cells, causes an increase in the intracellular concentration of zinc. This cell based assay provides a means for the identification of small molecule based modulators of SLC39A12,.

The singular forms "a", "an", and "the" as used herein and in the claims include plural reference unless the context dictates otherwise. For example, "a cell" means as well a plurality of cells, and so forth.
The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined.
The term "level" as used herein is meant to comprise a gage of, or a measure of the amount of, or a concentration of a substance such as a transcription product, for instance an mRNA, or a translation product, for instance a protein or polypeptide.
The term "activity" as used herein shall be understood as a measure for the ability of a substance, such as transcription product or a translation product to produce a biological effect or a measure for a level of biologically active molecules. The term "activity" also refers to biological activity and/or pharmacological activity which refer to binding, antagonization, repression, blocking, neutralization or sequestration of a transporter or transporter subunit and which refers to activation, agonization, and up-regulation of a transporter or transporter subunit.
The terms "level" and/or "activity" as used herein further refer to gene expression levels or gene activity. Gene expression can be defined as the utilization of the information contained in a gene by transcription and translation leading to the production of a gene product.
"Dysregulation" shall mean an up-regulation or down-regulation of gene expression and/or an increase or decrease in the stability of the gene products. A gene product comprises either RNA or protein and is the result of expression of a gene. The amount of a gene product can be used to measure how active a gene is and how stable its gene products are.
The term "gene" as used in the present specification and in the claims comprises both coding regions (exons) as well as non-coding regions (e.g. non-coding regulatory elements such as promoters or enhancers, introns, leader and trailer sequences).
The term "ORF" is an acronym for "open reading frame" and refers to a nucleic acid sequence that does not possess a stop codon in at least one reading frame and therefore can potentially be translated into a sequence of amino acids.
"Regulatory elements" shall comprise inducible and non-inducible promoters, enhancers, operators, and other elements that drive and regulate gene expression.
The term "fragment" as used herein is meant to comprise e.g. an alternatively spliced, or truncated, or otherwise cleaved transcription product or translation product. For example, the proteins having SEQ ID NO: 1, SEQ ID NO: 2 and SEQ ID NO: 3 are translation products of the gene coding for SLC39A12 proteins and constitute splice variants.
The term "derivative" as used herein refers to a mutant, or an RNA-edited, on a chemically modified, or otherwise altered transcription product, or to a mutant, or chemically modified, or otherwise altered translation product. For the purpose of clarity, a derivative transcript, for instance, refers to a transcript having alterations in the nucleic acid sequence such as single or multiple nucleotide deletions, insertions, or exchanges. A derivative translation product, for instance, may be generated by processes such as altered phosphorylation, or glycosylation, or acetylation, or lipidation, or by altered signal peptide cleavage or other types of maturation cleavage. These processes may occur post-translationally.
The term "modulator" as used in the present invention and in the claims refers to a molecule capable of changing or altering the level and/or the activity of a gene, or a transcription product of a gene, or a translation product of a gene. A "modulator" refers to a molecule which has the capacity to either enhance or inhibit, thus to "modulate" a functional property of a protein, to "modulate" binding, antagonization, repression, blocking, neutralization or sequestration, activation, agonization and up-regulation. "Modulation" will be also used to refer to the capacity to affect the biological activity of a cell. Preferably, a "modulator" is capable of changing or altering the biological activity of a transcription product or a translation product of a gene. Said modulation, for instance, may be an increase or a decrease in the biological activity and/or pharmacological activity, a change in binding characteristics, or any other change or alteration in the biological, functional, or immunological properties of said translation product of a gene.

The terms "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes.

The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample.

As used herein, "homolog or homology" is a term used in the art to describe the relatedness of a nucleotide or peptide sequence to another nucleotide or peptide sequence, which is determined by the degree of identity and/or similarity between said sequences compared.

In the art, the terms "identity" and "similarity" mean the degree of polypeptide or polynucleotide sequence relatedness which are determined by matching a query sequence and other sequences of preferably the same type (nucleic acid or protein sequence) with each other. Preferred computer program methods to calculate and determine "identity" and "similarity" include, but are not limited to GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acids Res. 1984, 12: 387), BLASTN 2.0 (Gish W., http://blast.wustl.edu, 1996-2002), FASTA (Pearson and Lipman, Proc. Natl. Acad. Sci. USA 1988, 85: 2444-2448), and GCG GelMerge which determines and aligns a pair of contigs with the longest overlap (Wilbur and Lipman, SIAM J. Appl. Math. 1984, 44: 557-567; Needleman and Wunsch, J. Mol. Biol. 1970, 48: 443-453).

The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention, but retains its essential properties. Furthermore the term "variant" as used herein refers to any mRNA, in reference to gene transcripts disclosed in the present invention, in which one or more nucleotides are added and/or substituted and/or deleted.

Furthermore, the term "variant" shall include any shorter or longer version of a polypeptide or protein. "Variants" shall also comprise a sequence that has at least about 80 % sequence identity, more preferably at least about 85 % sequence identity, and most preferably at least about 90 % sequence identity over a length of at least 200 amino acids of SLC39A12 proteins having SEQ ID NO: 1, or SEQ ID NO:2, or SEQ ID NO:3. "Variants" also include, for example, proteins with conservative amino acid substitutions in highly conservative regions.

Furthermore, the term "variant" shall include any shorter or longer version of a gene transcript. "Variants" shall also comprise a sequence that has at least about 80 % sequence identity, more preferably at least about 85 % sequence identity, and most preferably at least about 90 % sequence identity over a length of at least 600 nucleotides of SLC39A12 gene transcripts having SEQ ID NO: 4, or SEQ ID NO: 5, or SEQ ID NO: 6. Sequence variations shall be included wherein a codon is replaced with another codon due to alternative base sequences, but the amino acid sequence translated by the DNA sequence remains unchanged. This known in the art phenomenon is called redundancy of the set of codons which translate specific amino acids.

"Proteins and polypeptides" include variants, fragments and chemical derivatives of the proteins comprising the amino acid sequences of SLC39A12 proteins having SEQ ID NO: 1, or SEQ ID NO: 2, or SEQ ID NO: 3. included shall be such exchange of amino acids which would have no effect on functionality, such as arginine for lysine, valine for leucine, asparagine for glutamine. Proteins and polypeptides can be included which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants.

The term "isolated" as used herein is considered to refer to molecules or substances which have been changed and/or that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state and such molecules can be produced by recombinant and/or synthetic means, it is also said that they are "non-native". Even if for said purposes those sequences may be introduced into living or non-living organism by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.

The term "AD" shall mean Alzheimer's disease. "AD-type neuropathology", "AD pathology" as used herein refers to neuropathological, neurophysiological, histopathological and clinical hallmarks, signs and symptoms as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998).

The term "Braak stage" or "Braak staging" refers to the classification of brains according to the criteria proposed by Braak and Braak (Braak and Braak, Acta Neuropathology 1991, 82: 239-259). Braak staging of AD rates the extent and distribution of neurofibrillary pathology in determined regions of the forebrain and divides the neuropathologic progression of AD into six stages (stage 0 to 6). It is a well established and universally accepted procedure in post-mortem neuropathological staging of AD. It has convincingly been shown that there is a significant correlation between an AD patient's clinical condition with respect to mental status and cognitive function/impairment and the corresponding Braak stage obtained after autopsy (Bancher et al., Neuroscience Letters 1993, 162:179-182; Gold et al., Acta Neuropathol 2000, 99: 579-582). Likewise, a correlation between neurofibrillary changes and neuronal cellular pathology has been found (Rössler et al., Acta Neuropathol 2002, 103:363-369), and both have been reported to predict cognitive function (Giannakopoulos et al., Neurology 2003, 60:1495-1500; Bennett et al., Arch Neurol 2004, 61:378-384). Moreover, a pathogenic cascade has been proposed that involves the deposition of beta-amyloid peptide and finally cumulates in the formation of neurofibrillary tangles, the latter thus witnessing the precedence of earlier AD-specific events at the molecular/cellular level (Metsaars et al., Neurobiol Aging 2003, 24:563-572).
In the instant invention, Braak stages are therefore used as a surrogate marker of disease progression independent of the clinical presentation/condition of the individual donor, i.e. independent of the presence or absence of reported mental illness, cognitive deficits, decline in other neuropsychiatric parameters, or the overt clinical diagnosis of AD. I.e. it is presumed that the neurofibrillary changes on which the Braak staging reflect the underlying molecular and cellular pathomechanisms in general and hence define a (pre-)morbid condition of the brain, meaning that e.g. a donor staged Braak 1 represents by definition an earlier stage of molecular/cellular pathogenesis than a donor staged 2 (or higher), and that therefore a donor of Braak stage 1 can e.g. be regarded as a control individual when compared to donors of any higher Braak stage. In this regard, the differentiation between control individual and affected individual may not necessarily be the same as the clinical diagnosis based differentiation between healthy control donor and AD patient, but it rather refers to a presumed difference in the (pre-) morbid status as deduced from and mirrored by a surrogate marker, the Braak stage.

In the instant invention Braak stage 0 may represent persons which are not considered to suffer from Alzheimer's disease signs and symptoms, and Braak stages 1 to 4 may represent either healthy control individuals or AD patients depending on whether said individuals were suffering already from clinical signs and symptoms of AD. The higher the Braak stage the more likely is the possibility to display signs and symptoms of AD or the risk to develop signs and symptoms of AD. For a neuropathological assessment, i.e. an estimation of the probability that pathological changes of AD are the underlying cause of dementia, a recommendation is given by Braak H. (www.alzforum.org).

The values obtained from controls are the reference values representing a known health status and the values obtained from patients are the reference values representing a known disease status.

Neurodegenerative diseases or disorders comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, ischemic stroke, age-related macular degeneration, narcolepsy, motor neuron diseases, prion diseases, traumatic nerve injury and repair, and multiple sclerosis.

The present invention discloses the identification, the differential expression, the differential regulation, a dysregulation of a gene of the solute carrier family, the metal ion transporter solute carrier family 39 member 12, also named SLC39A12, and of the protein products of said gene SLC39A12, in specific samples, in specific brain regions of AD patients, in specific brain regions of individuals grouped into different Braak stages, in comparison with each other and/or in comparison to age-matched control individuals. The present invention discloses that the gene expression for SLC39A12 is varied, is dysregulated in brains of AD patients as compared to the respective brain regions of control individuals, in that SLC39A12 mRNA levels are increased, are up-regulated in the inferior temporal cortex and in the frontal cortex of AD patients. Further, the present invention discloses that the SLC39A12 expression differs in specific brain regions of individuals grouped into different Braak stages with an increase in expression level starting already at early Braak stages (Braak 1-3) and with a progressive increase with the course of late Braak stages (Braak 4-6) predominantly in the inferior temporal cortex.

The differences observed at the SLC39A12 gene transcriptional level, when compared between AD patients and control individuals but also between the different Braak stages, are further supported by substantial differences that can be found at the SLC39A12 protein level. In comparison to the control individuals, in brain specimens from AD patients the levels of SLC39A12 protein are increased substantially. This dysregulation of the SLC39A12 gene expression and the changes in levels and localization of the corresponding gene products which parallels the development of AD-type pathology clearly reflects a link between SLC39A12 and AD and is indicative for the progressive pathological events in the course of the disease. Further evidence for this link is provided by the finding that in transgenic Drosophila co-expression of the SLC39A12 gene and P301L, a mutant form of the tau protein, results in an increase of the phosphorylation of Ser214, an amino acid position in tau that has been demonstrated to be abnormally phosphorylated in AD. Therefore SLC39A12 may be causally involved in the cascade of molecular pathological events leading to AD and therefore may represent a promising target for the identification and development of small molecule based therapeutics for AD and other neurodegenerative diseases.

To date, no experiments have been described that demonstrate a relationship between the dysregulation of SLC39A12 gene expression and the pathology of neurodegenerative diseases, in particular AD. Likewise, no mutations in the SLC39A12 gene have been described to be associated with said diseases. Linking the SLC39A12 gene to such diseases offers new ways, inter alia, for the diagnosis and treatment of said diseases. Additionally, linking SLC39A12 to pathological events occurring already early in the course of AD provides the possibility of a treatment which will prevent the initiation of AD pathology, a treatment which will be applied before non-repairable damages of the brain occur. Consequently, the disclosed methods have utility for diagnostic evaluation, for diagnostic monitoring of persons undergoing a treatment, for prognosis as well as for the identification of a predisposition to a neurodegenerative disease, in particular AD.

The present invention discloses a dysregulation of a gene coding for SLC39A12 and of its gene products in specific brain regions of AD patients. Neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions and display a selective vulnerability to neuronal loss and degeneration in AD. In contrast, neurons within the frontal cortex, the occipital cortex, and the cerebellum remain largely intact and preserved from neurodegenerative processes. Brain tissues from the frontal cortex (F) and the inferior temporal cortex (T) of AD patients and of age-matched controls were used for the herein disclosed examples. Consequently, the SLC39A12 gene and its corresponding transcription and/or translation products play a causative role, and/or have an influence on the selective neuronal degeneration and/or neuroprotection.

In one aspect, the invention features a method of diagnosing or prognosticating a neurodegenerative disease in a subject, or of determining whether a subject has a predisposition of developing said disease. The method comprises: determining a level, or an activity, or both said level, and said activity of (i) a transcription product of a gene coding for SLC39A12 proteins, and/or of (ii) a translation product of a gene coding for SLC39A12 proteins, in a sample obtained from said subject and comparing said level, and/or said activity of said transcription product and/or said translation product to a reference value representing a known disease status (patient) and/or to a reference value representing a known health status (control), and/or to a reference value representing a known Braak stage and analysing whether said level and/or said activity is varied, is altered compared to a reference value representing a known health status, and/or is similar or equal to a reference value representing a known disease status and/or is similar compared to a reference value representing a known Braak stage which is an indication that said subject has a neurodegenerative disease, or that said subject is at increased risk of developing said disease, thereby diagnosing or prognosticating said neurodegenerative disease in said subject, or determining whether said subject is at increased risk of developing said neurodegenerative disease. The wording "in a subject" refers to results of the methods disclosed as far as they relate to a disease afflicting a subject, that is to say, said disease being "in" a subject.

In a further aspect, the invention discloses a method of monitoring the progression of a neurodegenerative disease in a subject. A level, expression or an activity, or both said level, expression and said activity, of (i) a transcription product of a gene coding for SLC39A12 proteins, and/or of (ii) a translation product of a gene coding for SLC39A12 proteins, and/or of (iii) a fragment, or derivative, or variant of said transcription or translation product in a sample obtained from said subject is determined. Said level, expression and/or said activity are compared to a reference value representing a known disease or ) health status or a known Braak stage. Thereby, the progression of said neurodegenerative disease in said subject is monitored.

In still a further aspect, the invention discloses a method of evaluating a treatment or monitoring the effect of a treatment for a neurodegenerative disease, comprising determining a level, expression or an activity, or both said level, expression and said activity of (i) a transcription product of a gene coding for SLC39A12 proteins, and/or of (ii) a translation product of a gene coding for SLC39A12 proteins, and/or of (iii) a fragment, or derivative, or variant of said transcription or translation product in a sample obtained from a subject being treated for said disease. Said level, expression or said activity, or both said level, expression and said activity are compared to a reference value representing a known disease or health status or a known Braak stage, thereby evaluating the treatment for said neurodegenerative disease.

Disclosed is that the level, expression or the activity, or both said level and said activity of (i) a transcription product of a gene coding for SLC39A12 proteins, and/or of (ii) a translation product of a gene coding for SLC39A12 proteins, and/or of (iii) a fragment, or derivative, or variant of said transcription or translation product in a series of samples taken from said subject over a period of time is compared, in order to monitor the progression of said disease. Said subject receives a treatment prior to one or more of said sample gatherings. In particular, said level and/or activity is determined before and after said treatment of said subject.

Said level, the expression and/or said activity of said transcription product and/or said translation product of SLC39A12 and of its fragments, derivatives, or variants, is increased, is up-regulated in samples obtained from AD patients as compared to samples obtained from persons not suffering from AD, control persons. For example, the expression and/or activity of the transcription product and/or the translation product of SLC39A12 and of its fragments, derivatives, or variants is measured from samples of patients and compared with the expression and/or activity of the transcription product and/or the translation product of SLC39A12 and of its fragments, derivatives, or variants in a sample of a healthy control subject (reference sample).

In the herein claimed methods, and uses of the instant invention, said SLC39A12 gene codes for proteins having SEQ ID NO: 1 (splice variant 1 (sv1), Genbank/Ensembl accession number BC094700/ENST00000377369), or SEQ ID NO: 2 (splice variant 2 (sv2), Genbank/Ensembl accession number Q5VWV9,BC035118/ENST00000377371), or SEQ ID NO: 3 (splice variant 3 (sv3), Genbank/Ensembl accession number Q5VWV8/ ENST00000277633). The amino acid sequences of said splice variants are deduced from the mRNA sequences of SEQ ID NO: 4 which correspond to the cDNA sequence of Ensembl ID ENST00000377369, of SEQ ID NO: 5 which correspond to the cDNA sequence of Ensembl ID ENST00000377371, and of SEQ ID NO: 6 which correspond to the cDNA sequence of Ensembl ID ENST00000277633 respectively. SLC39A12 also refers to the nucleic acid sequences SEQ ID NO: 7, SEQ ID NO: 8, and SEQ ID NO: 9 representing the coding sequences (cds) of human SLC39A12 splice variants. In the instant invention said sequences are "isolated" as the term is employed herein. Further, in the instant invention, the gene coding for said SLC39A12 proteins (splice variants sv1, sv2, and sv3) is also generally referred to as the SLC39A12 gene or simply SLC39A12. The proteins (splice variants sv1, sv2, and sv3) of SLC39A12 are also generally referred to as the SLC39A12 proteins, SLC39A12 splice variants or simply SLC39A12.

In the herein claimed methods, and uses of the instant invention, said neurodegenerative disease or disorder is Alzheimer's disease, and said subjects suffer from signs and symptoms of Alzheimer's disease.

It is preferred that the sample to be analyzed and determined is selected from the group comprising brain tissue or other tissues, or body cells. The sample can also comprise cerebrospinal fluid or other body fluids including saliva, urine, stool, blood, serum plasma, or mucus. The methods of diagnosis, prognosis or monitoring the progression for a neurodegenerative disease, according to the instant invention, are practiced *ex corpore,* and such methods relate to samples, for instance, body fluids or cells, removed, collected, or isolated from a subject or patient or a control person.

In further preferred embodiments, said reference value' is that of a level, or of an activity, or both of said level and said activity of (i) a transcription product of the gene coding for SLC39A12 proteins, and/or of (ii) a translation product of the gene coding for SLC39A12 proteins, in a sample obtained from a subject not suffering from said neurodegenerative disease (control sample, control, healthy control person) or in a sample obtained from a subject suffering from a neurodegenerative disease, in particular Alzheimer's disease (patient sample, patient, AD sample) or from a person with a defined Braak stage which may suffer or may not suffer from signs and symptoms of AD.

In preferred embodiments, an alteration in the level and/or activity of a transcription product of the gene coding for SLC39A12 proteins and/or of a translation product of the gene coding for SLC39A12 proteins in a sample cell, or tissue, or body fluid taken from said subject relative to a reference value representing a known health status (control sample) indicates a diagnosis, or prognosis, or increased risk of becoming diseased with AD.

In a further preferred embodiment, an equal or similar level and/or activity of a transcription product of the gene coding for SLC39A12 proteins and/or of a translation product of the gene coding for SLC39A12 proteins in a sample cell, or tissue, or body fluid obtained from a subject relative to a reference value representing a known disease status of Alzheimer's disease (AD patient sample), indicates a diagnosis, or prognosis, or increased risk of becoming diseased with said neurodegenerative disease.

In another further preferred embodiment, an equal or similar level, and/or activity of a transcription product of the gene coding for SLC39A12 proteins and/or of a translation product of the gene coding for SLC39A12 proteins in a sample cell, or tissue, or body fluid obtained from a subject relative to a reference value representing a known Braak stage which Braak stage reflects a high risk of developing signs and symptoms of AD, indicates a diagnosis, or prognosis, or an increased risk of becoming diseased with AD.

It is preferred however that said varied, altered level, and/or said altered activity of said transcription product and/or said translation product of SLC39A12, is an increase, an up-regulation.

In preferred embodiments, measurement of the level of transcription products of the gene coding for SLC39A12 proteins is performed in a sample obtained from a subject using a quantitative PCR-analysis with primer combinations to amplify said gene specific sequences from cDNA obtained by reverse transcription of RNA extracted from a sample of a subject. Primer combinations (SEQ ID NO: 10, SEQ ID NO: 11) are given in Example 1 (vi) of the instant invention, but also other primers generated from the sequences as disclosed in the instant invention can be used. A Northern blot or a ribonuclease protection assay (RPA) with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO02/14543.

The construction and the use of primers and probes which are unique to the nucleic acid sequences, or fragments, or variants thereof, are disclosed in the present invention. The oligonucleotide primers and/or probes can be labeled specifically with fluorescent, bioluminescent, magnetic, or radioactive substances. The disclosure relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects or subjects with defined Braak stages. Whether an amplification results in a specific nucleic acid product or not, and whether a fragment of different length can be obtained or not, may be indicative for a neurodegenerative disease, in particular Alzheimer's disease. Thus, disclosed are nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit. Primers for SLC39A12 are exemplarily described in Example 1 (vi).

Furthermore, a level and/or an activity and/or expression of a translation product of the gene coding for SLC39A12 proteins and/or of a fragment, or derivative, or variant of said translation product, and/or the level or activity of said translation product, and/or of a fragment, or derivative, or variant thereof, can be detected using an immunoassay, an activity assay, e.g. a cellular zinc uptake assay, an assay measuring tau aggregation and/or phosphorylation, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In another aspect, the invention features the use of a kit for diagnosing or prognosticating AD, in a subject, or determining the propensity or predisposition of a subject to develop AD, said kit comprising:

(a) at least one reagent which is selected from the group consisting of (i) reagents that selectively detect a transcription product of the gene coding for SLC39A12 proteins (ii) reagents that selectively detect a translation product of the gene coding for SLC39A12 proteins; as defined in claim 2.

The kit, may be particularly useful for the identification of individuals that are at risk of developing AD. Reagents that selectively detect a transcription product and/or a translation product of the gene coding for SLC39A12 proteins, preferably coding for the splice variants having SEQ ID NO: 1,or having SEQ ID NO: 2, or having SEQ ID NO: 3 can be sequences of various length, fragments of sequences, antibodies, aptamers, siRNA, microRNA, ribozymes. Such reagents may be used also to detect fragments, derivatives or variants thereof.

Consequently, the kit used according to the present invention, may serve as a means for targeting identified dividuals for early preventive measures or therapeutic intervention prior to disease onset, before irreversible damage in the course of the disease has been inflicted. Furthermore, the kit is useful for monitoring a progression of a neurodegenerative disease, in particul AD in a subject, as well as monitoring success or failure of therapeutic treatment for such a disease of said subject.

The invention discloses a method of treating or preventing a neurodegenerative disease, in particular AD, in a subject comprising the administration to said subject in need of such a treatment in a therapeutically or prophylactically effective amount and formulation an agent, agents, modulators, antagonist, agonists or antibodies which directly or indirectly affect a level, or an activity, or both said level and said activity, of (i) the gene coding for SLC39A12 proteins, and/or (ii) a transcription product of the gene coding for SLC39A12 proteins, and/or (iii) a translation product of the gene coding for SLC39A12 proteins, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence of a translation product of the gene coding for SLC39A12 proteins, or a fragment, or derivative, or a variant thereof. An agent for treating or preventing a neurodegenerative disease, in particular AD, may also consist of a nucleotide, an oligonucleotide, or a polynucleotide. Said oligonucleotide or polynucleotide may comprise a nucleotide sequence of the gene coding for SLC39A12 proteins, either in sense orientation or in antisense orientation.

The method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA co-precipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

In particular, the invention disclose a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechno! 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). In preferred embodiments, the subject to be treated is a human, and therapeutic antisense nucleic acids or derivatives thereof are directed against transcription products of the gene coding for SLC39A12 proteins. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligo-deoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418: 244-251).

The method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection (see Mc Celland and Pardee, Expression Genetics: Accelerated and High-Throughput Methods, Eaton Publishing, Natick, MA, 1999).

Said agent for treating and preventing a neurodegenerative disease, in particular AD, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

Methods of treatment or prevention, comprise the application of therapeutic cloning, transplantation, and stem cell therapy using embryonic stem cells or embryonic germ cells and neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Nuclei of those cells are transplanted into unfertilized egg cells from which the genetic material has been removed. Embryonic stem cells are isolated from the blastocyst stage of the cells which underwent somatic cell nuclear transfer. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42) Adult neural stem cells are of great potential for repairing damaged or diseased brain tissues, as the germinal centers of the adult brain are free of neuronal damage or dysfunction (Colman A, Drug Discovery World 2001, 7: 66-71).

The subject for treatment or prevention, can be a human, or a non-human experimental animal, e.g. a mouse or a rat, a domestic animal, or a non-human primate. The experimental animal can be an animal model for a neurodegenerative disorder, e.g. a transgenic mouse and/or a knock-out mouse with an AD-type neuropathology.

The invention discloses an agent, an antagonist, agonist or a modulator of an activity, or a level, or both said activity and said level, and/or of expression of at least one substance which is selected from the group consisting of (i) the gene coding for SLC39A12 proteins, and/or (ii) a transcription product of the gene coding for SLC39A12 proteins, and/or (iii) a translation product of the gene coding for SLC39A12 proteins, and/or (iv) a fragment, or derivative, or variant of (i) to (iii), and said agent, antagonist or agonist, or said modulator has a potential activity in the treatment of neurodegenerative diseases, in particular AD.

Further, the invention discloses the use of an agent, an antibody, an antagonist or agonist, or a modulator of an activity, or a level, or both said activity and said level, and/or of expression of at least one substance which is selected from the group consisting of (i) the gene coding for SLC39A12 proteins, and/or (ii) a transcription product of the gene coding for SLC39A12 proteins, and/or (iii) a translation product of the gene coding for SLC39A12 proteins, and/or (iv) a fragment, or derivative, or variant of (i) to (iii) in the manufacture of a medicament for treating or preventing a neurodegenerative disease, in particular AD. Said antibody may be specifically immunoreactive with an immunogen which is a translation product of a gene coding for SLC39A12 (preferably having SEQ ID NO: 1, or SEQ ID NO: 2, or SEQ ID NO: 3.) or a fragment, derivative or variant of such translation product.

Additionally, the invention discloses a pharmaceutical composition comprising said agent, antibody, antagonist or agonist, or modulator and preferably a pharmaceutical carrier. Said carrier refers to a diluent, adjuvant, excipient, or vehicle with which the modulator is administered. The present invention also discloses a kit comprising one or more containers filled with a therapeutically or prophylactically effective amount of said pharmaceutical composition.

In a further aspect, the invention discloses a recombinant, genetically modified non-human animal comprising a non-native SLC39A12 gene sequence coding for a SLC39A12 protein (preferably having SEQ ID NO: 1, SEQ ID NO: 2, or SEQ ID NO: 3), or a fragment, or a derivative, or variant thereof under the control of a transcriptional element which is not the native SLC39A12 gene transcriptional control element. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said gene sequence, wherein the expression of said gene, a mis-expression, under-expression, non-expression or over-expression, and wherein the disruption or alteration of said gene sequence results in said non-human animal exhibiting a predisposition to developing signs and symptoms of a neurodegenerative disease, in particular AD. Strategies and techniques for the generation and construction of such an animal are known to those of ordinary skill in the art (see e.g. Capecchi, Science 1989, 244: 1288-1292 and Hogan et al., Manipulating the Mouse Embryo: A Laboratory Manual, Cold Spring Harbor Laboratory Press, Cold Spring Harbor, New York, 1994 and Jackson and Abbott, Mouse Genetics and Transgenics: A Practical Approach, Oxford University Press, Oxford, England, 1999).

It is preferred to make use of such a genetically modified, recombinant non-human animal as an animal model, as test animal or as a control animal for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease. The use of such a genetically modified animal in a screening method is disclosed in the instant invention.

In a further aspect the invention discloses use of a cell, in which a gene sequence coding for a SLC39A12 protein (preferably having SEQ ID NO: 1, or SEQ ID NO: 2 or SEQ ID NO: 3), or a fragment, or derivative, or variant thereof is mis-expressed, under-expressed, non-expressed or over-expressed, or disrupted or in another way alterated for screening, testing and validating compounds, agents and modulators in the development of diagnostics and therapeutics to treat neurodegenerative diseases, in particular Alzheimer's disease. The use of such a cell in a screening method is disclosed in the instant invention.

In another aspect, the invention discloses a method of screening for an agent, a modulator, an antagonist or agonist for use in the treatment of neurodegenerative diseases, in particular AD, or related diseases and disorders, which agents, modulators, antagonists or agonists have an ability to alter expression and/or level and/or activity of one or more substances selected from the group consisting of (i) the gene coding for SLC39A12 protein (preferably having SEQ ID NO: 1, or SEQ ID NO: 2, or SEQ ID NO: 3), and/or (ii) a transcription product of the gene coding for SLC39A12 protein (preferably having SEQ ID NO: 1, or SEQ I,D NO: 2, or SEQ ID NO: 3), and/or (iii) a translation product of the gene coding for SLC39A12 protein (preferably having SEQ ID NO: 1, or SEQ ID NO: 2, or SEQ ID NO: 3), and/or (iv) a fragment, or derivative, or variant of (i) to (iii). This screening method comprises (a) contacting a cell with a test compound, and (b) measuring the activity and/or the level, or both the activity and the level, and/or the expression of one or more substances recited in (i) to (iv), and (c) measuring the activity and/or the level, or both the activity and the level and/or the expression of said substances in a control cell not contacted with said test compound, and (d) comparing the levels and/or activities and/or the expression of the substance in the cells of step (b) and (c), wherein an alteration in the activity and/or level and/or expression of said substances in the contacted cells indicates that the test compound is an agent, modulator, antagonist or agonist for use in the treatment of neurodegenerative diseases and disorders. Said cells may be cells as disclosed in the instant invention.

In one further aspect, the invention features a method of screening for an agent, a modulator, an antagonist or agonist for use in the treatment of AD, which agents, modulators, antagonists or agonists have an ability to alter level and/or activity of one or more substances selected from the group consisting of (i) a transcription product of the gene coding for SLC39A12 protein having SEQ ID NO: 1, or SEQ ID NO: 2, or SEQ ID NO: 3, and/or (iii) a translation product of the gene coding for SLC39A12 protein having SEQ ID NO: 1, or SEQ ID NO: 2, or SEQ ID NO: 3, comprising (a) administering a test compound to a non-human test animal which is predisposed to developing or has already developed signs and symptoms of AD, said animal may be an animal model as disclosed in the instant invention, and (b) measuring the activity and/or level of one or more substances recited in (i) to (ii) and (c) measuring the activity and/or level of said substances in a non-human control animal which is equally predisposed to developing or has already developed said signs and symptoms of AD, and to which non-human animal no such test compound has been administered, and (d) comparing the activity and/or level of the substances in the animals of step (b) and (c), wherein an alteration in the activity and/or level of substances in the non-human test animal indicates that the test compound is an agent, modulator, antagonist or agonist for use in the treatment of AD. The present invention discloses a method for producing a medicament comprising the steps of (i) identifying an agent, modulator, antagonists or agonists of neurodegenerative diseases by a method of the aforementioned screening assays and (ii) admixing said agent, modulator, antagonist or agonist with a pharmaceutical carrier. However, said agent, modulator, antagonist or agonist may also be identifiable by other types of screening methods and assays.

Further, the present invention discloses for an assay for testing a compound or compounds, preferably for screening a plurality of compounds in high-throughput format, to determine the degree of inhibition of binding or the enhancement of binding between a ligand and SLC39A12 protein (preferably having SEQ ID NO: 1, or SEQ ID NO: 2, or SEQ ID NO: 3), or a fragment, or derivative, or variant thereof and/or to determine the degree of binding of said compounds to SLC39A12 protein (preferably having SEQ ID NO: 1, or SEQ ID NO: 2, or SEQ ID NO: 3), or a fragment, or derivative, or variant thereof. For determination of inhibition of binding between a ligand and SLC39A12 protein, or a fragment, or derivative, or variant thereof, said screening assay comprises the steps of (i) adding a liquid suspension of said SLC39A12 protein, or a fragment, or derivative, or variant thereof, to a plurality of containers, and (ii) adding a compound or a plurality of compounds to be screened for said inhibition to said plurality of containers, and (iii) adding a detectable, preferably a fluorescently labelled ligand to said containers, and (iv) incubating said SLC39A12 protein, or said fragment, or derivative or variant thereof, and said compound or plurality of compounds, and said detectable, preferably fluorescently labelled ligand, and (v) measuring the amounts of ligand, preferably its fluorescence, associated with said SLC39A12 protein, or with said fragment, or derivative, or variant thereof, and (vi) determining the degree of inhibition by one or more of said compounds of binding of said ligand to said SLC39A12 protein, or said fragment, or derivative, or variant thereof. It might be preferred to reconstitute said SLC39A12 translation product, or fragment, or derivative, or variant thereof into artificial liposomes to generate the corresponding proteoliposomes to determine the inhibition of binding between a ligand and said SLC39A12 translation product. Methods of reconstitution of SLC39A12 translation products from detergent into liposomes have been detailed (Schwarz et al., Biochemistry 1999, 38: 9456-9464; Krivosheev and Usanov, Biochemistry-Moscow 1997, 62: 1064-1073). Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of the gene coding for SLC39A12 protein, or a fragment, or derivative, or variant thereof. One example of a fluorescent binding assay, in this case based on the use of carrier particles, is disclosed and described in patent application WO00/52451, A further example is the competitive assay method as described in patent WO02/01226. Preferred signal detection methods for screening assays of the instant invention are described in the following patent applications: WO96/13744, WO98/16814, WO98/23942, WO99/17086, WO99/34195, WO00/66985, WO01/59436, and WO01/59416.
Further discloses is a method for producing a medicament comprising the steps of (i) identifying a compound as an inhibitor of binding between a ligand and a gene product of the gene coding for SLC39A12 proteins by the aforementioned inhibitory binding assay and (ii) admixing the compound with a pharmaceutical carrier. However, said compound may also be identifiable by other types of screening assays.

Furthermore, the present invention discloses an assay for testing a compound or compounds, preferably for screening a plurality of compounds in high-throughput format to determine the degree of binding of said compounds to SLC39A12 protein (preferably having SEQ ID NO: 1, or SEQ ID NO: 2, or SEQ ID NO: 3), or to a fragment, or derivative, or variant thereof, said screening assay comprises (i) adding a liquid suspension of said SLC39A12 protein, or a fragment, or derivative, or variant thereof, to a plurality of containers, and (ii) adding a detectable, preferably a fluorescently labelled compound or a plurality of detectable, preferably fluorescently labelled compounds to be screened for said binding to said plurality of containers, and (iii) incubating said SLC39A12 protein, or said fragment, or derivative, or variant thereof, and said detectable, preferably fluorescently labelled compound or detectable, preferably fluorescently labelled compounds, and (iv) measuring the amounts of compound, preferably its fluorescence, associated with said SLC39A12 protein, or with said fragment, or derivative, or variant thereof, and (v) determining the degree of binding by one or more of said compounds to said SLC39A12 protein, or said fragment, or derivative, or variant thereof. In this type of assay it might be preferred to use a fluorescent label. However, any other type of detectable label might also be employed. Also in this type of assay it might be preferred to reconstitute a SLC39A12 translation product or a fragment, or derivative, or variant thereof into artificial liposomes as described in the present invention. Said assay methods may be useful for the identification of novel compounds as well as for evaluating compounds which have been improved or otherwise optimized in their ability to bind to SLC39A12 protein, or a fragment, or derivative, or variant thereof.
Further, the present invention discloses a method for producing a medicament comprising the steps of (i) identifying a compound as a binder to a gene product of the gene coding for SLC39A12 proteins by the aforementioned binding assays and (ii) admixing the compound with a pharmaceutical carrier. However, said compound may also be identifiable by other types of screening assays.

In another embodiment, the present invention discloses a medicament obtainable by any of the methods according to the herein claimed screening assays. In one further embodiment, the instant invention discloses a medicament obtained by any of the methods according to the herein claimed screening assays.

In general, the aforementioned assay and screening methods as well as potential drug molecules (e.g. agents, modulators, antagonists, agonists) identified therefrom have applicability in relation to the treatment or prevention of neurodegenerative diseases, in particular Alzheimer's disease.

Further, the present invention discloses protein molecules being translation products of the gene coding for SLC39A12 and the use of said protein molecules (preferably having SEQ ID NO: 1, or SEQ ID NO: 2, or SEQ ID NO: 3), or fragments, or derivatives, or variants thereof, as diagnostic targets for detecting a neurodegenerative disease, in particular Alzheimer's disease.

The present invention further discloses protein molecules being translation products of the gene coding for SLC39A12 and the use of said protein molecules (preferably having SEQ ID NO: 1, or SEQ ID NO: 2, or SEQ ID NO: 3), or fragments, or derivatives, or variants thereof, as screening targets for agents, modulators, antagonists, agonists, reagents or compounds preventing, or treating, or ameliorating a neurodegenerative disease, in particular Alzheimer's disease.

The present invention features the use of antibodies which are specifically immunoreactive with an immunogen, wherein said immunogen is a translation product of the SLC39A12 gene coding for SLC39A12 proteins having SEQ ID NO: 1, or SEQ ID NO: 2, or SEQ ID NO: 3 as defined in claim 9. The immunogen may comprise immunogenic of antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immunoassays (e.g. enzyme-linked immunosorbent assay, ELISA), radioimmunoassays, chemoluminescence-immunoassays, Western-blot, immunoprecipitation and antibody microarrays. These methods involve the detection of translation products of the SLC39A12 gene, or fragments, or derivatives, or variants thereof.

According to the invention, said antibodies are used for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell. The pathological state relates to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US6150173.

FIGURES:

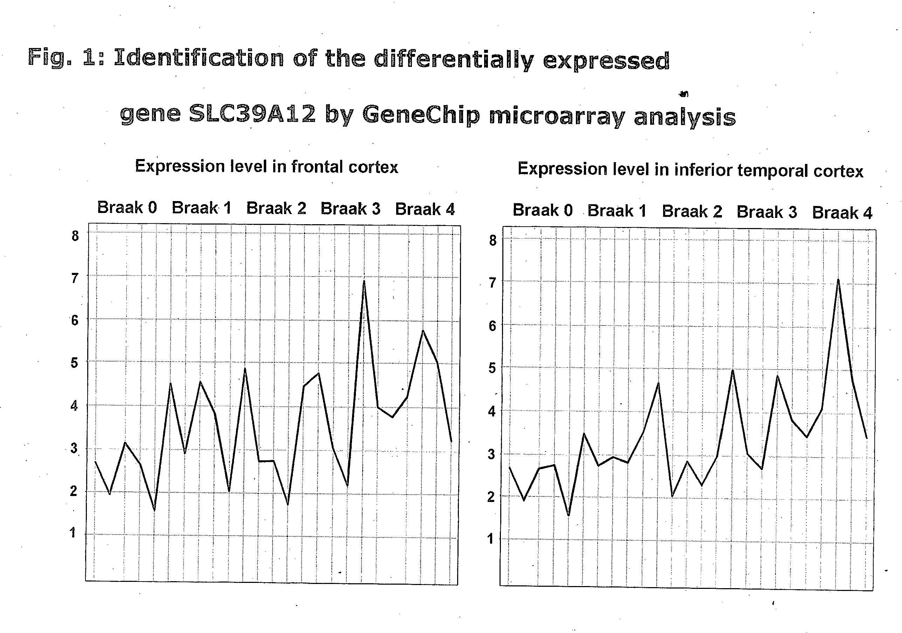

Figure 1 shows the identification of differences in the levels of SLC39A12 gene derived mRNA in human brain tissue samples from individuals corresponding to different Braak stages as measured and compared by GeneChip analyses. It indicates that the levels of the respective mRNA species correlate quantitatively with AD progression and thus are indicative for AD as measured by the neuropathological staging of brain tissue samples according to Braak and Braak (Braak staging). cRNA probes of frontal cortex as well as of inferior temporal cortex each of 5 different donors with Braak stage 0 (C011, C012, C026, C027, and C032), 7 different donors with Braak stage 1 (C014, C028, C029, C030, C036, C038, and C039), 5 different donors with Braak stage 2 (C008, C031, C033, C034, and DE03), 4 different donors with Braak stage 3 (C025, DE07, DE11, and C057), and 4 different donors with Braak stage 4 (P012, P046, P047, and P068) have been applied to an analysis of an Affymetrix Human Genome U133 Plus 2.0 Array respectively. Differences reflecting an up-regulation of the SLC39A12 gene progressively with Braak stages predominantly in inferior temporal tissue are shown.

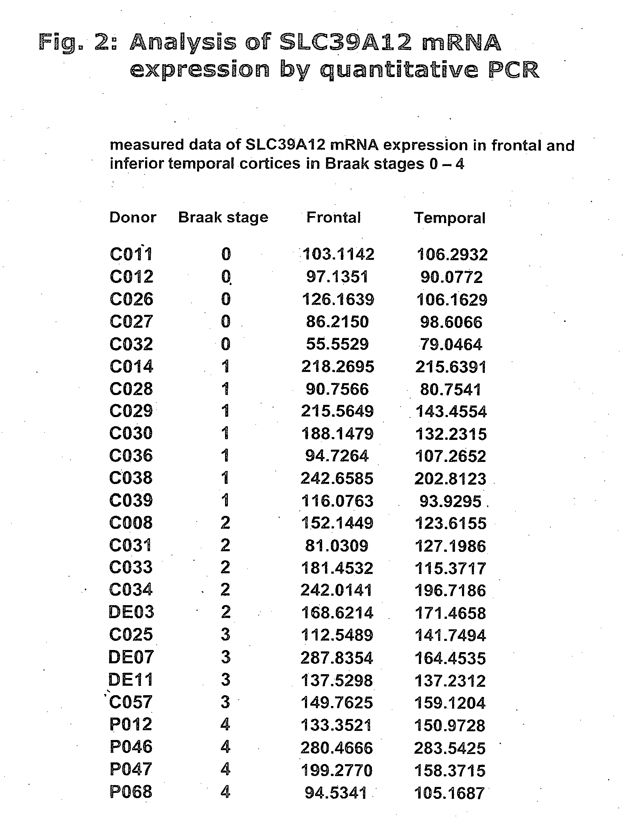

Figure 2 lists the data for the verification of differences in the levels of SLC39A12 gene derived mRNA in human brain tissue samples from individuals corresponding to different Braak stages indicative for AD as measured by quantitative RT-PCR analysis. Quantitative RT-PCR using the Roche Lightcycler rapid thermal cycling technique was performed applying cDNA of the frontal cortex (Frontal) and the inferior temporal cortex (Temporal) of the same donors as used for GeneChip analysis. The data were normalized to values of cyclophilin B a standard gene that showed no significant differences in its gene expression levels. The comparison between samples of the lowest Braak stage 0 with samples representing high Braak stage 4 clearly demonstrates a substantial difference in gene expression level of SLC39A12.

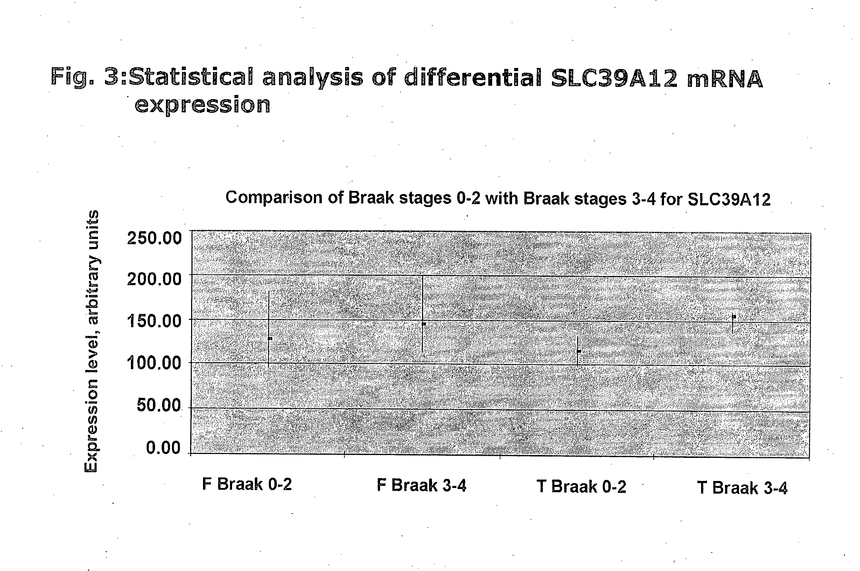

Figure 3 shows the analysis of absolute levels of SLC39A12 gene derived mRNA in human brain tissue samples from individuals corresponding to different Braak stages indicative for AD as measured by quantitative RT-PCR and using statistical method of the median at 98%-confidence level (Sachs L (1988) Statistische Methoden: Planung und Auswertung. Heidelberg New York, p. 60). The data were calculated by defining control groups including subjects with Braak stages 0 to 2, which are compared with the data calculated for the defined groups with advanced AD pathology including Braak stages 3 to 4. A substantial difference reflecting an up-regulation is shown in frontal as well as in inferior temporal cortices corroborating results from the GeneChip analysis. A significant difference reflecting an up-regulation is shown comparing inferior temporal cortex (T) of Braak stage 0-2 with Braak stage 3-4 corroborating results from the GeneChip analysis. Said difference reflects an up-regulation of SLC39A12 in the temporal cortex of individuals with advanced AD pathology relative to the inferior temporal cortex of control persons.

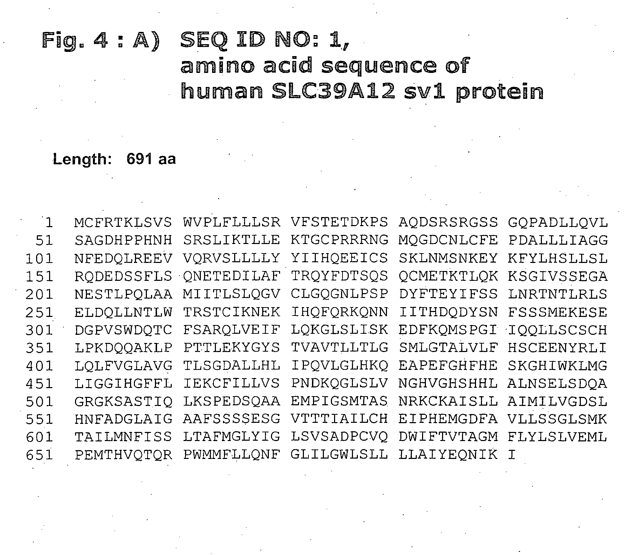

Figure 4A discloses SEQ ID NO: 1, the amino acid sequence of the human SLC39A12 protein (splice variant 1, sv1) (UniProt primary accession number Q504Y0). This SLC39A12 protein comprises 691 amino acids.

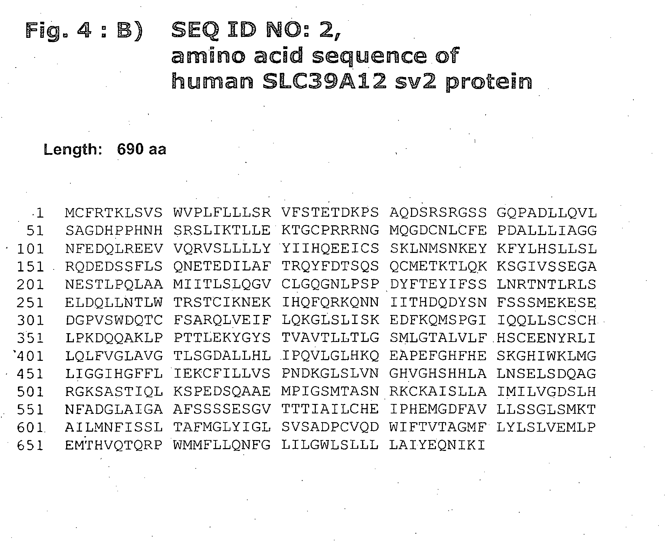

Figure 4B discloses SEQ ID NO: 2, the amino acid sequence of the human SLC39A12 protein (splice variant 2, sv2) (UniProt primary accession number Q5VWV9). This SLC39A12 protein comprises 690 amino acids.

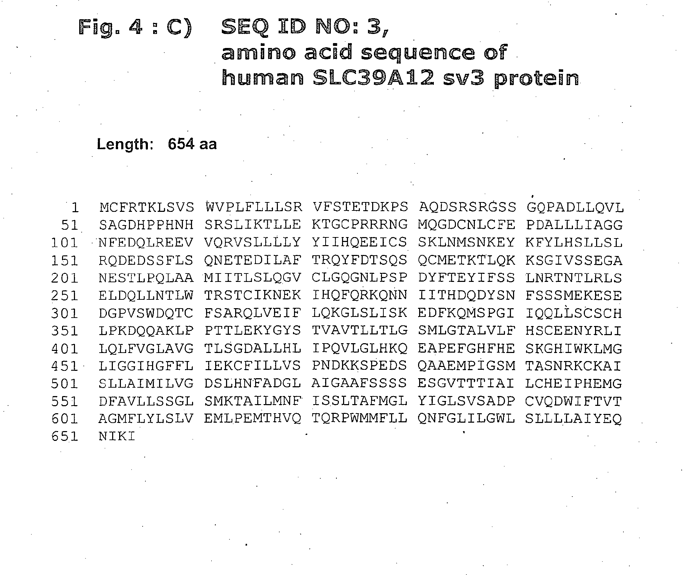

Figure 4C discloses SEQ ID NO: 3, the amino acid sequence of the human SLC39A12 protein (splice variant 3, sv3) (UniProt primary accession number Q5VWV8). This SLC39A12 protein comprises 654 amino acids.

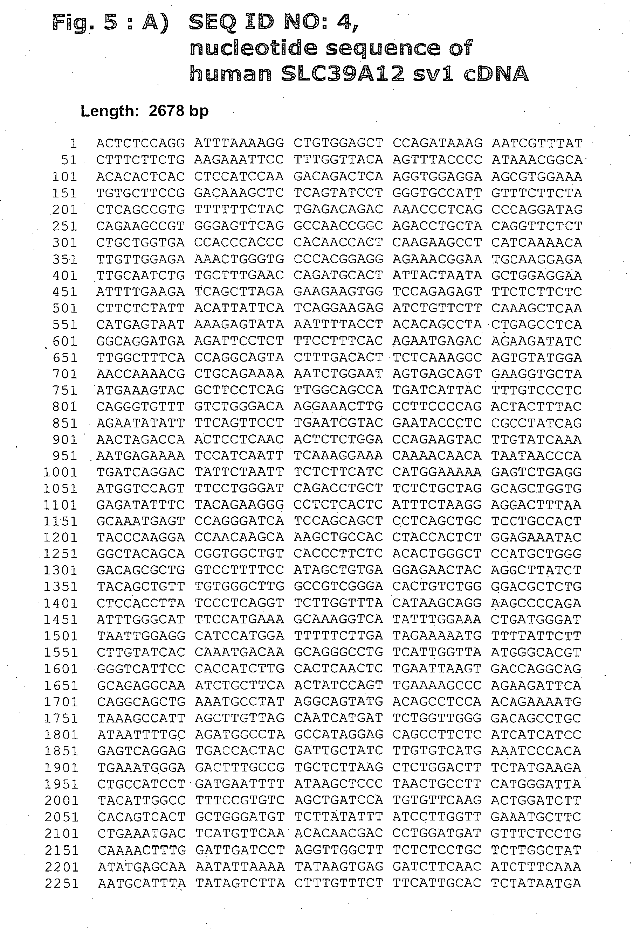

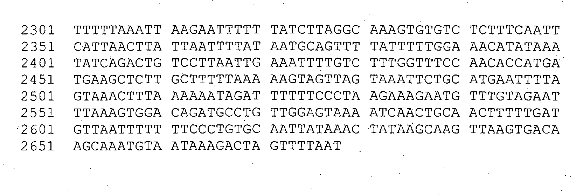

Figure 5A shows SEQ ID NO: 4, the nucleotide sequence of the human SLC39A12 cDNA (splice variant 1, sv1) (Ensembl transcript ID number ENST00000377369) encoding the SLC39A12 sv1 protein, comprising 2678 nucleotides.

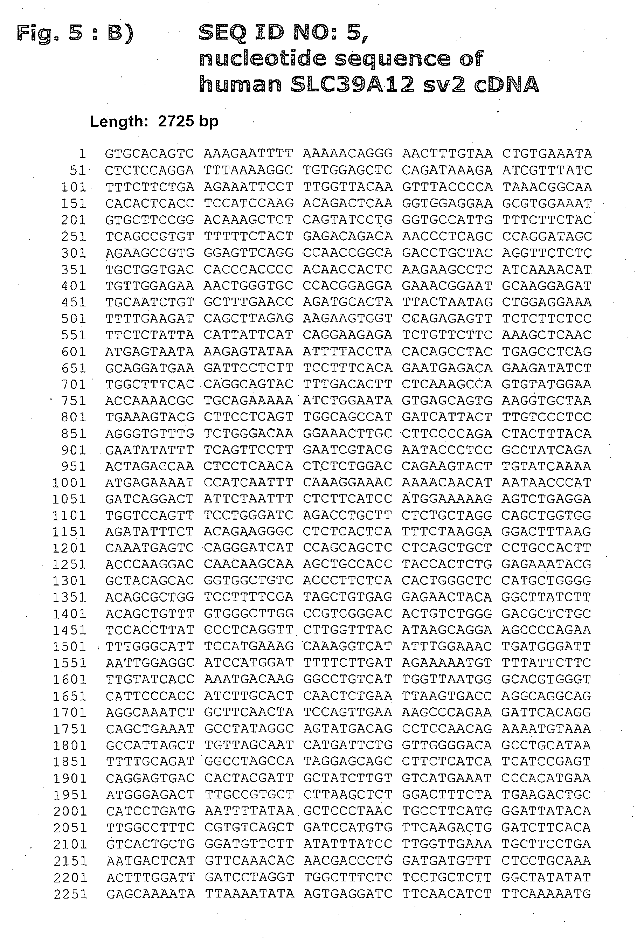

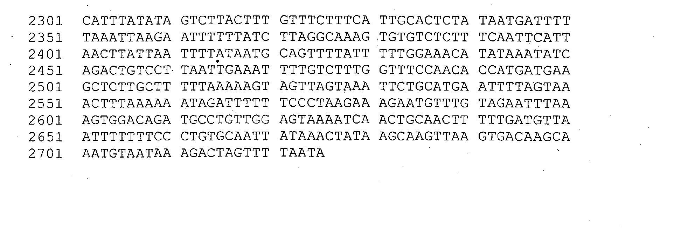

Figure 5B shows SEQ ID NO: 5, the nucleotide sequence of the human SLC39A12 cDNA (splice variant 2, sv2) (Ensembl transcript ID number ENST00000377371) encoding the SLC39A12 sv2 protein, comprising 2725 nucleotides.

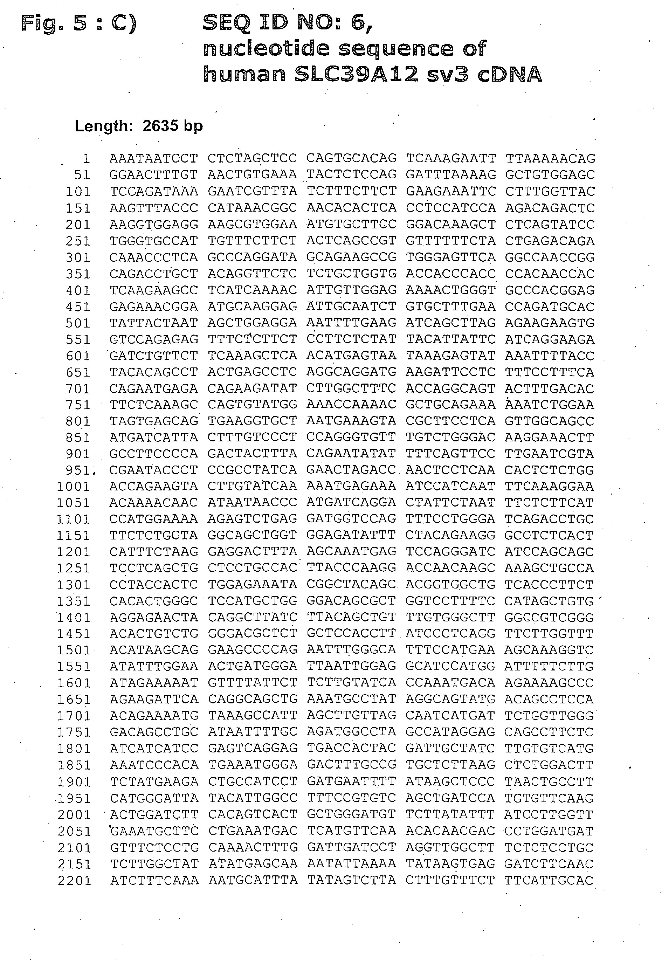

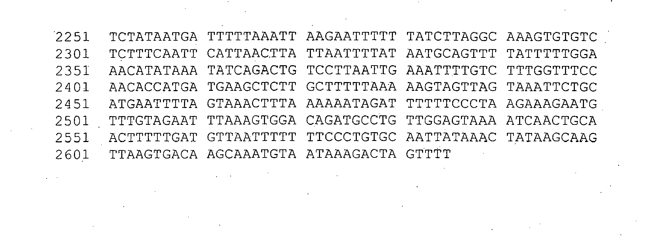

Figure 5C shows SEQ ID NO: 6, the nucleotide sequence of the human SLC39A12 cDNA (splice variant 3, sv3) (Ensembl transcript ID number ENST00000277633) encoding the SLC39A12 sv3 protein, comprising 2635 nucleotides.

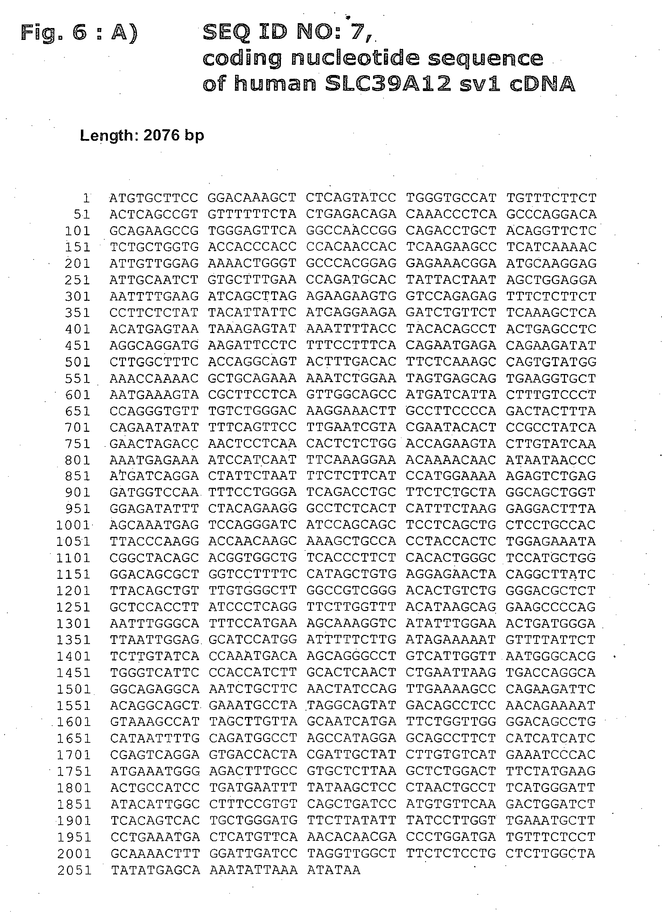

Figure 6A depicts SEQ ID NO: 7, the coding sequence (cds) of the human SLC39A12 sv1, comprising 2076 nucleotides, harbouring nucleotides 150 to 2225 of SEQ ID NO. 4.

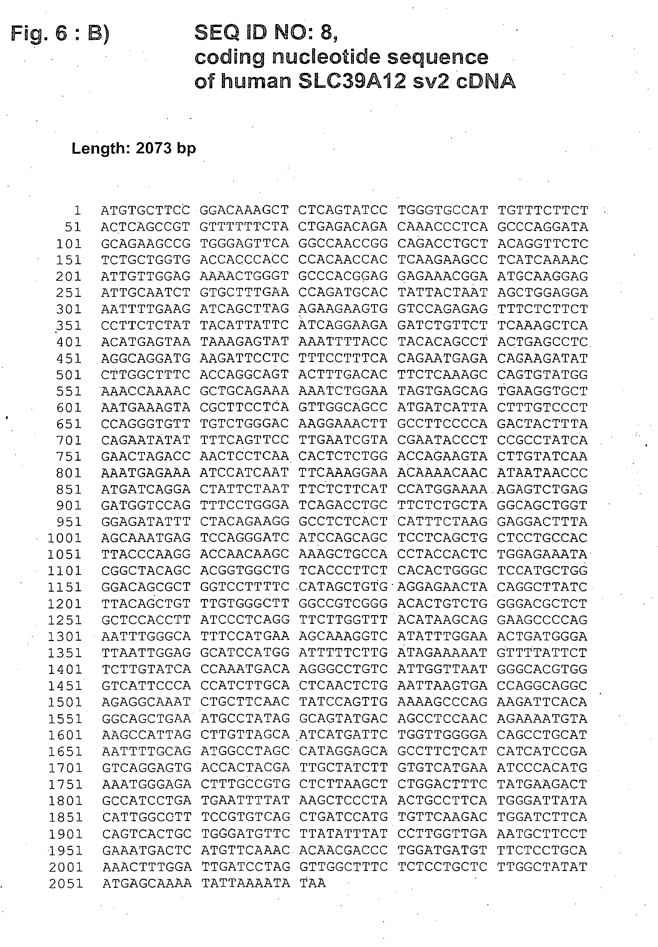

Figure 6B depicts SEQ ID NO: 8, the coding sequence (cds) of the human SLC39A12 sv2, comprising 2073 nucleotides, harbouring nucleotides 199 to 2271 of SEQ ID NO. 5.

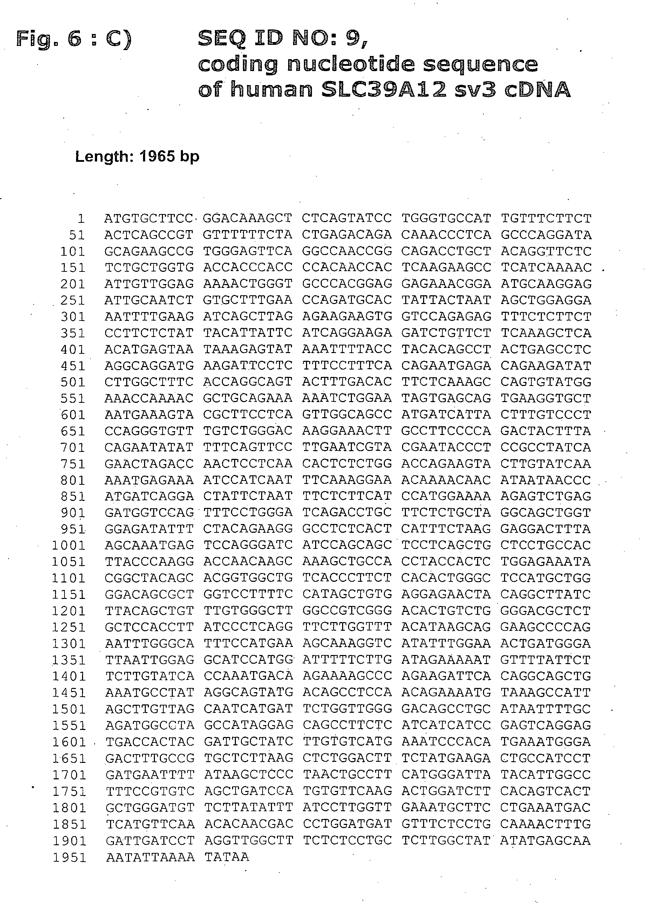

Figure 6C depicts SEQ ID NO: 9, the coding sequence (cds) of the human SLC39A12 sv3, comprising 1965 nucleotides, harbouring nucleotides 221 to 2185 of SEQ ID NO. 6.

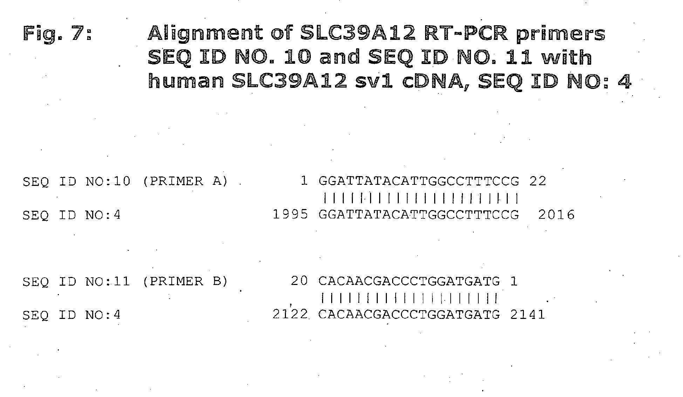

Figure 7 depicts the sequence alignment of the primers used for SLC39A12 transcription level profiling (primer A, SEQ ID NO: 10 and primer B, SEQ ID NO: 11) by quantitative RT-PCR with the corresponding clippings of SEQ ID NO: 4, SLC39A12 cDNA.

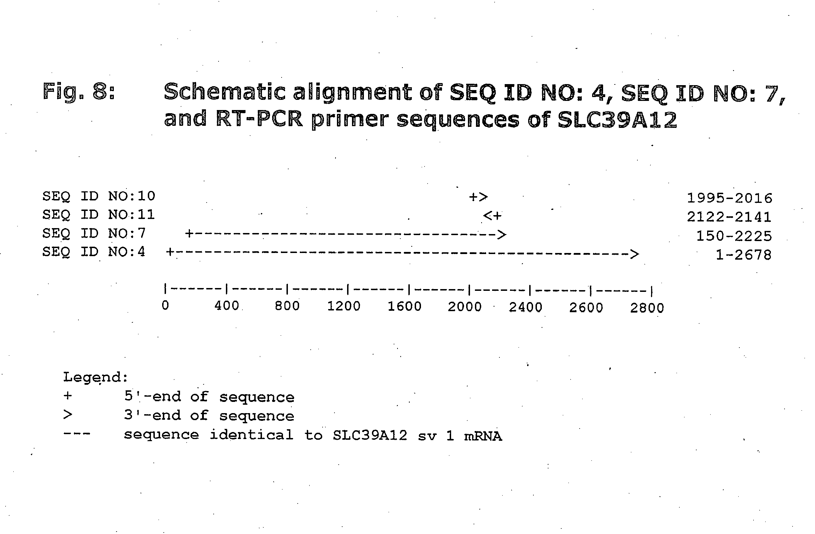

Figure 8 schematically charts the alignment of the SLC39A12 cDNA sequence SEQ ID NO: 4, the SLC39A12 coding sequence SEQ ID NO: 7 and both primer sequences used for SLC39A12 transcription level profiling (SEQ ID NO: 10, SEQ ID NO: 11). Sequence positions are indicated on the right side.

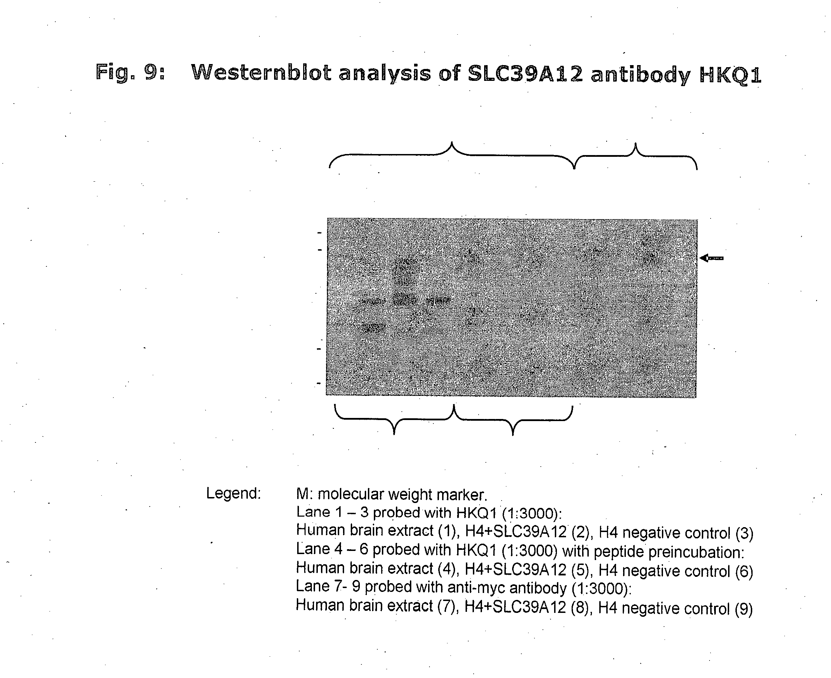

Figure 9 shows an immunoblot (Western blot) analysis of the affinity-purified polyclonal rabbit anti-SLC39A12 antiserum HKQ1. Protein extracts from human brain tissue homogenates and from lysates of either stably transfected H4 cells overexpressing C-terminally myc-tagged human SLC39A12 protein or of naive H4 cells (negative controls) were subjected to SDS-PAGE, blotted onto polyvinylidene difluoride membrane, and probed with either HKQ1 or anti-myc antibody, followed by an appropriate horseradish-peroxidase conjugate secondary antiserum. Blots were soaked with enhanced chemiluminescence substrate, and luminescence was detected on X-ray films. Lanes 1, 4, 7 contain human brain neocortex protein extract, lanes 2, 5, 8 contain protein extract from myc-tagged human SLC39A12 overexpressing transfected H4 cells, and lanes 3, 6, 9 contain protein extract from naive H4 cells. Lanes 1 to 3 were probed with HKQ1 (1:3000), lanes 4 to 6 were probed with HKQ1 (1:3000) after preincubation with the specific peptide, lanes 7 to 9 were probed with an anti-myc antibody (1:3000). Lanes marked with "M" contain molecular weight marker. This example demonstrates that HKQ1 detects a double band migrating between approximately 70 to 90 kDa (arrow), probably reflecting the presence of different isoforms (sv1, sv2, sv3) and/or different states of post-translational modifications of the SLC39A12 full-length protein. This signal is weakly present in human brain extract (lane 1), whereas it is not detected in the negative control cell extract (lane 3). It is completely abolished by preincubation with the specific peptide. The anti-myc antibody detects the SLC39A12 full-length protein as a double band migrating at ~70-90 kDa (lane 8). The faster-migrating bands at ~40 and ~50 kDa, albeit blocked by the peptide, most likely represents unspecifically cross-reactive proteins since they are also detected in the negative control (lane 3). The bands at ~43 kDa and ~55 kDa in lanes 7-9 are unspecific.

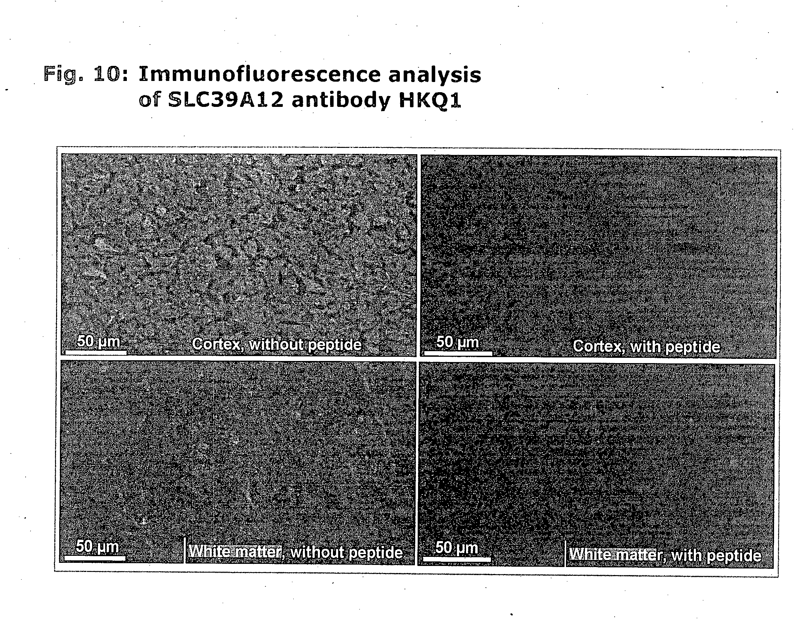

Figure 10 shows an immunohistochemical analysis of the production and localization of SLC39A12 protein in human brain tissue. This typical example demonstrates that SLC39A12 protein is specifically immunodetected in the nuclei and cytoplasm of neurons and glia, as well as in the neuropil. Affinity-purified polyclonal rabbit anti-SLC39A12 antiserum HKQ1 was used for indirect immunofluorescence staining. Depicted are immunofluorescence micrographs of acetone-fixed cryostat sections from a fresh-frozen post-mortem human temporal forebrain specimen. Specific SLC39A12 immunoreactivity is revealed by the affinity-purified polyclonal rabbit anti-SLC39A12 antiserum HKQ1 followed by AlexaFluor-488 conjugated goat anti-rabbit IgG secondary antiserum (Molecular Probes/Invitrogen), visualized as green signals in the left image ("without peptide"), which are abolished when the antiserum is neutralized (peptide-blocked) by pre-incubation with the specific peptide antigen (right image, "with peptide"), confirming the specificity of the immunoreactive signais..

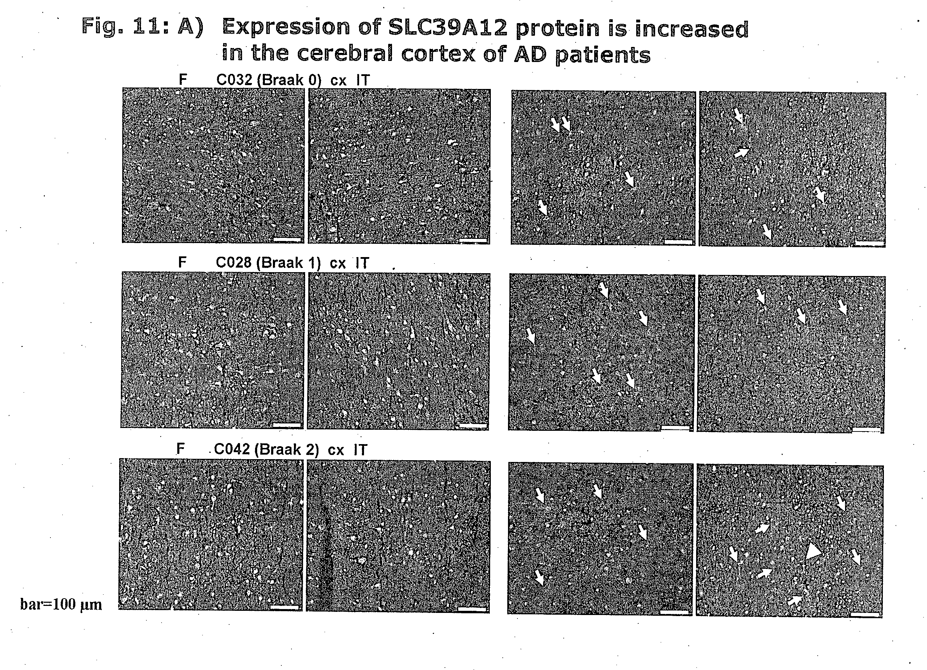

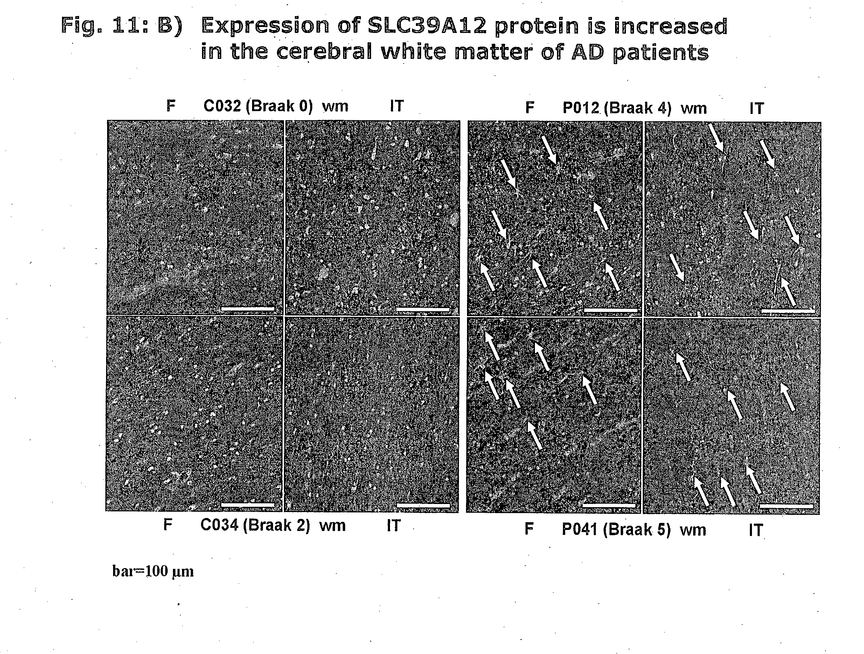

Figures 11 A and 11 B exemplifies an increased SLC39A12 protein expression in cerebral cortex as well as in cerebral white matter observed in human brain specimens from AD patient in comparison to brain specimens from age-matched non - AD control individuals. Depicted are double-immunofluorescence micrographs of acetone-fixed cryostat sections of fresh-frozen post-mortem human forebrain specimens from AD patients and age-matched non AD control donors at the Braak stages indicated in parentheses. Specific SLC39A12 immunoreactivity is revealed by the affinity-purified polyclonal rabbit anti-SLC39A12 antiserum HKQ1 followed by AlexaFluor-488 conjugated goat anti-rabbit IgG secondary antiserum (Molecular Probes/Invitrogen), visualized as green signals. The neuron-specific marker protein NeuN is detected by the mouse monoclonal anti-NeuN antibody (Chemicon) followed by Cy3-conjugated goat anti-mouse IgG secondary antiserum (Jackson/Dianova), visualized in red colour. Nuclei are stained blue by DAPI (Sigma). Scale bars represent 100 μm in length. Abbreviations indicate: F frontal, IT inferior temporal, cx telencephalic neocortex, wm telencephalic white matter. The findings presented here are representative and show a substantial difference between control and AD samples with respect to SLC39A12 expression at the protein-level. In the controls, only very few - if any - astrocytes (cell bodies and processes) with slightly to moderately elevated SLC39A12 immunoreactivity levels may be distinguished from the cortical neuropil (figure 11 A) or in the white matter (figure 11 B). In contrast to the controls, the AD patients' specimens consistently show many intensely SLC39A12 immunopositive, probably reactive astrocytes (cell bodies and processes), both in the cortex (figure 11 A) and in the white matter (figure 11 B). This finding is in agreement with the qPCR profiling data showing increased levels of SLC39A12 mRNA in AD patients' forebrain cortex specimens, as compared to controls, thus corroborating the relationship between up-regulated SLC39A12 gene expression and AD pathology.

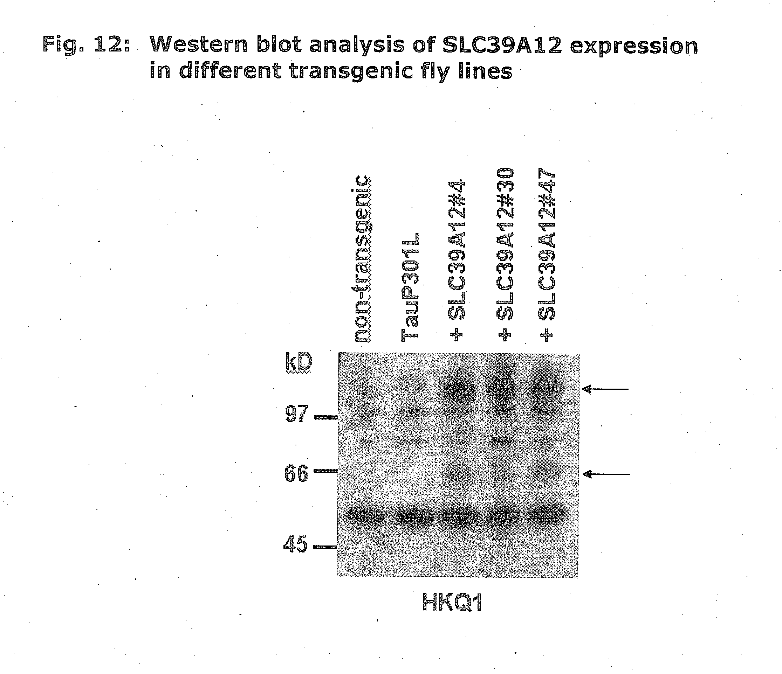

Figure 12 shows the detection of SLC39A12 protein expressed by three independent SLC39A12 transgenic fly lines under the control of gmr-GAL4 by Western blots using a rabbit polyclonal antibody directed against the human SLC39A12. Same amounts of protein were loaded in each lane. Lane 1: non transgenic control flies. SLC39A12 is not expressed in non-transgenic wild-type flies. Lane 2: w; gmr-GAL4/TauP301 L control flies representing the genetic background. Again, SLC39A12 is not expressed in non-transgenic control flies. Lane 3-5: Homogenates of flies co-expressing SLC39A12 and TauP301L. Note: SLC39A12 is detected by the antibody at an apparent molecular weight of 66 kD and 120 kD (black arrows). In transgenic flies, SLC39A12 protein is primarily detected in dimerized form. Genotypes used were (1) w1118; (2) w; gmr-GAL4, UAS-TauP310L/+; (3) w; +/UAS-SLC39A12#4; gmr-GAL4, UAS-TauP310L/+; (4) w; +/UAS-SLC39A12#30; gmr-GAL4, UAS-TauP310L/+; (5) w; gmr-GAL4, UAS-TauP310L/UAS-SLC39A12#47.

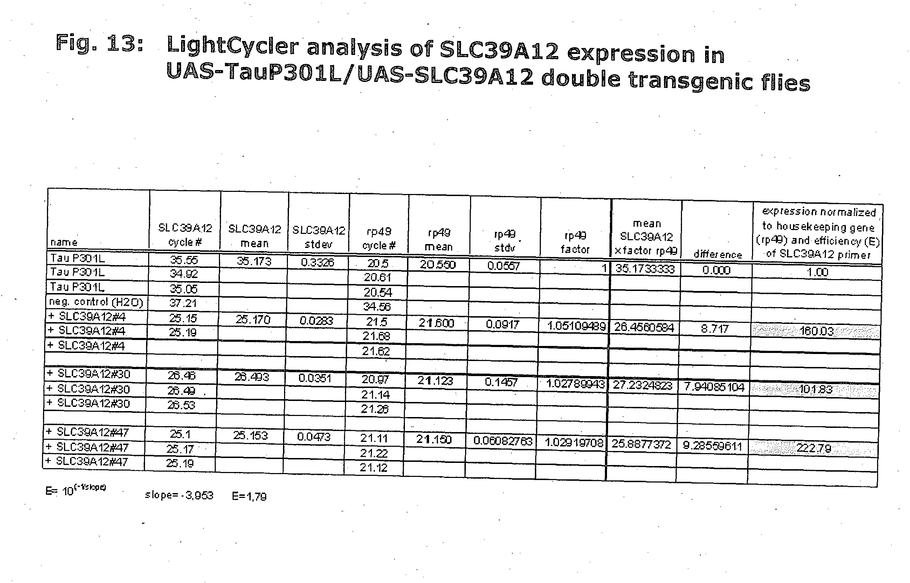

Figure 13 shows a comparison of SLC39A12 mRNA level expressed by three independent SLC39A12 transgenic fly lines under the control of gmr-GAL4 by RT-PCR using SLC39A12 specific primers. The efficiency of the reaction is calculated according to the cycle number and efficiency of the RT-PCR reaction of the SLC39A12 specific primer pair (E=1.79) and was normalized to the expression of a Drosophila housekeeping gene (rp49, ribosomal protein 49). Based on this calculation SLC39A12 is not expressed in control flies expressing TauP301L under the control of gmr-GAL4 (TauP301L). SLC39A12 is expressed at different levels in UAS-TauP301L/UAS-SLC39A12 double transgenic flies. The order of expression efficiency is: SLC39A12#47 > SLC39A12#4 > SLC39A12#30. Measurements were done in triplicates for each genotype. Genotypes used were (1) w; gmr-GAL4, UAS-TauP310L/+; (2) w; +/UAS-SLC39A12#4; gmr-GAL4, UAS-TauP310L/+; (3) w; +/UAS-SLC39A12#30; gmr-GAL4, UAS-TauP310L/+; (4) w; gmr-GAL4, UAS-TauP310L/UAS-SLC39A12#47.

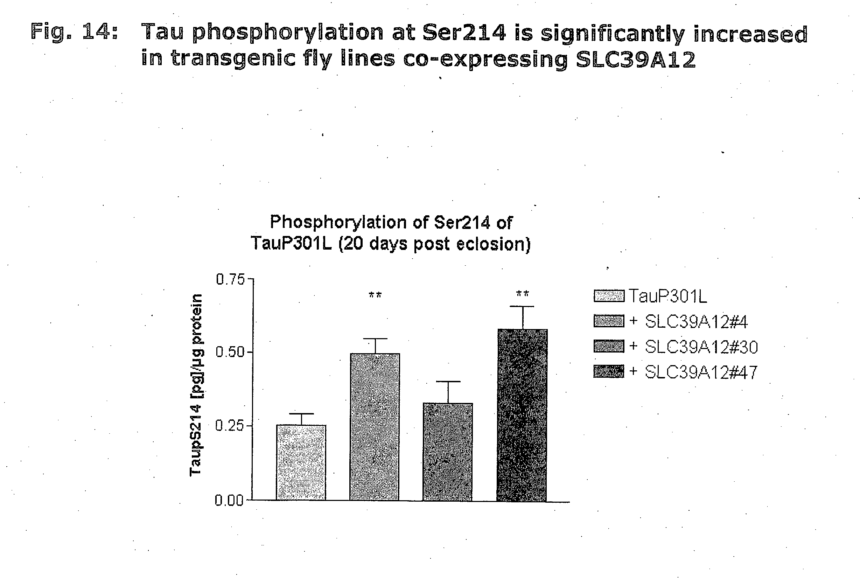

Figure 14 shows that overexpression of SLC39A12 in Drosophila accelerates TauP301 L phosphorylation. Quantification of phosphorylated Ser214 of TauP301L reveals a significant and dosage dependent increase of TauP301 L phosphorylation in two out of three fly lines co-expressing TauP301L and SLC39A12 (#4; #47) in comparison to control flies (TauP301L). Please note: SLC39A12#4 and #47 are strongly expressed in comparison to SLC39A12#30. Statistical analysis was done using GraphPad Prism 3.02. n=12/genotype; p=<0.002. Genotypes used were: (1) w; gmr-GAL4, UAS-TauP310L/+; (2) w; +/UAS-SLC39A12#4; gmr-GAL4, UAS-TauP310L/+; (3) w; +/UAS-SLC39A12#30; gmr-GAL4, UAS-TauP310L/+; (4) w; gmr-GAL4, UAS-TauP310L/UAS-SLC39A12#47.

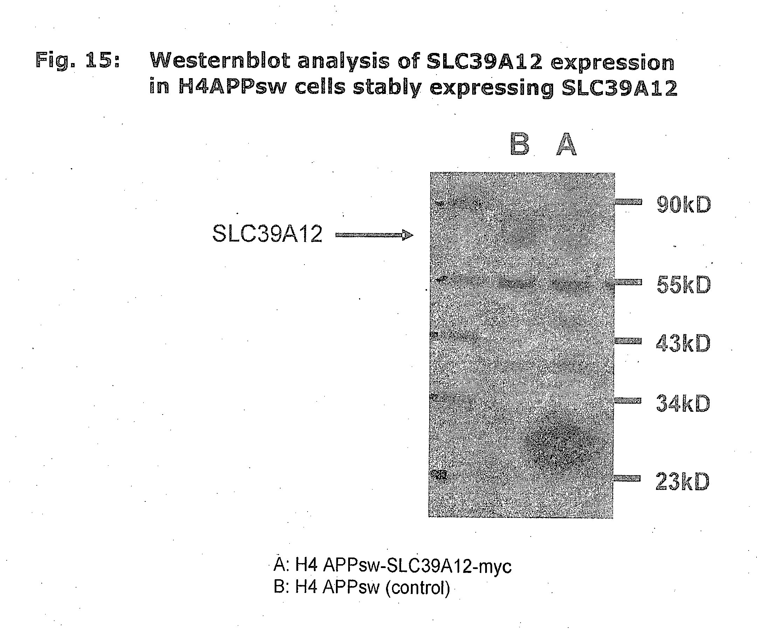

Figure 15 shows Western blot analysis of SLC39A12-expression in H4-neuroglioma cells stably co-expressing the Swedish Mutant APP. SLC39A12 was myc-tagged at the C-terminus and introduced into tissue culture cells. Expression of SLC39A12 is driven by the CMV-promoter. Cells were harvested, lysed and subjected to Western Blot analysis using an antibody directed against the myc-epitope at a 1:3000 dilution. In lane B bands running at approx. 85 kDa becomes visible. In the control cell line expressing an unrelated protein no corresponding band running at the same molecular weight is visible (lane A)

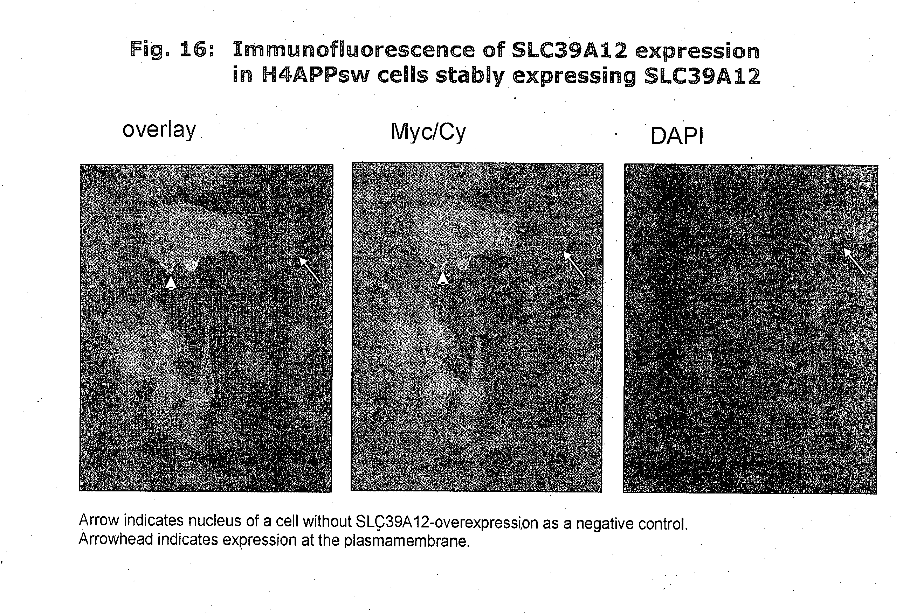

Figure 16 shows SLC39A12 expression in H4-neuroglioma cells stably expressing the Swedish Mutant APP by immunofluorescence analysis. SLC39A12 was myc-tagged at the C-terminus and introduced into tissue culture cells. Expression of SLC39A12 is under the control of the CMV-promoter. SLC39A12 -expressing cells were seeded onto a glass cover slip and after 24 hours of incubation cells where fixed with methanol for immunofluorescence analysis. Expression of SLC39A12 was detected using an antibody directed against the myc-epitope at a 1:3000 dilution followed by incubation with a fluorescently labelled antibody directed against the anti-myc antibody (1:1000). Cells were then mounted onto a microscope slide and analysed under a fluorescence microscope. Expression of SLC39A12 is visible in the cytoplasm and at the plasma-membrane of the cells in the left and middle pictures (see arrowhead pointing to the strong expression at the border of the cell indicating the localization at the membrane). Arrow points to the nucleus of a cell where no green fluorescence can be detected indicating no expression of SLC39A12. The blue colour in the left and right pictures is indicative of the nucleus of the cells and has been visualized by means of DAPI (1:1000).

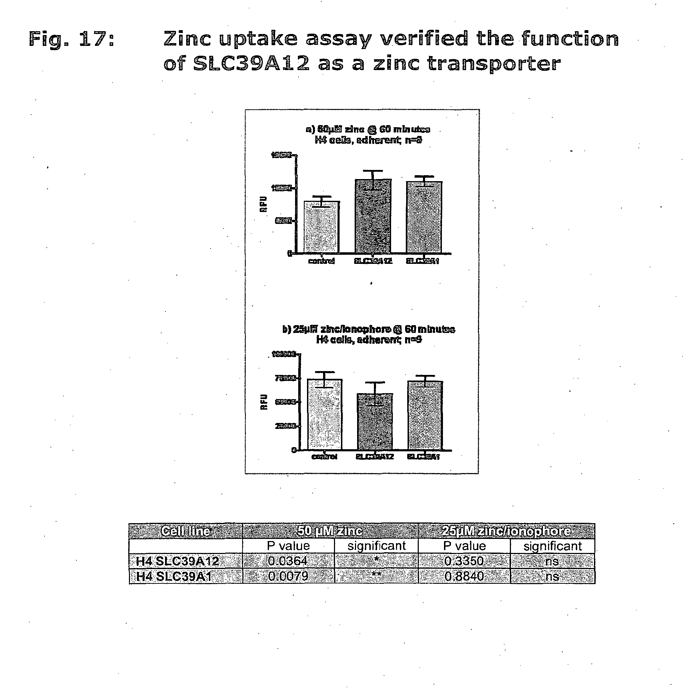

Figure 17 shows the verification of the function of SLC39A12 as zinc transporter by using a zinc uptake assay. Depicted is the zinc accumulation in adherent H4 cells over-expressing SLC39A12 or SLC39A1 60 minutes after application of a) 50$\mu$M zinc or b) 25$\mu$M zinc/ionophore. (RFU = relative fluorescence units). The RFU signals indicate that H4 or H4APPsw cells over-expressing SLC39A12 and SLC39A1 have accumulated 1.5-2 times more zinc after 60 minutes as compared to control cells. The addition of the zinc/ionophore to the cells leads to a zinc influx independently of transporters being expressed in the cells which results in an approximately 7 times higher fluorescent value as compared to cells incubated with zinc only. For statistical analysis RFU values of zinc and zinc/ionophore treated cells listed in the table were compared to RFU values of H4 control cells by T-Test analysis at 95% confidence interval. T-Test analysis revealed that the fluorescence signals in SLC39A12 and SLC39A1 over-expressing cells are statistically significant as compared to H4 control cells in presence of zinc pointing to an increased uptake of the zinc ions. No statistically significant differences were obtained in case of the zinc/ionophore treated cells which also points to identical amounts of cells plated. In conclusion SLC39A12 when over-expressed in H4-cells increases the intracellular concentration of zinc, therefore it was demonstrated that SLC39A12 is functioning as a zinc transporter.

EXAMPLES:

EXAMPLE 1: Identification and verification of Alzheimer's disease related differentially expressed genes in human brain tissue samples.

In order to identify specific differences in the expression of genes that are associated with AD, GeneChip microarray (Affymetrix) analyses were performed with a diversity of cRNA probes derived from human brain tissue specimens from clinically and neuropathologically well characterized individuals. This technique is widely used to generate expression profiles of multiple genes and to compare populations of mRNA present in different tissue samples. In the present invention, mRNA populations present in selected post-mortem brain tissue specimens (frontal and inferior temporal cortex) were analyzed. Tissue samples were derived from individuals that could be grouped into different Braak stages reflecting the full range between healthy control individuals (Braak 0) and individuals that suffered from AD signs and symptoms (Braak 4). Verification of the differential expression of individual genes was performed applying real-time quantitative PCR using gene-specific oligonucleotides. Furthermore specific differences between healthy and disease stages were analysed at the protein level using gene product specific antibodies for immunohistochemical analyses. The methods were designed to specifically detect differences of expression levels at early Braak stages, which is indicative for pathological events occurring early in the course of the disease. Thus, said genes identified to be differential are effectively implicated in the pathogenesis of AD.

(i) Brain tissue dissection from patients with AD:

Brain tissues from AD patients and age-matched control subjects, were collected. Within 6 hours post-mortem time the samples were immediately frozen on dry ice. Sample sections from each tissue were fixed in paraformaldehyde and neuropathologically staged at various stages of neurofibrillary pathology according to Braak and Braak into Braak stages (0-6). Brain areas for differential expression analysis were identified and stored at -80 °C until RNA extractions were performed.

(ii) Isolation of total mRNA:

Total RNA was extracted from frozen post-mortem brain tissue by using the RNeasy kit (Qiagen) according to the manufacturer's protocol. The accurate RNA concentration and the RNA quality were determined applying the Eukaryote total RNA Nano LabChip system by using the 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology (Roche) as described in the supplied protocol by the manufacturer.

(iii) Probe synthesis:

Here, total RNA was used as starting material, which had been extracted as described above (ii). For production of cDNAs, the cDNA Synthesis System was performed according to the manufacturer's protocol (Roche). cDNA samples were transcribed to cRNA and labeled with biotin applying the in vitro-transcription T7-Megascript-Kit (Ambion) according to the manufacturer's protocol. The cRNA quality was determined applying the mRNA Smear Nano LabChip system using the 2100 Bioanalyzer (Agilent Technologies). The accurate cRNA concentration was determined by photometric analysis (OD260/280 nm).

(iv) GeneChip hybridization:

The purified and fragmented biotin labeled cRNA probes together with commercial spike controls (Affymetrix) bioB (1.5 pM), bioC (5 pM), bioD (25 pM), and cre (100 pM) were resuspended each at a concentration of 60 ng/μl in hybridization buffer (0.1 mg/ml Herring Sperm DNA, 0.5 mg/ml Acetylated BSA, 1 x MES) and subsequently denaturated for 5 min at 99 °C. Subsequently, probes were applied each onto one prehybridized (1 x MES) Human Genome U133 Plus 2.0 Array (Affymetrix). Array hybridization was performed over night at 45°C and 60 rpm. Washing and staining of the microarrays followed according to the instruction EukGe_WS2v4 (Affymetrix) controlled by GeneChip Operating System (GCOS) 1.2 (Affymetrix).

(v) GeneChip data analysis:

Fluorescence raw data were taken using the GeneScanner 3000 (Affymetrix) controlled by GCOS 1.2 software (Affymetrix). Data analysis was performed using DecisionSite 8.0 for Functional Genomics (Spotfire): raw data were delimitated to those that were flagged as "present" by the GCOS 1.2 software (Affymetrix); normalization of raw data was performed by percentile value; detection of differential mRNA expression profiles was performed using profile search tool of the Spotfire software. The result of such GeneChip data analysis for the gene coding for SLC39A12 protein is shown in Figure 1.

(vi) Quantitative RT-PCR:

Positive corroboration of differential SLC39A12 gene expression was performed using the LightCycler technology (Roche). This technique features rapid thermal cycling for the polymerase chain reaction as well as real-time measurement of fluorescent signals during amplification and therefore allows for highly accurate quantification of RT-PCR products by using a kinetic, rather than endpoint readout. The relative quantity of SLC39A12 cDNAs from the frontal and temporal cortices of AD patients and age-matched control individuals respectively, were determined in a number of four up to nine tissues per Braak stage.
First, a standard curve was generated to determine the efficiency of the PCR with specific primers for the gene coding for SLC39A12:

Primer A, SEQ ID NO: 10, 5'-GGATTATACATTGGCCTTTCCG-3' (nucleotides 2043-2064 of SEQ ID NO: 4) and
Primer B, SEQ ID NO: 11, 3'-CACAACGACCCTGGATGATG-5' (nucleotides 2170-2189 of SEQ ID NO: 4).

PCR amplification (95°C and 1 sec, 56°C and 5 sec, and 72°C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and additional 3 mM $MgCl_2$. Melting curve analysis revealed a single peak at approximately 82.5°C with no visible primer dimers. Quality and size of the qPCR product were determined applying the *DNA 500* LabChip system using the *2100 Bioanalyzer* (Agilent Technologies). A single peak at the expected size of 147 bp for the gene coding for SLC39A12 proteins was observed in the electropherogram of the sample.
In an analogous manner, the qPCR protocol was applied to determine the PCR efficiency of cyclophilin B, using the specific primers SEQ ID NO: 12, 5'-ACTGAAGCACTACGGGCCTG-3' and SEQ ID NO: 13, 5'-AGCCGTTGGT-GTCTTT-GCC-3' except for $MgCl_2$ (an additional 1 mM was added instead of 3 mM). Melting curve analysis revealed a single peak at approximately 87°C with no visible primer dimers. Bioanalyzer analysis of the PCR product showed one single peak of the expected size (62 bp).

For calculation of the standard values, first the logarithm of the used cDNA concentration was plotted against the threshold cycle value $C_t$ for SLC39A12 and Cyclophilin B respectively. The slopes and the intercepts of the standard curves (i.e. linear regressions) were calculated. In a second step, mRNA expression from frontal and inferior temporal cortices of controls and AD patients were analyzed in parallel. The $C_t$ values were measured and converted to ng total brain cDNA using the corresponding standard curves:

$$10 \wedge (C_t \text{ value - intercept}) / \text{slope} \quad [\text{ng total brain cDNA}]$$

Calculated cDNA concentration values were normalized to Cyclophilin B that was analyzed in parallel for each tested tissue probe, thus resulting values are defined as arbitrary relative expression levels. The results of such quantitative RT-PCR analysis for the gene coding for SLC39A12 proteins are shown in Figure 2.

(vii) Statistical analysis of the mRNA expression comparing donor groups with different Braak stages.

For this analysis it was proven that absolute values of real-time quantitative PCR (Lightcycler method) between different experiments at different time points are consistent enough to be used for quantitative comparisons without usage of calibrators. Cyclophilin was used as a standard for normalization in any of the qPCR experiments for more than 100 tissues. Between others it was found to be the most consistently expressed housekeeping gene in the normalization experiments. Therefore a proof of concept was done by using values that were generated for cyclophilin.

First analysis used cyclophilin values from qPCR experiments of frontal cortex and inferior temporal cortex tissues from three different donors. From each tissue the same cDNA preparation was used in all analyzed experiments. Within this analysis no normal distribution of values was achieved due to small number of data. Therefore the method of median and its 98 %-confidence level was applied (Sachs L (1988) Statistische Methoden: Planung und Auswertung. Heidelberg New York, p. 60). This analysis revealed a middle deviation of 8.7 % from the median for comparison of absolute values and a middle deviation of 6.6 % from the median for relative comparison.

Second analysis used cyclophilin values from qPCR experiments of frontal cortex and inferior temporal cortex tissues from two different donors each, but different cDNA preparations from different time points were used. This analysis revealed a middle deviation of 29.2 % from the median for comparison of absolute values and a middle deviation of 17.6 % from the median for relative comparison. From this analysis it was concluded, that absolute values from qPCR experiments can be used, but the middle deviation from median should be taken into further considerations.

A detailed analysis of absolute values for SLC39A12 was performed using the method of median and its 98 %-confidence level. Because in contrast to the mean the calculation of the median is not affected by single data outliers; therefore latter is the method of choice for a small number of data that are distributed non-normal and/or assymetric (Sachs L (1988) Statistische Methoden: Planung und Auswertung. Heidelberg New York, p. 60). Therefore, absolute levels of SLC39A12 were used after relative normalization with cyclophilin. The median as well as the 98 %-confidence level was calculated for a group consisting of low level Braak stages (Braak 0 - Braak 2) and the group consisting of high level Braak stages (Braak 3 - Braak 4). The analysis was aimed to identify early onset of mRNA expression differences within the course of AD pathology. Said analysis described above is shown in Figure 3.

(viii) Generation of specific antibodies:

Antibodies specifically recognizing human SLC39A12 protein were raised in rabbits according to a standard immunization protocol followed by affinity purification of the resulting polyclonal immune sera (Davids Biotechnologie GmbH, Regensburg, Germany). Specific antigen peptides were designed and synthesized, mimicking unique amino acid sequence clippings of the human SLC39A12 full-length protein. E.g., the peptide HKQEAPEFGHFHESKGH (from amino-terminus to carboxy-terminus, in single-letter code), representing a unique amino acid sequence corresponding to positions 428 to 434 of the human SLC39A12 full-length protein, was selected and synthesized as specific antigen for immunization and affinity purification. The resulting affinity-purified antiserum is called "HKQ1", and its specificity and sensitivity were confirmed on immunoblots with protein extracts prepared from human brain tissue homogenates and from cell lysates of either stably transfected cells overexpressing myc-tagged human SLC39A12 protein or untransfected naive cells as negative control. Neutralization of the antibodies by pre-incubation with the corresponding specific peptide resulted in the abolishment of immunoreactive signals, confirming their specificity (peptide block), which was performed on immunoblots and on tissue sections (immunofluorescence histology, immunohistochemistry).

(ix) Verification of differential expression of the SLC39A12 gene and association with AD at the protein level applying immunohistochemical analyses:

For immunofluorescence staining of SLC39A12 in human brain, and for the comparison of AD-affected tissues with control tissues, post-mortem fresh-frozen frontal and temporal forebrain specimens from donors comprising patients with clinically diagnosed and neuropathologically confirmed AD at various stages of neurofibrillary pathology according to Braak and Braak (herein before and after briefly called "Braak stages") as well as age-matched non AD control individuals with neither clinical nor neuropathological signs of AD were cut at 14 μm thickness using a cryostat (Leica CM3050S). The tissue sections were air-dried at room temperature and fixed in acetone for 10 min, and air-dried again. After washing in PBS, the sections were pre-incubated with 10% normal goat serum in phosphate buffered saline (PBS) for 30 min and then incubated with affinity-purified anti-SLC39A12 rabbit polyclonal antiserum HKQ1 diluted 1:20 in blocking buffer (1% bovine serum albumin in PBS), overnight at 4°C. After rinsing three times in PBS, the sections were incubated with AlexaFluor-488-conjugated goat anti-rabbit IgG antiserum (Jackson/Dianova, Hamburg, Germany), in a 1:1500 dilution in blocking buffer for 2 hours at room temperature and then again washed with PBS. Simultaneous staining of neuronal somata (including nuclei) was performed as described above using a mouse monoclonal antibody against the neuron-specific marker protein NeuN (Chemicon, Hampshire, UK; dilution 1:350), followed by a secondary Cy3-conjugated goat anti-mouse antibody (Jackson/Dianova; dilution 1:1000). Staining of nuclei was performed by incubation of the sections with 0.5 μM DAPI

in PBS for 3 min. In order to block lipofuscin autofluoresence, the sections were treated with the lipophilic black dye Sudan Black B (1% w/v) in 70% ethanol for 5 min at room temperature and then sequentially dipped in 70% ethanol, destilled water and PBS. The sections were coverslipped with ProLong-Gold antifade mounting medium (Invitrogen/Molecular Probes, Karlsruhe, Germany). Microscopic epifluorescence images were obtained using an upright microscope with a mercury arc lamp (BX51, Olympus, Hamburg, Germany). The appropriate dichromic filter and mirror combinations (hereinafter called "channels") were used for the specific excitation of either fluorochrome (AlexaFluor-488, Cy3, DAPI) and for reading out the emitted fluorescence light resulting from the specific labeling by said antibodies or the nuclear DAPI stain. Microscopic images were digitally captured with a charge-coupled display camera and the appropriate image acquisiton and processing software (ColorView-II and AnalySIS, Olympus Soft Imaging Solutions GmbH, Münster, Germany). Fluorescence micrographs obtained from the different channels were overlaid in order to generate simultaneous views of the above specified immunolabelings and nuclei (DAPI) in the RGB mode, e.g. for analyzing the potential co-localization of signals from the three different channels.

EXAMPLE 2: Analyses of SLC39A12 functions in AD using transgenic Drosophila melanogaster and cell based zinc uptake assay.

Human BACE transgenic flies were generated according to Greeve et al. (Greeve et al., J. Neurosci. 2004, 24: 3899-3906). Human TauP301L transgenic flies and SLC39A12myc transgenic flies were generated as follows. A 1.4 kb EcoRI restriction fragment containing the entire open reading frame of the human microtubule-associated protein tau isoform NP_005901.2 (RefSeq peptide ID) was subcloned into the EcoRI site of pUAST downstream of the GAL4 binding sites UAS (Brand and Perrimon, Development 1993, 118: 401-15). A C to T (cC/Tgggaggcg) mutation was introduced to change proline (CCG) to leucine (CTG) at amino acid position 301 (TauP301L, cDNA was kindly provided by Jürgen Goetz, Götz et al., Science 2001; 24 Vol. 293. no. 5534, pp. 1491 - 1495). A 2122 bp EcoRI/XhoI restriction fragment containing the entire open reading frame of SLC39A12 labelled C-terminal with a myc-tag (SLC39A12myc) was subcloned into the EcoRI/XhoI site of pUAST downstream of the GAL4 binding sites UAS (Brand and Perrimon, Development 1993, 118: 401-15). P-element-mediated germline transformation was performed as described by Spradling and Rubin (Rubin and Spradling, Science 1982, 218: 348-53; Spradling and Rubin, Science 1982, 218: 341-7). Three independent human TauP301L transgenic fly lines were generated and tested for expression of full length tau protein. Thirteen independent SLC39A12myc transgenic fly lines were generated and three lines were selected for the analysis.
Human APP and Drosophila Presenilin transgenic flies, the UAS-APP695II and the UAS-DPsn-mutants (L235P), were kindly provided by R. Paro and E. Fortini (Fossgreen et al., Proc Natl Acad Sci USA 1998, 95: 13703-8; Ye and Fortini, J Cell Biol 1999, 146: 1351-64). The gmr-GAL4 driver line obtained from F. Pignoni was used to achieve eye-specific expression of the transgenes.

(i) Genetics of transgenic flies:

Genetic crosses were set up on standard Drosophila culture medium at 25°C. Genotypes used were: w; UAS-hAPP695, UAS-hBACE437/CyO; gmr-GAL4/Tm3 - w; UAS-hAPP695, UAS-hBACE437/CyO; gmr-GAL4,UAS-DPsnL235P/Tm3 - w; gmr-GAL4, UAS-TauP310L/Tm6 - w; UAS-SLC39A12myc#4 (2nd chromosome, homozygous viable) - w; UAS-SLC39A12myc#30 (2nd chromosome, homozygous viable) - w; UAS-SLC39A12myc#47 (3rd chromosome, homozygous viable).

(ii) Expression analysis of SLC39A12 transgenic flies by Immunoblot analysis:

For protein analyses by western blotting, fly heads were homogenized in 1xPBS, 5 mM EDTA, 0.5 % Triton X-100 and a protease-inhibitor mix Complete (Roche Applied Science). Equal amounts of protein were separated by 10 % SDS-PAGE, transferred to Immobilon membranes (Millipore GmbH), blocked in 5 % low fat milk for two hours at room temperature and incubated with rabbit polyclonal antibody HKQ1 (anti-SLC39A12). Bound antibodies were detected with goat anti-rabbit peroxidase conjugated secondary antibodies (Dianova).

(iii) Expression analysis of SLC39A12 transgenic flies by RT-PCR:

For the detection of transgenic SLC39A12 expression in transgenic Drosophila a reverse transcriptase PCR (RT-PCR Reaction) reaction was performed using SLC39A12 specific primers (5'-GGATTATACATTGGCCTTTCCG, 3'-CATCATCCAG GGTCGTTGTG) as described in the present invention (Example (vi)).

(iv) pSer214 specific ELISA:

Ten fly heads were homogenized in 50 μl cell lysis buffer containing phosphatase inhibitors (10 mM Tris, pH7,4; 100 mM NaCl; 1 mM EDTA; 1 mM EGTA; 1 mM NaF; 20 mM Na4P2O7; 2 mM Na3VO4; 1 % Triton X-100; 10 % glycerol; 0,1 % SDS; 0,5 % deoxycholate, 10 μM okadaic acid). Lysates were centrifuged 3 min at 13000 rpm and the supernatant was used at a 1:100 dilution to determine phosphorylation at Ser214 of TauP301L by using a Human Tau [pS214] Immunoassay Kit (Biosource) according to the manufacturer's protocol. The amount of phosphorylated S214 was normalized to the total amount of TauP301L by using a Human Tau [total] Immunoassay Kit (Biosource). The same lysates were used at a dilution of 1:8000. GraphPad Prism 3.02 was used for the statistical analysis.

(v) Generation of cells stably expressing SLC39A12 or SLC39A1:

SLC39A12 or SLC39A1 were transduced into the H4-neuroglioma cell line expressing the Swedish mutant amyloid precursor protein (APP) and clonal cell lines were isolated after the addition of the antibiotic G418 essentially following the manufacturer's protocol (Stratagene, Cat. No. 217561). Both, SLC39A12 and SLC39A1 were myc-epitope-tagged at the C-terminus to allow an analysis in immunofluorescence.

(vi) Zinc-uptake assay:

$1.10^4$ cells were seeded in 384-well cell culture plates (black, clear bottom) and incubated over night at 37°C and 8.5% CO2. Medium was removed, cells were washed once with 50 μl of buffer consisting of 50 mM MOPS, pH 7.0 and incubated for 30 minutes in 50 mM MOPS containing 3.5 mM FluoZin 1 (Molecular Probes, F-24181) at 37 °C and 8.5 % CO2. A quencher (Brillant

Black in 50 mM MOPS, pH 7.0) was applied to reduce the background fluorescence in the medium and 50 μM zinc or 25 μM zinc together with 25 μM of the zinc-specific ionophore pyrithione (Sigma H7029) was added. Fluorescence intensity was measured after 60 minutes using a BMG Fluostar reader. The mean RFU (relative fluorescence unit) of buffer controls was subtracted from RFU values of zinc and zinc/ionophore-treated cells. For statistical analysis RFU values of zinc or zinc/ionophore treated cells overexpressing SLC39A12, or SLC39A1 were compared to RFU values of zinc or zinc/ionophore-treated control cells by applying a T-test at 95% confidence interval (GraphPad Prism) 60 minutes after zinc application. Data from at least 2 independent experiments were combined ($n \geq 8$).

## Claims

**1.** A method of diagnosing or prognosticating a neurodegenerative disease in a subject, or of determining whether a subject has a predisposition of developing said disease, which method comprises the steps of:

(a) determining a level and/or an activity of (i) a transcription product of the gene coding for SLC39A12 proteins, and/or (ii) a translation product of the gene coding for SLC39A12 proteins, in a sample obtained from said subject,
(b) comparing said level and/or said activity of said transcription product and/or said translation product to a reference value representing a known disease status and/or representing a known health status and/or representing a known Braak stage,

(c) analysing whether said level and/or said activity is varied compared to a reference value representing a known health status, and/or is similar or equal to a reference value representing a known disease status or representing a known Braak stage which is an indication that said subject has a neurodegenerative disease, or that said subject is at increased risk of developing said disease,
wherein said neurodegenerative disease is Alzheimer's disease, and wherein said gene coding for SLC39A12 proteins is the gene coding for a SLC39A12 protein having SEQ ID NO: 1, or SEQ ID NO: 2, or SEQ ID NO: 3 and wherein said translation product of the gene coding for SLC39A12 proteins is the SLC39A12 protein having SEQ ID NO: 1, or SEQ ID NO: 2, or SEQ ID NO: 3.

**2.** Use of a kit for diagnosing or prognosticating Alzheimer's disease, in a subject, or for determining the predisposition of a subject to develop such a disease, according to the method of claim 1, wherein the kit comprises at least one reagent which is selected from the group consisting of reagents that detect (i) a transcription product of the gene coding for SLC39A12 proteins and/or (ii) a translation product of the gene coding for SLC39A12 proteins, wherein the SLC39A12 proteins have the SEQ ID NO: 1, or SEQ ID NO: 2, or SEQ ID NO: 3.

**3.** A use of a genetically modified non-human animal as a non-human test animal and/or control animal for screening, testing, and/or validating compounds, agents, and modulators in the development of diagnostics and therapeutics useful for the treatment of Alzheimer's disease, wherein the genetically modified non-human animal comprises a non-native gene sequence coding for a SLC39A12 protein of SEQ ID NO: 1, or SEQ ID NO: 2, or SEQ ID NO: 3 , under the control of a transcriptional element which is not the native SLC39A12 gene transcriptional control element, and wherein the expression, disruption or alteration of said gene sequence results in said non-human animal exhibiting a predisposition to developing signs which are related to Alzheimer's disease.

**4.** The use of the non-human animal according to claim 3 wherein said signs comprise the formation of neurofibrillary tangles and/or wherein said animal is an insect or a rodent.

**5.** A use of a cell in which a gene sequence coding for a SLC39A12 protein, is mis-expressed, under-expressed, non-expressed, over-expressed, disrupted, or in another way altered for screening, testing, and/or validating compounds, agents, and modulators in the development of diagnostics and therapeutics useful for the treatment of Alzheimer's disease, wherein the SLC39A12 protein has SEQ ID NO: 1, or SEQ ID NO: 2, or SEQ ID NO: 3.

**6.** The use of a cell according to claim 5 wherein the altered expression, disruption, or alteration of said gene sequence results in said cell exhibiting a predisposition to developing signs which are related to Alzheimer's disease, and more particular signs which comprise the formation of paired helical filaments (PHF), and/or aggregation of tau protein, and/or phosphorylation of tau protein.

**7.** A method of screening for identifying agents, modulators, antagonists or agonists for use in the treatment or prevention of Alzheimer's disease, which agents, modulators, antagonists or agonists have an ability to alter level or activity of one or more substances selected from the group consisting of

(i) a transcription product of the gene coding for SLC39A12 proteins, and/or
(ii) a translation product of the gene coding for SLC39A12 proteins,

said method comprises:

(a) contacting a cell with a test compound;
(b) measuring the activity and/or level of one or more substances recited in (i) or (ii);
(c) measuring the activity and/or level of one or more substances recited in (i) or (ii) in a control cell not contacted with said test compound; and
comparing the levels and/or activities of the substances in the cells of step (b) and (c), wherein an alteration in the activity and/or level of the substances in the contacted cells indicates that the test compound is an agent, modulator, or antagonist or agonist for use in the treatment of Alzheimer's diseases, and wherein the SLC39A12 proteins have the SEQ ID NO: 1, or SEQ ID NO: 2, or SEQ ID NO: 3.

**8.** A method of screening for identifying agents, modulators, antagonists or agonists for use in the treatment of Alzheimer's disease, which agents, modulators, antagonists or agonists have an ability to alter level or activity of one or more substances selected from the group consisting of

(i) a transcription product of the gene coding for SLC39A12 proteins, and/or
(ii) a translation product of the gene coding for SLC39A12 proteins,

said method comprises:

(a) administering a test compound to a non-human test animal which is predisposed to developing or has already developed signs and symptoms of Alzheimer's disease;
(b) measuring the activity and/or level of one or more substances recited in (i) or (ii);
(c) measuring the activity and/or level of one or more substances recited in (i) or (ii) in a non-human control animal which is predisposed to developing or has already developed signs and symptoms of Alzheimer's disease or related diseases or disorders and to which non-human animal no such test compound has been administered;

comparing the activity and/or level of the substances in the animals of step (b) and (c), wherein an alteration in the activity and/or level of substances in the non-human test animal indicates that the test compound is an agent, modulator, antagonist or agonist for use in the treatment of Alzheimer's disease, and wherein the SLC39A12 proteins have SEQ ID NO: 1, or SEQ ID NO: 2, or SEQ ID NO: 3.

**9.** A use of an antibody specifically immunoreactive with an immunogen, wherein said immunogen is a translation product of a gene coding for SLC39A12 proteins, for detecting the pathological state of a cell in a sample obtained from a subject, comprising immunocytochemical staining of said cell with said antibody, wherein an altered degree of staining, or an altered staining pattern in said cell compared to a cell representing a known health status indicates a pathological state of said cell which relates to Alzheimer's disease, wherein the SLC39A12 proteins have SEQ ID NO: 1, or SEQ ID NO: 2, or SEQ ID NO: 3.

## Patentansprüche

**1.** Verfahren zum Diagnostizieren oder Prognostizieren einer neurodegenerativen Krankheit bei einem Patienten oder zum Bestimmen, ob ein Patient eine Prädisposition hat, diese Krankheit zu entwickeln, wobei das Verfahren die folgenden Schritte umfasst:

(a) Bestimmen einer Konzentration und/oder einer Aktivität von (i) einem Transcriptionsprodukt des Gens, das für SLC39A12-Proteine codiert, und/oder (ii) einem Translationsprodukt des Gens, das für SLC39A12-Proteine codiert, in einer von dem Patienten erhaltenen Probe;
(b) Vergleichen der Konzentration und/oder der Aktivität des Transcriptionsprodukts und/oder des Translationsprodukts mit einem Referenzwert, der einen bekannten Krankheitszustand darstellt und/oder einen bekannten Gesundheitszustand darstellt und/oder ein bekanntes Braak-Stadium darstellt;
(c) Analysieren, ob die Konzentration und/oder die Aktivität im Vergleich zu einem Referenzwert, der einen bekannten Gesundheitszustand darstellt, verändert ist und/oder ähnlich oder gleich ist wie ein Referenzwert, der einen bekannten Krankheitszustand darstellt oder ein bekanntes Braak-Stadium darstellt, was darauf hinweist, dass der Patient an einer neurodegenerativen Krankheit leidet oder dass der Patient ein erhöhtes Risiko hat, diese Krankheit zu entwickeln;
wobei es sich bei der neurodegenerativen Krankheit um Alzheimer-Krankheit handelt und wobei das Gen, das für SLC39A12-Proteine codiert, das Gen ist, das für ein SLC39A12-Protein mit SEQ ID Nr. 1 oder SEQ ID Nr. 2 oder SEQ ID Nr. 3 codiert, und wobei das Translationsprodukt des Gens, das für SLC39A12-Proteine codiert, das SLC39A12-Protein mit SEQ ID Nr. 1 oder SEQ ID Nr. 2 oder SEQ ID Nr. 3 ist.

**2.** Verwendung eines Kits zum Diagnostizieren oder Prognostizieren der Alzheimer-Krankheit bei einem Patienten oder zum Bestimmen der Prädisposition eines Patienten, diese Krankheit zu entwickeln, gemäß dem Verfahren von Anspruch 1, wobei der Kit wenigstens ein Reagens umfasst, das aus der Gruppe ausgewählt ist, die aus Reagentien besteht, die (i) ein Transcriptionsprodukt des Gens, das für SLC39A12-Proteine codiert, und/oder (ii) ein Translationsprodukt des Gens, das für SLC39A12-Proteine codiert, nachweisen, wobei die SLC39A12-Proteine die SEQ ID Nr. 1 oder SEQ ID Nr. 2 oder SEQ ID Nr. 3 aufweisen.

**3.** Verwendung eines genetisch modifizierten nichthumanen Tiers als nichthumanes Versuchstier und/oder Kontrolltier zum Screening, Testen und/oder Validieren von Verbindungen, Mitteln und Modulatoren bei der Entwicklung von Diagnostika und Therapeutika, die für die Behandlung von Alzheimer-Krankheit geeignet sind, wobei das genetisch modifizierte nichthumane Tier eine nichtnative Gensequenz, die für ein SLC39A12-Protein der SEQ ID Nr. 1 oder SEQ ID Nr. 2 oder SEQ ID Nr. 3 codiert, unter der Kontrolle eines Transcriptionselements, bei dem es sich nicht um das native Transcriptionskontrollelement des SLC39A12-Gens handelt, umfasst, wobei die Expression, Disruption oder Veränderung der Gensequenz dazu führt, dass das nichthumane Tier eine Prädisposition für die Entwicklung von Symptomen aufweist, die mit der Alzheimer-Krankheit zusammenhängen.

**4.** Verwendung des nichthumanen Tiers gemäß Anspruch 3, wobei die Symptome die Bildung von neurofibrillären Tangles umfassen und/oder wobei das Tier ein Insekt oder ein Nagetier ist.

**5.** Verwendung einer Zelle, in der eine für ein SLC39A12-Protein codierende Gensequenz falsch exprimiert, unterexprimiert, nicht exprimiert, überexprimiert, disrumpiert oder in anderer Weise verändert ist, zum Screening, Testen und/oder Validieren von Verbindungen, Mitteln und Modulatoren bei der Entwicklung von Diagnostika und Therapeutika, die für die Behandlung von Alzheimer-Krankheit geeignet sind, wobei das SLC39A12-Protein die SEQ ID Nr. 1 oder SEQ ID Nr. 2 oder SEQ ID Nr. 3 aufweist.

**6.** Verwendung einer Zelle gemäß Anspruch 5, wobei die veränderte Expression, Disruption oder Veränderung der Gensequenz dazu führt, dass die Zelle eine Prädisposition für die Entwicklung von Symptomen aufweist, die mit der Alzheimer-Krankheit zusammenhängen, insbesondere von Symptomen, die die Bildung von gepaarten helikalen Filamenten (PHF) und/oder die Aggregation des Tau-Proteins und/oder die Phosphorylierung des Tau-Proteins umfassen.

**7.** Verfahren zum Screening nach Mitteln, Modulatoren, Antagonisten oder Agonisten zur Verwendung bei der Behandlung oder Prävention von Alzheimer-Krankheit, wobei die Mittel, Modulatoren, Antagonisten oder Agonisten die Fähigkeit haben, die Konzentration oder Aktivität von einer oder mehreren Substanzen zu verändern, die aus der Gruppe ausgewählt sind, die aus Folgenden besteht:

(i) einem Transcriptionsprodukt des Gens, das für SLC39A12-Proteine codiert; und/oder
(ii) einem Translationsprodukt des Gens, das für SLC39A12-Proteine codiert;

wobei das Verfahren Folgendes umfasst:

(a) In-Kontakt-Bringen einer Zelle mit einer Testverbindung;
(b) Messen der Aktivität und/oder der Konzentration von einer oder mehreren Substanzen, die in (i) oder (ii) genannt sind;
(c) Messen der Aktivität und/oder der Konzentration von einer oder mehreren Substanzen, die in (i) oder (ii) genannt sind, in einer Kontrollzelle, die nicht mit der Testverbindung in Kontakt gebracht wurde; und

Vergleichen der Konzentrationen und/oder Aktivitäten der Substanzen in den Zellen der Schritte (b) und (c), wobei eine Änderung der Aktivität und/oder der Konzentration der Substanzen in den kontaktierten Zellen anzeigt, dass die Testverbindung ein Mittel, Modulator oder Antagonist oder Agonist zur Verwendung bei der Behandlung der Alzheimer-Krankheit ist, und wobei die SLC39A12-Proteine die SEQ ID Nr. 1 oder SEQ ID Nr. 2 oder SEQ ID Nr. 3 aufweisen.

**8.** Verfahren zum Screening nach Mitteln, Modulatoren, Antagonisten oder Agonisten zur Verwendung bei der Behandlung von Alzheimer-Krankheit, wobei die Mittel, Modulatoren, Antagonisten oder Agonisten die Fähigkeit haben, die Konzentration oder Aktivität von einer oder mehreren Substanzen zu verändern, die aus der Gruppe ausgewählt sind, die aus Folgenden besteht:

(i) einem Transcriptionsprodukt des Gens, das für SLC39A12-Proteine codiert; und/oder
(ii) einem Translationsprodukt des Gens, das für SLC39A12-Proteine codiert;

wobei das Verfahren Folgendes umfasst:

(a) Verabreichen einer Testverbindung an ein nichthumanes Versuchstier, das prädisponiert ist, Alzheimer-Krankheit zu entwickeln, oder das bereits Anzeichen und Symptome der Alzheimer-Krankheit entwickelt hat;
(b) Messen der Aktivität und/oder der Konzentration von einer oder mehreren Substanzen, die in (i) oder (ii) genannt sind;
(c) Messen der Aktivität und/oder der Konzentration von einer oder mehreren Substanzen, die in (i) oder (ii) genannt sind, in einem nichthumanen Kontrolltier, das prädisponiert ist, Alzheimer-Krankheit oder verwandte Krankheiten oder Störungen zu entwickeln, oder das bereits Anzeichen und Symptome solcher Krankheiten entwickelt hat, wobei dem nichthumanen Tier keine solche Testverbindung verabreicht wurde;

Vergleichen der Aktivität und/oder Konzentration der Substanzen in den Tieren der Schritte (b) und (c), wobei eine Änderung der Aktivität und/oder der Konzentration von Substanzen in dem nichthumanen Versuchstier anzeigt, dass die Testverbindung ein Mittel, Modulator, Antagonist oder Agonist zur Verwendung bei der Behandlung der Alzheimer-Krankheit ist, und wobei die SLC39A12-Proteine die SEQ ID Nr. 1 oder SEQ ID Nr. 2 oder SEQ ID Nr. 3 aufweisen.

**9.** Verwendung eines Antikörpers, der spezifisch immunreaktiv gegenüber einem Immunogen ist, wobei das Immunogen ein Translationsprodukt eines Gens, das für SLC39A12-Proteine codiert, ist, zum Nachweisen des pathologischen Zustands einer Zelle in einer Probe, die von einem Patienten erhalten wurde, umfassend das immuncytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand darstellt, einen pathologischen Zustand der Zelle anzeigt, der mit der Alzheimer-Krankheit zusammenhängt, wobei die SLC39A12-Proteine die SEQ ID Nr. 1 oder SEQ ID Nr. 2 oder SEQ ID Nr. 3 aufweisen.

## Revendications

**1.** Procédé pour diagnostiquer ou pronostiquer une maladie neurodégénérative chez un sujet, ou pour déterminer si un sujet présente une prédisposition à développer ladite maladie, lequel procédé comprend les étapes consistant à :

(a) déterminer un niveau et/ou d'une activité de (i) un produit de transcription du gène codant pour des protéines SLC39A12, et/ou (ii) un produit de traduction du gène codant pour des protéines SLC39A12, dans un échantillon obtenu dudit sujet,
(b) comparer ledit niveau et/ou ladite activité dudit produit de transcription et/ou dudit produit de traduction à une valeur de référence représentant un état de maladie connu et/ou représentant un état de santé connu et/ou représentant un stade de Braak connu,
(c) analyser si ledit niveau et/ou ladite activité diffère d'une valeur de référence représentant un état de santé connu, et/ou s'il est similaire ou égal à une valeur de référence représentant un état de maladie connu ou représentant un stade de Braak connu, ce qui est une indication que ledit sujet présente une maladie neurodégénérative, ou que ledit sujet présente un risque accru de développer ladite maladie,

dans lequel ladite maladie neurodégénérative est la maladie d'Alzheimer, et dans lequel ledit gène codant pour des protéines SLC39A12 est le gène codant pour une protéine SLC39A12 ayant la SEQ ID NO : 1, ou SEQ ID NO : 2, ou SEQ ID NO : 3 et dans lequel ledit produit de traduction du gène codant pour des protéines SLC39A12 est la protéine SLC39A12 ayant la SEQ ID NO : 1, ou SEQ ID NO : 2, ou SEQ ID NO : 3.

**2.** Utilisation d'un kit pour diagnostiquer ou pronostiquer la maladie d'Alzheimer, chez un sujet, ou pour déterminer la prédisposition d'un sujet à développer une telle maladie, selon le procédé de la revendication 1, le kit comprenant au moins un réactif qui est choisi dans le groupe consistant en les réactifs qui détectent (i) un produit de transcription du gène codant pour des protéines SLC39A12 et/ou (ii) un produit de traduction du gène codant pour des protéines SLC39A12, dans laquelle les protéines SLC39A12 ont la SEQ ID NO : 1, ou SEQ ID NO : 2, ou SEQ ID NO : 3.

**3.** Utilisation d'un animal non humain génétiquement modifié comme un animal de laboratoire non humain et/ou un animal témoin pour cribler, tester, et/ou valider des composés, agents, et modulateurs dans le développement de diagnostics et de thérapeutiques utiles dans le traitement de la maladie d'Alzheimer, dans laquelle l'animal non humain génétiquement modifié comprend une séquence génique non native codant pour une protéine SLC39A12 de SEQ ID NO : 1, ou SEQ ID NO : 2, ou SEQ ID NO : 3, sous le contrôle d'un élément de transcription qui n'est pas l'élément de contrôle de transcription natif du gène SLC39A12, et dans laquelle l'expression, la rupture ou l'altération de ladite séquence génique conduit à ce que l'animal non humain présente une prédisposition à développer des signes qui sont liés à la maladie d'Alzheimer.

**4.** Utilisation de l'animal non humain selon la revendication 3, dans laquelle lesdits signes comprennent la formation d'enchevêtrements neurofibrillaires et/ou dans laquelle ledit animal est un insecte ou un rongeur.

**5.** Utilisation d'une cellule dans laquelle une séquence génique codant pour une protéine SLC39A12, est anormalement exprimée, sous-exprimée, non exprimée, surexprimée, interrompue ou altérée d'une autre façon pour le criblage, le test, et/ou la validation de composés, agents, et modulateurs dans le développement de diagnostics et de thérapeutiques utiles dans le traitement de la maladie d'Alzheimer, dans laquelle la protéine SLC39A12 a la SEQ ID NO : 1, ou SEQ ID NO : 2, ou SEQ ID NO : 3.

**6.** Utilisation d'une cellule selon la revendication 5, dans laquelle l'expression altérée, la rupture, ou l'altération de ladite séquence génique conduit à ce que ladite cellule présente une prédisposition à développer des signes liés à la maladie d'Alzheimer, et plus particulièrement des signes qui comprennent la formation de paires de filaments hélicoïdaux (PFH), et/ou l'agrégation de la protéine tau, et/ou la phosphorylation de la protéine tau.

**7.** Procédé de criblage pour l'identification d'agents, de modulateurs, d'antagonistes ou d'agonistes à utiliser dans le traitement ou la prévention de la maladie d'Alzheimer, lesquels agents, modulateurs, antagonistes ou agonistes ont une capacité à altérer le niveau ou l'activité d'une ou de plusieurs substances choisies dans le groupe consistant en

(i) un produit de transcription du gène codant pour des protéines SLC39A12, et/ou
(ii) un produit de traduction du gène codant pour des protéines SLC39A12,

ledit procédé comprend :

(a) la mise en contact d'une cellule avec un composé d'essai ;
(b) la mesure de l'activité et/ou du niveau d'une ou de plusieurs substances citées en (i) ou (ii) ;
(c) la mesure de l'activité et/ou du niveau d'une ou plusieurs substances citées en (i) ou (ii) dans une cellule témoin n'ayant pas été mise en contact avec ledit composé d'essai ; et

la comparaison des niveaux et/ou des activités des substances dans les cellules des étapes (b) et (c), dans laquelle une altération dans l'activité et/ou le niveau des substances dans les cellules ayant été mises en contact indique que le composé d'essai est un agent, un modulateur, ou un antagoniste ou agoniste à utiliser dans le traitement de la maladie d'Alzheimer, et dans laquelle les protéines SLC39A12 ont la SEQ ID NO : 1, ou SEQ ID NO : 2, ou SEQ ID NO : 3.

**8.** Procédé de criblage pour l'identification d'agents, de modulateurs, d'antagonistes ou d'agonistes à utiliser dans le traitement de la maladie d'Alzheimer, lesquels agents, modulateurs, antagonistes ou agonistes ont une capacité à altérer le niveau ou l'activité d'une ou de plusieurs substances choisies dans le groupe consistant en

(i) un produit de transcription du gène codant pour des protéines SLC39A12, et/ou
(ii) un produit de traduction du gène codant pour des protéines SLC39A12,

ledit procédé comprend :

(a) l'administration d'un composé d'essai à un animal de laboratoire non humain qui est prédisposé à développer ou a déjà développé des signes et des symptômes de la maladie d'Alzheimer ;
(b) la mesure de l'activité et/ou du niveau d'une ou de plusieurs substances citées en (i) ou (ii) ;
(c) la mesure de l'activité et/ou du niveau d'une ou de plusieurs substances citées en (i) ou (ii) chez un animal témoin non humain qui est prédisposé à développer ou a déjà développé des signes et des symptômes de la maladie d'Alzheimer ou de maladies ou troubles apparentés et auquel animal non humain on n'a pas administré un tel composé d'essai ;

la comparaison de l'activité et/ou du niveau des substances dans les animaux des étapes (b) et (c), dans laquelle une altération dans l'activité et/ou le niveau des substances chez l'animal de laboratoire non humain indique que le composé d'essai est un agent, un modulateur, un antagoniste ou agoniste à utiliser dans le traitement de la maladie d'Alzheimer, et dans laquelle les protéines SLC39A12 ont la SEQ ID NO : 1, ou SEQ ID NO : 2, ou SEQ ID NO : 3.

**9.** Utilisation d'un anticorps spécifiquement immunoréactif avec un immunogène, dans laquelle ledit immunogène est un produit de traduction d'un gène codant pour des protéines SLC39A12, pour la détection de l'état pathologique d'une cellule dans un échantillon obtenu d'un sujet, comprenant la coloration immunocytochimique de ladite cellule avec ledit anticorps, dans laquelle un degré altéré de coloration, ou un schéma de coloration altéré dans ladite cellule comparée à une cellule représentant un état de santé connu indique un état pathologique de ladite cellule qui est lié à la maladie d'Alzheimer, dans laquelle les protéines SLC39A12 ont la SEQ ID NO : 1, ou SEQ ID NO : 2, ou SEQ ID NO : 3.

Fig. 1: Identification of the differentially expressed gene SLC39A12 by GeneChip microarray analysis

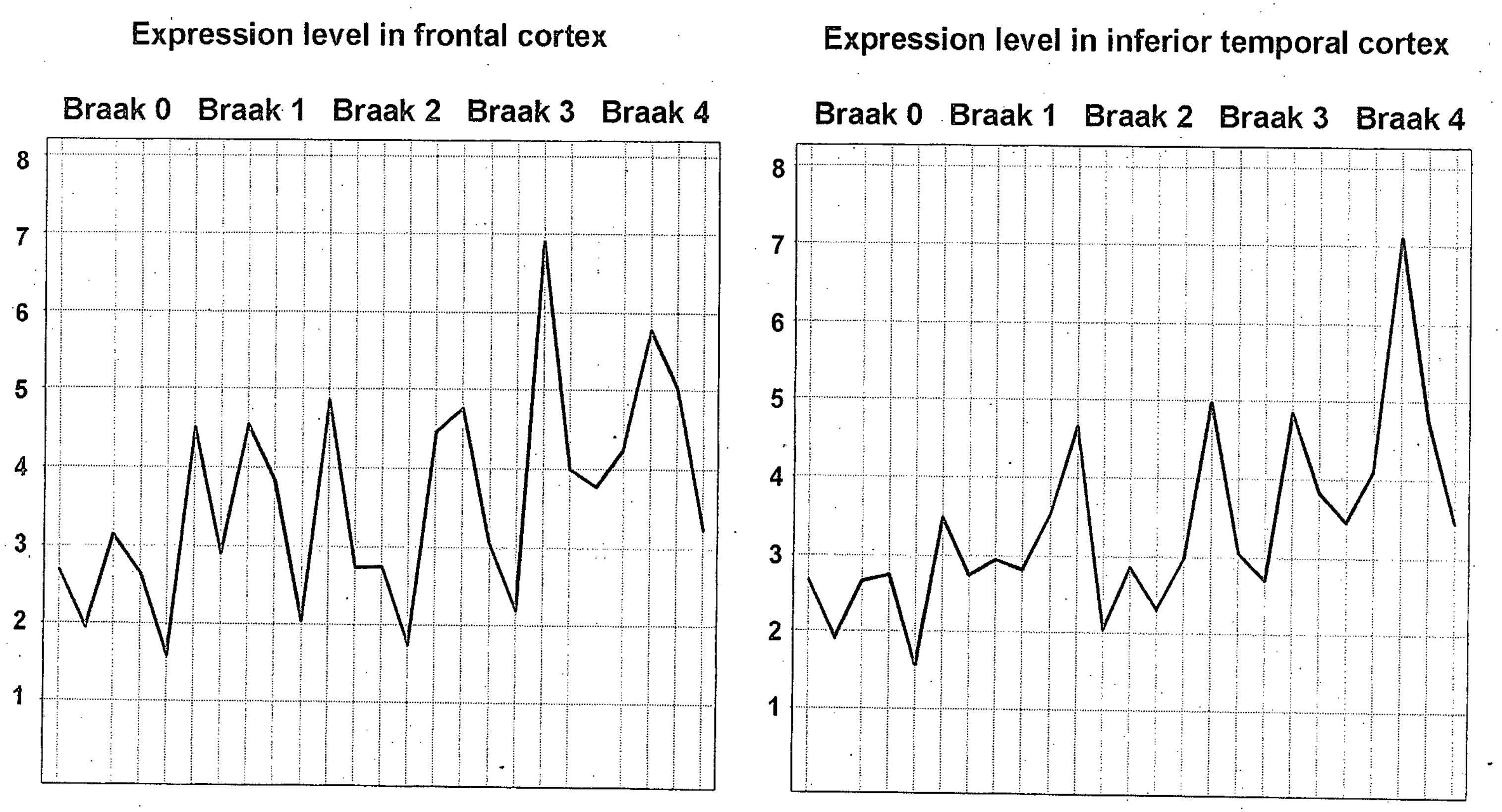

# Fig. 2: Analysis of SLC39A12 mRNA expression by quantitative PCR

measured data of SLC39A12 mRNA expression in frontal and inferior temporal cortices in Braak stages 0 – 4

| Donor | Braak stage | Frontal | Temporal |
|---|---|---|---|
| C011 | 0 | 103.1142 | 106.2932 |
| C012 | 0 | 97.1351 | 90.0772 |
| C026 | 0 | 126.1639 | 106.1629 |
| C027 | 0 | 86.2150 | 98.6066 |
| C032 | 0 | 55.5529 | 79.0464 |
| C014 | 1 | 218.2695 | 215.6391 |
| C028 | 1 | 90.7566 | 80.7541 |
| C029 | 1 | 215.5649 | 143.4554 |
| C030 | 1 | 188.1479 | 132.2315 |
| C036 | 1 | 94.7264 | 107.2652 |
| C038 | 1 | 242.6585 | 202.8123 |
| C039 | 1 | 116.0763 | 93.9295 |
| C008 | 2 | 152.1449 | 123.6155 |
| C031 | 2 | 81.0309 | 127.1986 |
| C033 | 2 | 181.4532 | 115.3717 |
| C034 | 2 | 242.0141 | 196.7186 |
| DE03 | 2 | 168.6214 | 171.4658 |
| C025 | 3 | 112.5489 | 141.7494 |
| DE07 | 3 | 287.8354 | 164.4535 |
| DE11 | 3 | 137.5298 | 137.2312 |
| C057 | 3 | 149.7625 | 159.1204 |
| P012 | 4 | 133.3521 | 150.9728 |
| P046 | 4 | 280.4666 | 283.5425 |
| P047 | 4 | 199.2770 | 158.3715 |
| P068 | 4 | 94.5341 | 105.1687 |

Fig. 3:Statistical analysis of differential SLC39A12 mRNA expression

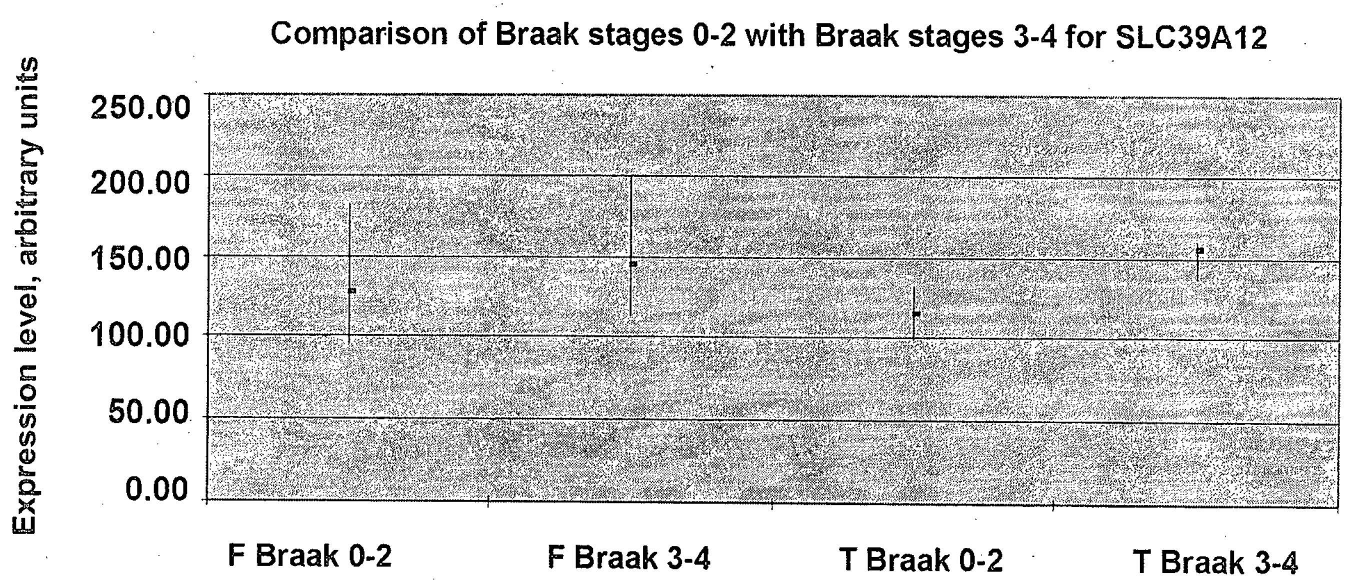

# Fig. 4 : A) SEQ ID NO: 1, amino acid sequence of human SLC39A12 sv1 protein

**Length: 691 aa**

| | | | | | |
|---|---|---|---|---|---|
| 1 | MCFRTKLSVS | WVPLFLLLSR | VFSTETDKPS | AQDSRSRGSS | GQPADLLQVL |
| 51 | SAGDHPPHNH | SRSLIKTLLE | KTGCPRRRNG | MQGDCNLCFE | PDALLLIAGG |
| 101 | NFEDQLREEV | VQRVSLLLLY | YIIHQEEICS | SKLNMSNKEY | KFYLHSLLSL |
| 151 | RQDEDSSFLS | QNETEDILAF | TRQYFDTSQS | QCMETKTLQK | KSGIVSSEGA |
| 201 | NESTLPQLAA | MIITLSLQGV | CLGQGNLPSP | DYFTEYIFSS | LNRTNTLRLS |
| 251 | ELDQLLNTLW | TRSTCIKNEK | IHQFQRKQNN | IITHDQDYSN | FSSSMEKESE |
| 301 | DGPVSWDQTC | FSARQLVEIF | LQKGLSLISK | EDFKQMSPGI | IQQLLSCSCH |
| 351 | LPKDQQAKLP | PTTLEKYGYS | TVAVTLLTLG | SMLGTALVLF | HSCEENYRLI |
| 401 | LQLFVGLAVG | TLSGDALLHL | IPQVLGLHKQ | EAPEFGHFHE | SKGHIWKLMG |
| 451 | LIGGIHGFFL | IEKCFILLVS | PNDKQGLSLV | NGHVGHSHHL | ALNSELSDQA |
| 501 | GRGKSASTIQ | LKSPEDSQAA | EMPIGSMTAS | NRKCKAISLL | AIMILVGDSL |
| 551 | HNFADGLAIG | AAFSSSSESG | VTTTIAILCH | EIPHEMGDFA | VLLSSGLSMK |
| 601 | TAILMNFISS | LTAFMGLYIG | LSVSADPCVQ | DWIFTVTAGM | FLYLSLVEML |
| 651 | PEMTHVQTQR | PWMMFLLQNF | GLILGWLSLL | LLAIYEQNIK | I |

## Fig. 4 : B) SEQ ID NO: 2, amino acid sequence of human SLC39A12 sv2 protein

**Length: 690 aa**

```
  1  MCFRTKLSVS WVPLFLLLSR VFSTETDKPS AQDSRSRGSS GQPADLLQVL
 51  SAGDHPPHNH SRSLIKTLLE KTGCPRRRNG MQGDCNLCFE PDALLLIAGG
101  NFEDQLREEV VQRVSLLLLY YIIHQEEICS SKLNMSNKEY KFYLHSLLSL
151  RQDEDSSFLS QNETEDILAF TRQYFDTSQS QCMETKTLQK KSGIVSSEGA
201  NESTLPQLAA MIITLSLQGV CLGQGNLPSP DYFTEYIFSS LNRTNTLRLS
251  ELDQLLNTLW TRSTCIKNEK IHQFQRKQNN IITHDQDYSN FSSSMEKESE
301  DGPVSWDQTC FSARQLVEIF LQKGLSLISK EDFKQMSPGI IQQLLSCSCH
351  LPKDQQAKLP PTTLEKYGYS TVAVTLLTLG SMLGTALVLF HSCEENYRLI
401  LQLFVGLAVG TLSGDALLHL IPQVLGLHKQ EAPEFGHFHE SKGHIWKLMG
451  LIGGIHGFFL IEKCFILLVS PNDKGLSLVN GHVGHSHHLA LNSELSDQAG
501  RGKSASTIQL KSPEDSQAAE MPIGSMTASN RKCKAISLLA IMILVGDSLH
551  NFADGLAIGA AFSSSSESGV TTTIAILCHE IPHEMGDFAV LLSSGLSMKT
601  AILMNFISSL TAFMGLYIGL SVSADPCVQD WIFTVTAGMF LYLSLVEMLP
651  EMTHVQTQRP WMMFLLQNFG LILGWLSLLL LAIYEQNIKI
```

## Fig. 4 : C) SEQ ID NO: 3, amino acid sequence of human SLC39A12 sv3 protein

**Length: 654 aa**

```
  1  MCFRTKLSVS WVPLFLLLSR VFSTETDKPS AQDSRSRGSS GQPADLLQVL
 51  SAGDHPPHNH SRSLIKTLLE KTGCPRRRNG MQGDCNLCFE PDALLLIAGG
101  NFEDQLREEV VQRVSLLLLY YIIHQEEICS SKLNMSNKEY KFYLHSLLSL
151  RQDEDSSFLS QNETEDILAF TRQYFDTSQS QCMETKTLQK KSGIVSSEGA
201  NESTLPQLAA MIITLSLQGV CLGQGNLPSP DYFTEYIFSS LNRTNTLRLS
251  ELDQLLNTLW TRSTCIKNEK IHQFQRKQNN IITHDQDYSN FSSSMEKESE
301  DGPVSWDQTC FSARQLVEIF LQKGLSLISK EDFKQMSPGI IQQLLSCSCH
351  LPKDQQAKLP PTTLEKYGYS TVAVTLLTLG SMLGTALVLF HSCEENYRLI
401  LQLFVGLAVG TLSGDALLHL IPQVLGLHKQ EAPEFGHFHE SKGHIWKLMG
451  LIGGIHGFFL IEKCFILLVS PNDKKSPEDS QAAEMPIGSM TASNRKCKAI
501  SLLAIMILVG DSLHNFADGL AIGAAFSSSS ESGVTTTIAI LCHEIPHEMG
551  DFAVLLSSGL SMKTAILMNF ISSLTAFMGL YIGLSVSADP CVQDWIFTVT
601  AGMFLYLSLV EMLPEMTHVQ TQRPWMMFLL QNFGLILGWL SLLLLAIYEQ
651  NIKI
```

# Fig. 5 : A) SEQ ID NO: 4, nucleotide sequence of human SLC39A12 sv1 cDNA

**Length: 2678 bp**

```
   1 ACTCTCCAGG ATTTAAAAGG CTGTGGAGCT CCAGATAAAG AATCGTTTAT
  51 CTTTCTTCTG AAGAAATTCC TTTGGTTACA AGTTTACCCC ATAAACGGCA
 101 ACACACTCAC CTCCATCCAA GACAGACTCA AGGTGGAGGA AGCGTGGAAA
 151 TGTGCTTCCG GACAAAGCTC TCAGTATCCT GGGTGCCATT GTTTCTTCTA
 201 CTCAGCCGTG TTTTTTCTAC TGAGACAGAC AAACCCTCAG CCCAGGATAG
 251 CAGAAGCCGT GGGAGTTCAG GCCAACCGGC AGACCTGCTA CAGGTTCTCT
 301 CTGCTGGTGA CCACCCACCC CACAACCACT CAAGAAGCCT CATCAAAACA
 351 TTGTTGGAGA AAACTGGGTG CCCACGGAGG AGAAACGGAA TGCAAGGAGA
 401 TTGCAATCTG TGCTTTGAAC CAGATGCACT ATTACTAATA GCTGGAGGAA
 451 ATTTTGAAGA TCAGCTTAGA GAAGAAGTGG TCCAGAGAGT TTCTCTTCTC
 501 CTTCTCTATT ACATTATTCA TCAGGAAGAG ATCTGTTCTT CAAAGCTCAA
 551 CATGAGTAAT AAAGAGTATA AATTTTACCT ACACAGCCTA CTGAGCCTCA
 601 GGCAGGATGA AGATTCCTCT TTCCTTTCAC AGAATGAGAC AGAAGATATC
 651 TTGGCTTTCA CCAGGCAGTA CTTTGACACT TCTCAAAGCC AGTGTATGGA
 701 AACCAAAACG CTGCAGAAAA AATCTGGAAT AGTGAGCAGT GAAGGTGCTA
 751 ATGAAAGTAC GCTTCCTCAG TTGGCAGCCA TGATCATTAC TTTGTCCCTC
 801 CAGGGTGTTT GTCTGGGACA AGGAAACTTG CCTTCCCCAG ACTACTTTAC
 851 AGAATATATT TTCAGTTCCT TGAATCGTAC GAATACCCTC CGCCTATCAG
 901 AACTAGACCA ACTCCTCAAC ACTCTCTGGA CCAGAAGTAC TTGTATCAAA
 951 AATGAGAAAA TCCATCAATT TCAAAGGAAA CAAAACAACA TAATAACCCA
1001 TGATCAGGAC TATTCTAATT TCTCTTCATC CATGGAAAAA GAGTCTGAGG
1051 ATGGTCCAGT TTCCTGGGAT CAGACCTGCT TCTCTGCTAG GCAGCTGGTG
1101 GAGATATTTC TACAGAAGGG CCTCTCACTC ATTTCTAAGG AGGACTTTAA
1151 GCAAATGAGT CCAGGGATCA TCCAGCAGCT CCTCAGCTGC TCCTGCCACT
1201 TACCCAAGGA CCAACAAGCA AAGCTGCCAC CTACCACTCT GGAGAAATAC
1251 GGCTACAGCA CGGTGGCTGT CACCCTTCTC ACACTGGGCT CCATGCTGGG
1301 GACAGCGCTG GTCCTTTTCC ATAGCTGTGA GGAGAACTAC AGGCTTATCT
1351 TACAGCTGTT TGTGGGCTTG GCCGTCGGGA CACTGTCTGG GGACGCTCTG
1401 CTCCACCTTA TCCCTCAGGT TCTTGGTTTA CATAAGCAGG AAGCCCCAGA
1451 ATTTGGGCAT TTCCATGAAA GCAAAGGTCA TATTTGGAAA CTGATGGGAT
1501 TAATTGGAGG CATCCATGGA TTTTTCTTGA TAGAAAAATG TTTTATTCTT
1551 CTTGTATCAC CAAATGACAA GCAGGGCCTG TCATTGGTTA ATGGGCACGT
1601 GGGTCATTCC CACCATCTTG CACTCAACTC TGAATTAAGT GACCAGGCAG
1651 GCAGAGGCAA ATCTGCTTCA ACTATCCAGT TGAAAAGCCC AGAAGATTCA
1701 CAGGCAGCTG AAATGCCTAT AGGCAGTATG ACAGCCTCCA ACAGAAAATG
1751 TAAAGCCATT AGCTTGTTAG CAATCATGAT TCTGGTTGGG GACAGCCTGC
1801 ATAATTTTGC AGATGGCCTA GCCATAGGAG CAGCCTTCTC ATCATCATCC
1851 GAGTCAGGAG TGACCACTAC GATTGCTATC TTGTGTCATG AAATCCCACA
1901 TGAAATGGGA GACTTTGCCG TGCTCTTAAG CTCTGGACTT TCTATGAAGA
1951 CTGCCATCCT GATGAATTTT ATAAGCTCCC TAACTGCCTT CATGGGATTA
2001 TACATTGGCC TTTCCGTGTC AGCTGATCCA TGTGTTCAAG ACTGGATCTT
2051 CACAGTCACT GCTGGGATGT TCTTATATTT ATCCTTGGTT GAAATGCTTC
2101 CTGAAATGAC TCATGTTCAA ACACAACGAC CCTGGATGAT GTTTCTCCTG
2151 CAAAACTTTG GATTGATCCT AGGTTGGCTT TCTCTCCTGC TCTTGGCTAT
2201 ATATGAGCAA AATATTAAAA TATAAGTGAG GATCTTCAAC ATCTTTCAAA
2251 AATGCATTTA TATAGTCTTA CTTTGTTTCT TTCATTGCAC TCTATAATGA
```

```
2301  TTTTTAAATT AAGAATTTTT TATCTTAGGC AAAGTGTGTC TCTTTCAATT
2351  CATTAACTTA TTAATTTTAT AATGCAGTTT TATTTTTGGA AACATATAAA
2401  TATCAGACTG TCCTTAATTG AAATTTTGTC TTTGGTTTCC AACACCATGA
2451  TGAAGCTCTT GCTTTTTAAA AAGTAGTTAG TAAATTCTGC ATGAATTTTA
2501  GTAAACTTTA AAAAATAGAT TTTTTCCCTA AGAAAGAATG TTTGTAGAAT
2551  TTAAAGTGGA CAGATGCCTG TTGGAGTAAA ATCAACTGCA ACTTTTTGAT
2601  GTTAATTTTT TTCCCTGTGC AATTATAAAC TATAAGCAAG TTAAGTGACA
2651  AGCAAATGTA ATAAAGACTA GTTTTAAT
```

# Fig. 5 : B) SEQ ID NO: 5, nucleotide sequence of human SLC39A12 sv2 cDNA

**Length: 2725 bp**

```
   1 GTGCACAGTC AAAGAATTTT AAAAACAGGG AACTTTGTAA CTGTGAAATA
  51 CTCTCCAGGA TTTAAAAGGC TGTGGAGCTC CAGATAAAGA ATCGTTTATC
 101 TTTCTTCTGA AGAAATTCCT TTGGTTACAA GTTTACCCCA TAAACGGCAA
 151 CACACTCACC TCCATCCAAG ACAGACTCAA GGTGGAGGAA GCGTGGAAAT
 201 GTGCTTCCGG ACAAAGCTCT CAGTATCCTG GGTGCCATTG TTTCTTCTAC
 251 TCAGCCGTGT TTTTTCTACT GAGACAGACA AACCCTCAGC CCAGGATAGC
 301 AGAAGCCGTG GGAGTTCAGG CCAACCGGCA GACCTGCTAC AGGTTCTCTC
 351 TGCTGGTGAC CACCCACCCC ACAACCACTC AAGAAGCCTC ATCAAAACAT
 401 TGTTGGAGAA AACTGGGTGC CCACGGAGGA GAAACGGAAT GCAAGGAGAT
 451 TGCAATCTGT GCTTTGAACC AGATGCACTA TTACTAATAG CTGGAGGAAA
 501 TTTTGAAGAT CAGCTTAGAG AAGAAGTGGT CCAGAGAGTT TCTCTTCTCC
 551 TTCTCTATTA CATTATTCAT CAGGAAGAGA TCTGTTCTTC AAAGCTCAAC
 601 ATGAGTAATA AAGAGTATAA ATTTTACCTA CACAGCCTAC TGAGCCTCAG
 651 GCAGGATGAA GATTCCTCTT TCCTTTCACA GAATGAGACA GAAGATATCT
 701 TGGCTTTCAC CAGGCAGTAC TTTGACACTT CTCAAAGCCA GTGTATGGAA
 751 ACCAAAAACGC TGCAGAAAAA ATCTGGAATA GTGAGCAGTG AAGGTGCTAA
 801 TGAAAGTACG CTTCCTCAGT TGGCAGCCAT GATCATTACT TTGTCCCTCC
 851 AGGGTGTTTG TCTGGGACAA GGAAACTTGC CTTCCCCAGA CTACTTTACA
 901 GAATATATTT TCAGTTCCTT GAATCGTACG AATACCCTCC GCCTATCAGA
 951 ACTAGACCAA CTCCTCAACA CTCTCTGGAC CAGAAGTACT TGTATCAAAA
1001 ATGAGAAAAT CCATCAATTT CAAAGGAAAC AAAACAACAT AATAACCCAT
1051 GATCAGGACT ATTCTAATTT CTCTTCATCC ATGGAAAAAG AGTCTGAGGA
1101 TGGTCCAGTT TCCTGGGATC AGACCTGCTT CTCTGCTAGG CAGCTGGTGG
1151 AGATATTTCT ACAGAAGGGC CTCTCACTCA TTTCTAAGGA GGACTTTAAG
1201 CAAATGAGTC CAGGGATCAT CCAGCAGCTC CTCAGCTGCT CCTGCCACTT
1251 ACCCAAGGAC CAACAAGCAA AGCTGCCACC TACCACTCTG GAGAAATACG
1301 GCTACAGCAC GGTGGCTGTC ACCCTTCTCA CACTGGGCTC CATGCTGGGG
1351 ACAGCGCTGG TCCTTTTCCA TAGCTGTGAG GAGAACTACA GGCTTATCTT
1401 ACAGCTGTTT GTGGGCTTGG CCGTCGGGAC ACTGTCTGGG GACGCTCTGC
1451 TCCACCTTAT CCCTCAGGTT CTTGGTTTAC ATAAGCAGGA AGCCCCAGAA
1501 TTTGGGCATT TCCATGAAAG CAAAGGTCAT ATTTGGAAAC TGATGGGATT
1551 AATTGGAGGC ATCCATGGAT TTTTCTTGAT AGAAAAAATGT TTTATTCTTC
1601 TTGTATCACC AAATGACAAG GGCCTGTCAT TGGTTAATGG GCACGTGGGT
1651 CATTCCCACC ATCTTGCACT CAACTCTGAA TTAAGTGACC AGGCAGGCAG
1701 AGGCAAATCT GCTTCAACTA TCCAGTTGAA AAGCCCAGAA GATTCACAGG
1751 CAGCTGAAAT GCCTATAGGC AGTATGACAG CCTCCAACAG AAAATGTAAA
1801 GCCATTAGCT TGTTAGCAAT CATGATTCTG GTTGGGGACA GCCTGCATAA
1851 TTTTGCAGAT GGCCTAGCCA TAGGAGCAGC CTTCTCATCA TCATCCGAGT
1901 CAGGAGTGAC CACTACGATT GCTATCTTGT GTCATGAAAT CCCACATGAA
1951 ATGGGAGACT TTGCCGTGCT CTTAAGCTCT GGACTTTCTA TGAAGACTGC
2001 CATCCTGATG AATTTTATAA GCTCCCTAAC TGCCTTCATG GGATTATACA
2051 TTGGCCTTTC CGTGTCAGCT GATCCATGTG TTCAAGACTG GATCTTCACA
2101 GTCACTGCTG GGATGTTCTT ATATTTATCC TTGGTTGAAA TGCTTCCTGA
2151 AATGACTCAT GTTCAAACAC AACGACCCTG GATGATGTTT CTCCTGCAAA
2201 ACTTTGGATT GATCCTAGGT TGGCTTTCTC TCCTGCTCTT GGCTATATAT
2251 GAGCAAAATA TTAAAATATA AGTGAGGATC TTCAACATCT TTCAAAAATG
```

```
2301  CATTTATATA GTCTTACTTT GTTTCTTTCA TTGCACTCTA TAATGATTTT
2351  TAAATTAAGA ATTTTTTATC TTAGGCAAAG TGTGTCTCTT TCAATTCATT
2401  AACTTATTAA TTTTATAATG CAGTTTTATT TTTGGAAACA TATAAATATC
2451  AGACTGTCCT TAATTGAAAT TTTGTCTTTG GTTTCCAACA CCATGATGAA
2501  GCTCTTGCTT TTTAAAAAGT AGTTAGTAAA TTCTGCATGA ATTTTAGTAA
2551  ACTTTAAAAA ATAGATTTTT TCCCTAAGAA AGAATGTTTG TAGAATTTAA
2601  AGTGGACAGA TGCCTGTTGG AGTAAAATCA ACTGCAACTT TTTGATGTTA
2651  ATTTTTTTCC CTGTGCAATT ATAAACTATA AGCAAGTTAA GTGACAAGCA
2701  AATGTAATAA AGACTAGTTT TAATA
```

## Fig. 5 : C) SEQ ID NO: 6, nucleotide sequence of human SLC39A12 sv3 cDNA

**Length: 2635 bp**

```
   1 AAATAATCCT CTCTAGCTCC CAGTGCACAG TCAAAGAATT TTAAAAACAG
  51 GGAACTTTGT AACTGTGAAA TACTCTCCAG GATTTAAAAG GCTGTGGAGC
 101 TCCAGATAAA GAATCGTTTA TCTTTCTTCT GAAGAAATTC CTTTGGTTAC
 151 AAGTTTACCC CATAAACGGC AACACACTCA CCTCCATCCA AGACAGACTC
 201 AAGGTGGAGG AAGCGTGGAA ATGTGCTTCC GGACAAAGCT CTCAGTATCC
 251 TGGGTGCCAT TGTTTCTTCT ACTCAGCCGT GTTTTTTCTA CTGAGACAGA
 301 CAAACCCTCA GCCCAGGATA GCAGAAGCCG TGGGAGTTCA GGCCAACCGG
 351 CAGACCTGCT ACAGGTTCTC TCTGCTGGTG ACCACCCACC CCACAACCAC
 401 TCAAGAAGCC TCATCAAAAC ATTGTTGGAG AAAACTGGGT GCCCACGGAG
 451 GAGAAACGGA ATGCAAGGAG ATTGCAATCT GTGCTTTGAA CCAGATGCAC
 501 TATTACTAAT AGCTGGAGGA AATTTTGAAG ATCAGCTTAG AGAAGAAGTG
 551 GTCCAGAGAG TTTCTCTTCT CCTTCTCTAT TACATTATTC ATCAGGAAGA
 601 GATCTGTTCT TCAAAGCTCA ACATGAGTAA TAAAGAGTAT AAATTTTACC
 651 TACACAGCCT ACTGAGCCTC AGGCAGGATG AAGATTCCTC TTTCCTTTCA
 701 CAGAATGAGA CAGAAGATAT CTTGGCTTTC ACCAGGCAGT ACTTTGACAC
 751 TTCTCAAAGC CAGTGTATGG AAACCAAAAC GCTGCAGAAA AAATCTGGAA
 801 TAGTGAGCAG TGAAGGTGCT AATGAAAGTA CGCTTCCTCA GTTGGCAGCC
 851 ATGATCATTA CTTTGTCCCT CCAGGGTGTT TGTCTGGGAC AAGGAAACTT
 901 GCCTTCCCCA GACTACTTTA CAGAATATAT TTTCAGTTCC TTGAATCGTA
 951 CGAATACCCT CCGCCTATCA GAACTAGACC AACTCCTCAA CACTCTCTGG
1001 ACCAGAAGTA CTTGTATCAA AAATGAGAAA ATCCATCAAT TTCAAAGGAA
1051 ACAAAACAAC ATAATAACCC ATGATCAGGA CTATTCTAAT TTCTCTTCAT
1101 CCATGGAAAA AGAGTCTGAG GATGGTCCAG TTTCCTGGGA TCAGACCTGC
1151 TTCTCTGCTA GGCAGCTGGT GGAGATATTT CTACAGAAGG GCCTCTCACT
1201 CATTTCTAAG GAGGACTTTA AGCAAATGAG TCCAGGGATC ATCCAGCAGC
1251 TCCTCAGCTG CTCCTGCCAC TTACCCAAGG ACCAACAAGC AAAGCTGCCA
1301 CCTACCACTC TGGAGAAATA CGGCTACAGC ACGGTGGCTG TCACCCTTCT
1351 CACACTGGGC TCCATGCTGG GGACAGCGCT GGTCCTTTTC CATAGCTGTG
1401 AGGAGAACTA CAGGCTTATC TTACAGCTGT TTGTGGGCTT GGCCGTCGGG
1451 ACACTGTCTG GGGACGCTCT GCTCCACCTT ATCCCTCAGG TTCTTGGTTT
1501 ACATAAGCAG GAAGCCCCAG AATTTGGGCA TTTCCATGAA AGCAAAGGTC
1551 ATATTTGGAA ACTGATGGGA TTAATTGGAG GCATCCATGG ATTTTTCTTG
1601 ATAGAAAAAT GTTTTATTCT TCTTGTATCA CCAAATGACA AGAAAAGCCC
1651 AGAAGATTCA CAGGCAGCTG AAATGCCTAT AGGCAGTATG ACAGCCTCCA
1701 ACAGAAAATG TAAAGCCATT AGCTTGTTAG CAATCATGAT TCTGGTTGGG
1751 GACAGCCTGC ATAATTTTGC AGATGGCCTA GCCATAGGAG CAGCCTTCTC
1801 ATCATCATCC GAGTCAGGAG TGACCACTAC GATTGCTATC TTGTGTCATG
1851 AAATCCCACA TGAAATGGGA GACTTTGCCG TGCTCTTAAG CTCTGGACTT
1901 TCTATGAAGA CTGCCATCCT GATGAATTTT ATAAGCTCCC TAACTGCCTT
1951 CATGGGATTA TACATTGGCC TTTCCGTGTC AGCTGATCCA TGTGTTCAAG
2001 ACTGGATCTT CACAGTCACT GCTGGGATGT TCTTATATTT ATCCTTGGTT
2051 GAAATGCTTC CTGAAATGAC TCATGTTCAA ACACAACGAC CCTGGATGAT
2101 GTTTCTCCTG CAAAACTTTG GATTGATCCT AGGTTGGCTT TCTCTCCTGC
2151 TCTTGGCTAT ATATGAGCAA AATATTAAAA TATAAGTGAG GATCTTCAAC
2201 ATCTTTCAAA AATGCATTTA TATAGTCTTA CTTTGTTTCT TTCATTGCAC
```

```
2251  TCTATAATGA TTTTTAAATT AAGAATTTTT TATCTTAGGC AAAGTGTGTC
2301  TCTTTCAATT CATTAACTTA TTAATTTTAT AATGCAGTTT TATTTTTGGA
2351  AACATATAAA TATCAGACTG TCCTTAATTG AAATTTTGTC TTTGGTTTCC
2401  AACACCATGA TGAAGCTCTT GCTTTTTAAA AAGTAGTTAG TAAATTCTGC
2451  ATGAATTTTA GTAAACTTTA AAAAATAGAT TTTTTCCCTA AGAAAGAATG
2501  TTTGTAGAAT TTAAAGTGGA CAGATGCCTG TTGGAGTAAA ATCAACTGCA
2551  ACTTTTTGAT GTTAATTTTT TTCCCTGTGC AATTATAAAC TATAAGCAAG
2601  TTAAGTGACA AGCAAATGTA ATAAAGACTA GTTTT
```

## Fig. 6 : A) SEQ ID NO: 7, coding nucleotide sequence of human SLC39A12 sv1 cDNA

**Length: 2076 bp**

```
   1 ATGTGCTTCC GGACAAAGCT CTCAGTATCC TGGGTGCCAT TGTTTCTTCT
  51 ACTCAGCCGT GTTTTTTCTA CTGAGACAGA CAAACCCTCA GCCCAGGACA
 101 GCAGAAGCCG TGGGAGTTCA GGCCAACCGG CAGACCTGCT ACAGGTTCTC
 151 TCTGCTGGTG ACCACCCACC CCACAACCAC TCAAGAAGCC TCATCAAAAC
 201 ATTGTTGGAG AAAACTGGGT GCCCACGGAG GAGAAACGGA ATGCAAGGAG
 251 ATTGCAATCT GTGCTTTGAA CCAGATGCAC TATTACTAAT AGCTGGAGGA
 301 AATTTTGAAG ATCAGCTTAG AGAAGAAGTG GTCCAGAGAG TTTCTCTTCT
 351 CCTTCTCTAT TACATTATTC ATCAGGAAGA GATCTGTTCT TCAAAGCTCA
 401 ACATGAGTAA TAAAGAGTAT AAATTTTACC TACACAGCCT ACTGAGCCTC
 451 AGGCAGGATG AAGATTCCTC TTTCCTTTCA CAGAATGAGA CAGAAGATAT
 501 CTTGGCTTTC ACCAGGCAGT ACTTTGACAC TTCTCAAAGC CAGTGTATGG
 551 AAACCAAAAC GCTGCAGAAA AAATCTGGAA TAGTGAGCAG TGAAGGTGCT
 601 AATGAAAGTA CGCTTCCTCA GTTGGCAGCC ATGATCATTA CTTTGTCCCT
 651 CCAGGGTGTT TGTCTGGGAC AAGGAAACTT GCCTTCCCCA GACTACTTTA
 701 CAGAATATAT TTTCAGTTCC TTGAATCGTA CGAATACACT CCGCCTATCA
 751 GAACTAGACC AACTCCTCAA CACTCTCTGG ACCAGAAGTA CTTGTATCAA
 801 AAATGAGAAA ATCCATCAAT TTCAAAGGAA ACAAAAACAC ATAATAACCC
 851 ATGATCAGGA CTATTCTAAT TTCTCTTCAT CCATGGAAAA AGAGTCTGAG
 901 GATGGTCCAA TTTCCTGGGA TCAGACCTGC TTCTCTGCTA GGCAGCTGGT
 951 GGAGATATTT CTACAGAAGG GCCTCTCACT CATTTCTAAG GAGGACTTTA
1001 AGCAAATGAG TCCAGGGATC ATCCAGCAGC TCCTCAGCTG CTCCTGCCAC
1051 TTACCCAAGG ACCAACAAGC AAAGCTGCCA CCTACCACTC TGGAGAAATA
1101 CGGCTACAGC ACGGTGGCTG TCACCCTTCT CACACTGGGC TCCATGCTGG
1151 GGACAGCGCT GGTCCTTTTC CATAGCTGTG AGGAGAACTA CAGGCTTATC
1201 TTACAGCTGT TTGTGGGCTT GGCCGTCGGG ACACTGTCTG GGGACGCTCT
1251 GCTCCACCTT ATCCCTCAGG TTCTTGGTTT ACATAAGCAG GAAGCCCCAG
1301 AATTTGGGCA TTTCCATGAA AGCAAAGGTC ATATTTGGAA ACTGATGGGA
1351 TTAATTGGAG GCATCCATGG ATTTTTCTTG ATAGAAAAAT GTTTTATTCT
1401 TCTTGTATCA CCAAATGACA AGCAGGGCCT GTCATTGGTT AATGGGCACG
1451 TGGGTCATTC CCACCATCTT GCACTCAACT CTGAATTAAG TGACCAGGCA
1501 GGCAGAGGCA AATCTGCTTC AACTATCCAG TTGAAAAGCC CAGAAGATTC
1551 ACAGGCAGCT GAAATGCCTA TAGGCAGTAT GACAGCCTCC AACAGAAAAT
1601 GTAAAGCCAT TAGCTTGTTA GCAATCATGA TTCTGGTTGG GGACAGCCTG
1651 CATAATTTTG CAGATGGCCT AGCCATAGGA GCAGCCTTCT CATCATCATC
1701 CGAGTCAGGA GTGACCACTA CGATTGCTAT CTTGTGTCAT GAAATCCCAC
1751 ATGAAATGGG AGACTTTGCC GTGCTCTTAA GCTCTGGACT TTCTATGAAG
1801 ACTGCCATCC TGATGAATTT TATAAGCTCC CTAACTGCCT TCATGGGATT
1851 ATACATTGGC CTTTCCGTGT CAGCTGATCC ATGTGTTCAA GACTGGATCT
1901 TCACAGTCAC TGCTGGGATG TTCTTATATT TATCCTTGGT TGAAATGCTT
1951 CCTGAAATGA CTCATGTTCA AACACAACGA CCCTGGATGA TGTTTCTCCT
2001 GCAAAACTTT GGATTGATCC TAGGTTGGCT TTCTCTCCTG CTCTTGGCTA
2051 TATATGAGCA AAATATTAAA ATATAA
```

## Fig. 6 : B) SEQ ID NO: 8, coding nucleotide sequence of human SLC39A12 sv2 cDNA

**Length: 2073 bp**

```
   1 ATGTGCTTCC GGACAAAGCT CTCAGTATCC TGGGTGCCAT TGTTTCTTCT
  51 ACTCAGCCGT GTTTTTTCTA CTGAGACAGA CAAACCCTCA GCCCAGGATA
 101 GCAGAAGCCG TGGGAGTTCA GGCCAACCGG CAGACCTGCT ACAGGTTCTC
 151 TCTGCTGGTG ACCACCCACC CCACAACCAC TCAAGAAGCC TCATCAAAAC
 201 ATTGTTGGAG AAAACTGGGT GCCCACGGAG GAGAAACGGA ATGCAAGGAG
 251 ATTGCAATCT GTGCTTTGAA CCAGATGCAC TATTACTAAT AGCTGGAGGA
 301 AATTTTGAAG ATCAGCTTAG AGAAGAAGTG GTCCAGAGAG TTTCTCTTCT
 351 CCTTCTCTAT TACATTATTC ATCAGGAAGA GATCTGTTCT TCAAAGCTCA
 401 ACATGAGTAA TAAAGAGTAT AAATTTTACC TACACAGCCT ACTGAGCCTC
 451 AGGCAGGATG AAGATTCCTC TTTCCTTTCA CAGAATGAGA CAGAAGATAT
 501 CTTGGCTTTC ACCAGGCAGT ACTTTGACAC TTCTCAAAGC CAGTGTATGG
 551 AAACCAAAAC GCTGCAGAAA AAATCTGGAA TAGTGAGCAG TGAAGGTGCT
 601 AATGAAAGTA CGCTTCCTCA GTTGGCAGCC ATGATCATTA CTTTGTCCCT
 651 CCAGGGTGTT TGTCTGGGAC AAGGAAACTT GCCTTCCCCA GACTACTTTA
 701 CAGAATATAT TTTCAGTTCC TTGAATCGTA CGAATACCCT CCGCCTATCA
 751 GAACTAGACC AACTCCTCAA CACTCTCTGG ACCAGAAGTA CTTGTATCAA
 801 AAATGAGAAA ATCCATCAAT TTCAAAGGAA ACAAAACAAC ATAATAACCC
 851 ATGATCAGGA CTATTCTAAT TTCTCTTCAT CCATGGAAAA AGAGTCTGAG
 901 GATGGTCCAG TTTCCTGGGA TCAGACCTGC TTCTCTGCTA GGCAGCTGGT
 951 GGAGATATTT CTACAGAAGG GCCTCTCACT CATTTCTAAG GAGGACTTTA
1001 AGCAAATGAG TCCAGGGATC ATCCAGCAGC TCCTCAGCTG CTCCTGCCAC
1051 TTACCCAAGG ACCAACAAGC AAAGCTGCCA CCTACCACTC TGGAGAAATA
1101 CGGCTACAGC ACGGTGGCTG TCACCCTTCT CACACTGGGC TCCATGCTGG
1151 GGACAGCGCT GGTCCTTTTC CATAGCTGTG AGGAGAACTA CAGGCTTATC
1201 TTACAGCTGT TTGTGGGCTT GGCCGTCGGG ACACTGTCTG GGGACGCTCT
1251 GCTCCACCTT ATCCCTCAGG TTCTTGGTTT ACATAAGCAG GAAGCCCCAG
1301 AATTTGGGCA TTTCCATGAA AGCAAAGGTC ATATTTGGAA ACTGATGGGA
1351 TTAATTGGAG GCATCCATGG ATTTTTCTTG ATAGAAAAAT GTTTTATTCT
1401 TCTTGTATCA CCAAATGACA AGGGCCTGTC ATTGGTTAAT GGGCACGTGG
1451 GTCATTCCCA CCATCTTGCA CTCAACTCTG AATTAAGTGA CCAGGCAGGC
1501 AGAGGCAAAT CTGCTTCAAC TATCCAGTTG AAAAGCCCAG AAGATTCACA
1551 GGCAGCTGAA ATGCCTATAG GCAGTATGAC AGCCTCCAAC AGAAAATGTA
1601 AAGCCATTAG CTTGTTAGCA ATCATGATTC TGGTTGGGGA CAGCCTGCAT
1651 AATTTTGCAG ATGGCCTAGC CATAGGAGCA GCCTTCTCAT CATCATCCGA
1701 GTCAGGAGTG ACCACTACGA TTGCTATCTT GTGTCATGAA ATCCCACATG
1751 AAATGGGAGA CTTTGCCGTG CTCTTAAGCT CTGGACTTTC TATGAAGACT
1801 GCCATCCTGA TGAATTTTAT AAGCTCCCTA ACTGCCTTCA TGGGATTATA
1851 CATTGGCCTT TCCGTGTCAG CTGATCCATG TGTTCAAGAC TGGATCTTCA
1901 CAGTCACTGC TGGGATGTTC TTATATTTAT CCTTGGTTGA AATGCTTCCT
1951 GAAATGACTC ATGTTCAAAC ACAACGACCC TGGATGATGT TTCTCCTGCA
2001 AAACTTTGGA TTGATCCTAG GTTGGCTTTC TCTCCTGCTC TTGGCTATAT
2051 ATGAGCAAAA TATTAAAATA TAA
```

# Fig. 6 : C) SEQ ID NO: 9, coding nucleotide sequence of human SLC39A12 sv3 cDNA

**Length: 1965 bp**

```
   1  ATGTGCTTCC GGACAAAGCT CTCAGTATCC TGGGTGCCAT TGTTTCTTCT
  51  ACTCAGCCGT GTTTTTTCTA CTGAGACAGA CAAACCCTCA GCCCAGGATA
 101  GCAGAAGCCG TGGGAGTTCA GGCCAACCGG CAGACCTGCT ACAGGTTCTC
 151  TCTGCTGGTG ACCACCCACC CCACAACCAC TCAAGAAGCC TCATCAAAAC
 201  ATTGTTGGAG AAAACTGGGT GCCCACGGAG GAGAAACGGA ATGCAAGGAG
 251  ATTGCAATCT GTGCTTTGAA CCAGATGCAC TATTACTAAT AGCTGGAGGA
 301  AATTTTGAAG ATCAGCTTAG AGAAGAAGTG GTCCAGAGAG TTTCTCTTCT
 351  CCTTCTCTAT TACATTATTC ATCAGGAAGA GATCTGTTCT TCAAAGCTCA
 401  ACATGAGTAA TAAAGAGTAT AAATTTTACC TACACAGCCT ACTGAGCCTC
 451  AGGCAGGATG AAGATTCCTC TTTCCTTTCA CAGAATGAGA CAGAAGATAT
 501  CTTGGCTTTC ACCAGGCAGT ACTTTGACAC TTCTCAAAGC CAGTGTATGG
 551  AAACCAAAAC GCTGCAGAAA AAATCTGGAA TAGTGAGCAG TGAAGGTGCT
 601  AATGAAAGTA CGCTTCCTCA GTTGGCAGCC ATGATCATTA CTTTGTCCCT
 651  CCAGGGTGTT TGTCTGGGAC AAGGAAACTT GCCTTCCCCA GACTACTTTA
 701  CAGAATATAT TTTCAGTTCC TTGAATCGTA CGAATACCCT CCGCCTATCA
 751  GAACTAGACC AACTCCTCAA CACTCTCTGG ACCAGAAGTA CTTGTATCAA
 801  AAATGAGAAA ATCCATCAAT TTCAAAGGAA ACAAAACAAC ATAATAACCC
 851  ATGATCAGGA CTATTCTAAT TTCTCTTCAT CCATGGAAAA AGAGTCTGAG
 901  GATGGTCCAG TTTCCTGGGA TCAGACCTGC TTCTCTGCTA GGCAGCTGGT
 951  GGAGATATTT CTACAGAAGG GCCTCTCACT CATTTCTAAG GAGGACTTTA
1001  AGCAAATGAG TCCAGGGATC ATCCAGCAGC TCCTCAGCTG CTCCTGCCAC
1051  TTACCCAAGG ACCAACAAGC AAAGCTGCCA CCTACCACTC TGGAGAAATA
1101  CGGCTACAGC ACGGTGGCTG TCACCCTTCT CACACTGGGC TCCATGCTGG
1151  GGACAGCGCT GGTCCTTTTC CATAGCTGTG AGGAGAACTA CAGGCTTATC
1201  TTACAGCTGT TTGTGGGCTT GGCCGTCGGG ACACTGTCTG GGGACGCTCT
1251  GCTCCACCTT ATCCCTCAGG TTCTTGGTTT ACATAAGCAG GAAGCCCCAG
1301  AATTTGGGCA TTTCCATGAA AGCAAAGGTC ATATTTGGAA ACTGATGGGA
1351  TTAATTGGAG GCATCCATGG ATTTTTCTTG ATAGAAAAAT GTTTTATTCT
1401  TCTTGTATCA CCAAATGACA AGAAAAGCCC AGAAGATTCA CAGGCAGCTG
1451  AAATGCCTAT AGGCAGTATG ACAGCCTCCA ACAGAAAATG TAAAGCCATT
1501  AGCTTGTTAG CAATCATGAT TCTGGTTGGG GACAGCCTGC ATAATTTTGC
1551  AGATGGCCTA GCCATAGGAG CAGCCTTCTC ATCATCATCC GAGTCAGGAG
1601  TGACCACTAC GATTGCTATC TTGTGTCATG AAATCCCACA TGAAATGGGA
1651  GACTTTGCCG TGCTCTTAAG CTCTGGACTT TCTATGAAGA CTGCCATCCT
1701  GATGAATTTT ATAAGCTCCC TAACTGCCTT CATGGGATTA TACATTGGCC
1751  TTTCCGTGTC AGCTGATCCA TGTGTTCAAG ACTGGATCTT CACAGTCACT
1801  GCTGGGATGT TCTTATATTT ATCCTTGGTT GAAATGCTTC CTGAAATGAC
1851  TCATGTTCAA ACACAACGAC CCTGGATGAT GTTTCTCCTG CAAAACTTTG
1901  GATTGATCCT AGGTTGGCTT TCTCTCCTGC TCTTGGCTAT ATATGAGCAA
1951  AATATTAAAA TATAA
```

**Fig. 7:** **Alignment of SLC39A12 RT-PCR primers SEQ ID NO. 10 and SEQ ID NO. 11 with human SLC39A12 sv1 cDNA, SEQ ID NO: 4**

```
SEQ ID NO:10 (PRIMER A)        1 GGATTATACATTGGCCTTTCCG 22
                                 ||||||||||||||||||||||
SEQ ID NO:4                 1995 GGATTATACATTGGCCTTTCCG  2016


SEQ ID NO:11 (PRIMER B)       20 CACAACGACCCTGGATGATG 1
                                 ||||||||||||||||||||
SEQ ID NO:4                 2122 CACAACGACCCTGGATGATG 2141
```

Fig. 8: **Schematic alignment of SEQ ID NO: 4, SEQ ID NO: 7, and RT-PCR primer sequences of SLC39A12**

```
SEQ ID NO:10                                             +>                 1995-2016
SEQ ID NO:11                                              <+                2122-2141
SEQ ID NO:7    +-------------------------------------------->                150-2225
SEQ ID NO:4   +-------------------------------------------------------->      1-2678

              |------|------|------|------|------|------|------|------|
              0     400    800    1200   1600   2000   2400   2600   2800
```

Legend:
- `+` 5'-end of sequence
- `>` 3'-end of sequence
- `---` sequence identical to SLC39A12 sv 1 mRNA

## Fig. 9: Westernblot analysis of SLC39A12 antibody HKQ1

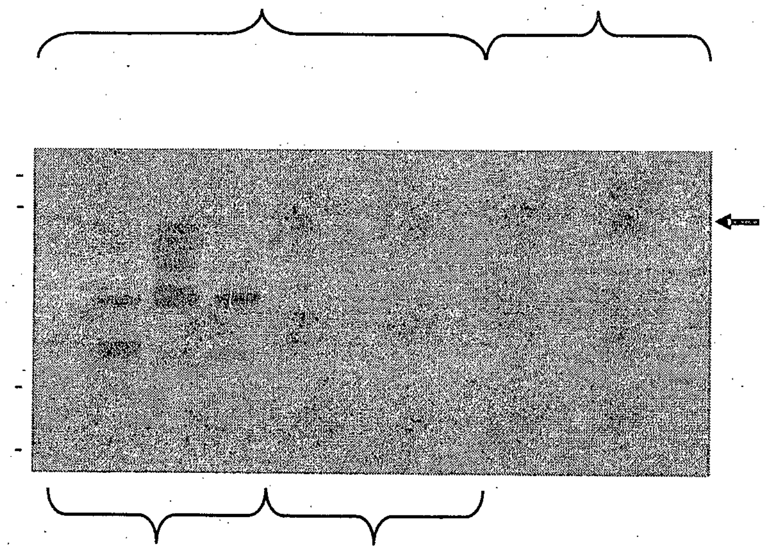

Legend:

M: molecular weight marker.
Lane 1 – 3 probed with HKQ1 (1:3000):
Human brain extract (1), H4+SLC39A12 (2), H4 negative control (3)
Lane 4 – 6 probed with HKQ1 (1:3000) with peptide preincubation:
Human brain extract (4), H4+SLC39A12 (5), H4 negative control (6)
Lane 7- 9 probed with anti-myc antibody (1:3000):
Human brain extract (7), H4+SLC39A12 (8), H4 negative control (9)

Fig. 10: Immunofluorescence analysis of SLC39A12 antibody HKQ1

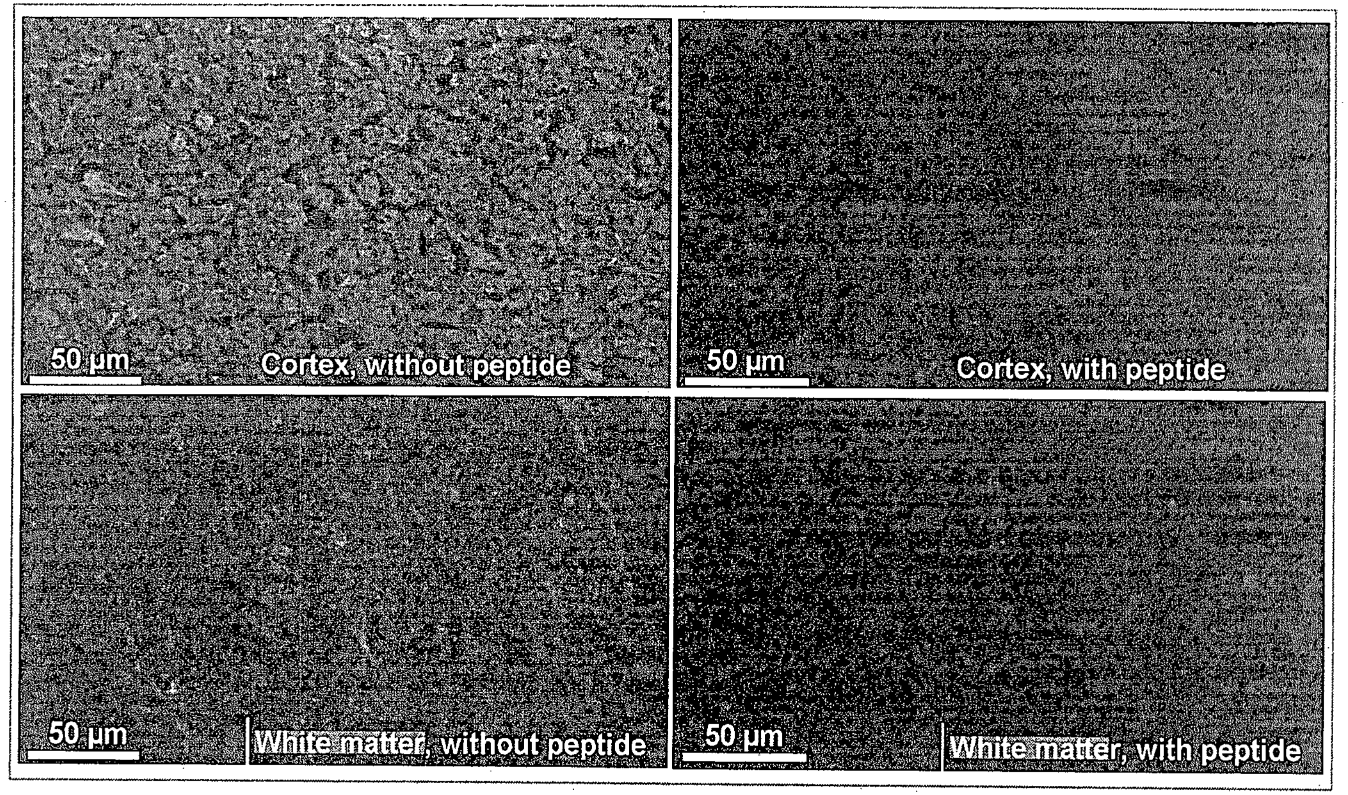

Fig. 11: A) Expression of SLC39A12 protein is increased in the cerebral cortex of AD patients

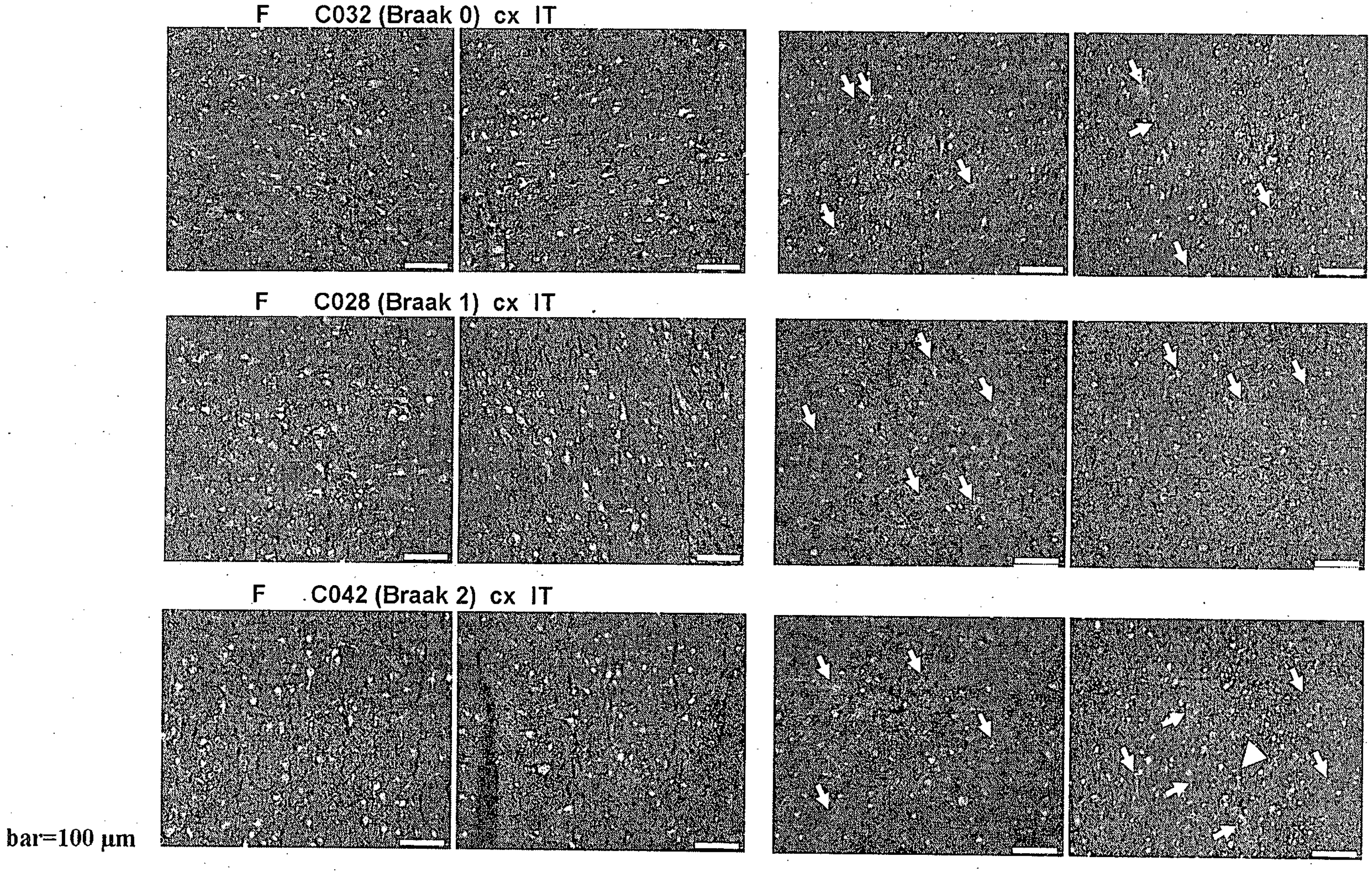

Fig. 11: B) Expression of SLC39A12 protein is increased in the cerebral white matter of AD patients

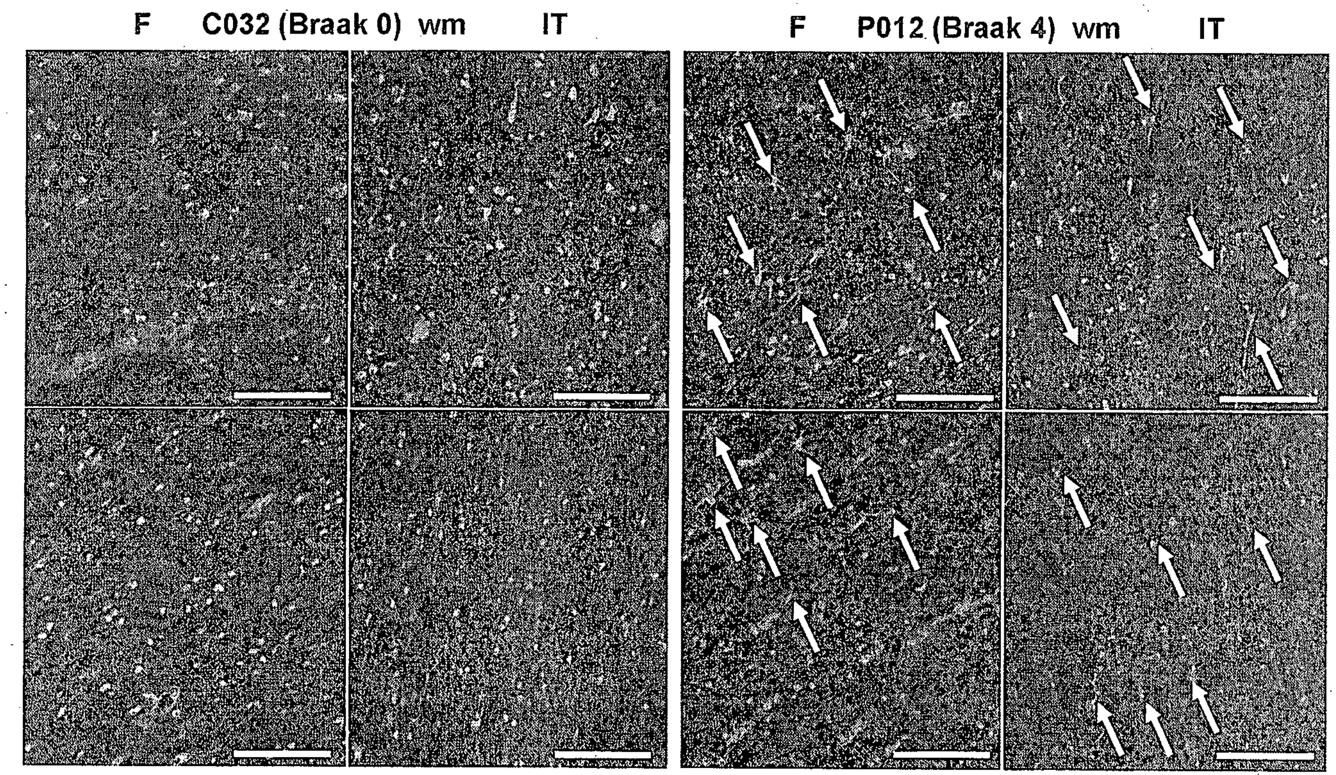


bar=100 µm

Fig. 12: Western blot analysis of SLC39A12 expression in different transgenic fly lines

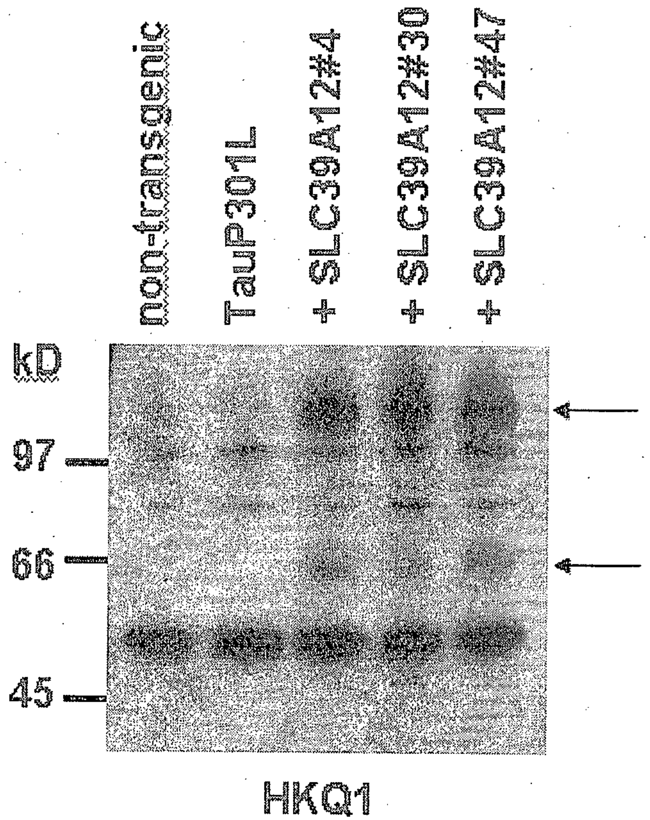

# Fig. 13: LightCycler analysis of SLC39A12 expression in UAS-TauP301L/UAS-SLC39A12 double transgenic flies

| name | SLC39A12 cycle # | SLC39A12 mean | SLC39A12 stdev | rp49 cycle # | rp49 mean | rp49 stdv | rp49 factor | mean SLC39A12 x factor rp49 | difference | expression normalized to housekeeping gene (rp49) and efficiency (E) of SLC39A12 primer |
|---|---|---|---|---|---|---|---|---|---|---|
| Tau P301L | 35.55 | 35.173 | 0.3326 | 20.5 | 20.550 | 0.0557 | 1 | 35.1733333 | 0.000 | 1.00 |
| Tau P301L | 34.92 | | | 20.61 | | | | | | |
| Tau P301L | 35.05 | | | 20.54 | | | | | | |
| neg. control (H2O) | 37.21 | | | 34.56 | | | | | | |
| + SLC39A12#4 | 25.15 | 25.170 | 0.0283 | 21.5 | 21.600 | 0.0917 | 1.05109489 | 26.4560584 | 8.717 | 160.03 |
| + SLC39A12#4 | 25.19 | | | 21.68 | | | | | | |
| + SLC39A12#4 | | | | 21.62 | | | | | | |
| | | | | | | | | | | |
| + SLC39A12#30 | 26.46 | 26.493 | 0.0351 | 20.97 | 21.123 | 0.1457 | 1.02789943 | 27.2324823 | 7.94085104 | 101.83 |
| + SLC39A12#30 | 26.49 | | | 21.14 | | | | | | |
| + SLC39A12#30 | 26.53 | | | 21.26 | | | | | | |
| | | | | | | | | | | |
| + SLC39A12#47 | 25.1 | 25.153 | 0.0473 | 21.11 | 21.150 | 0.06082763 | 1.02919708 | 25.8877372 | 9.28559611 | 222.79 |
| + SLC39A12#47 | 25.17 | | | 21.22 | | | | | | |
| + SLC39A12#47 | 25.19 | | | 21.12 | | | | | | |

$E = 10^{(-1/slope)}$ slope= -3,953 E=1,79

Fig. 14: Tau phosphorylation at Ser214 is significantly increased in transgenic fly lines co-expressing SLC39A12

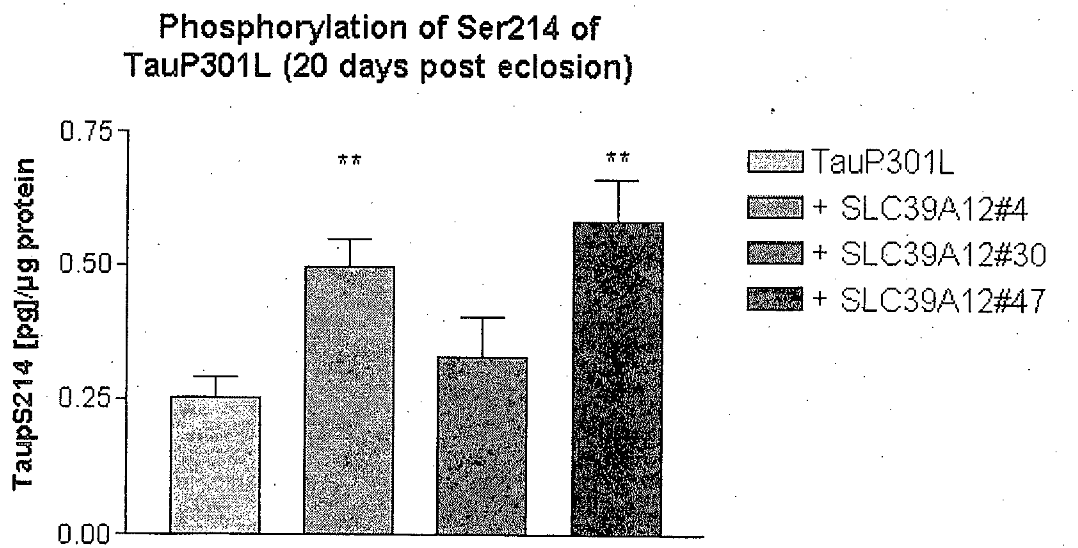

**Fig. 15: Westernblot analysis of SLC39A12 expression in H4APPsw cells stably expressing SLC39A12**

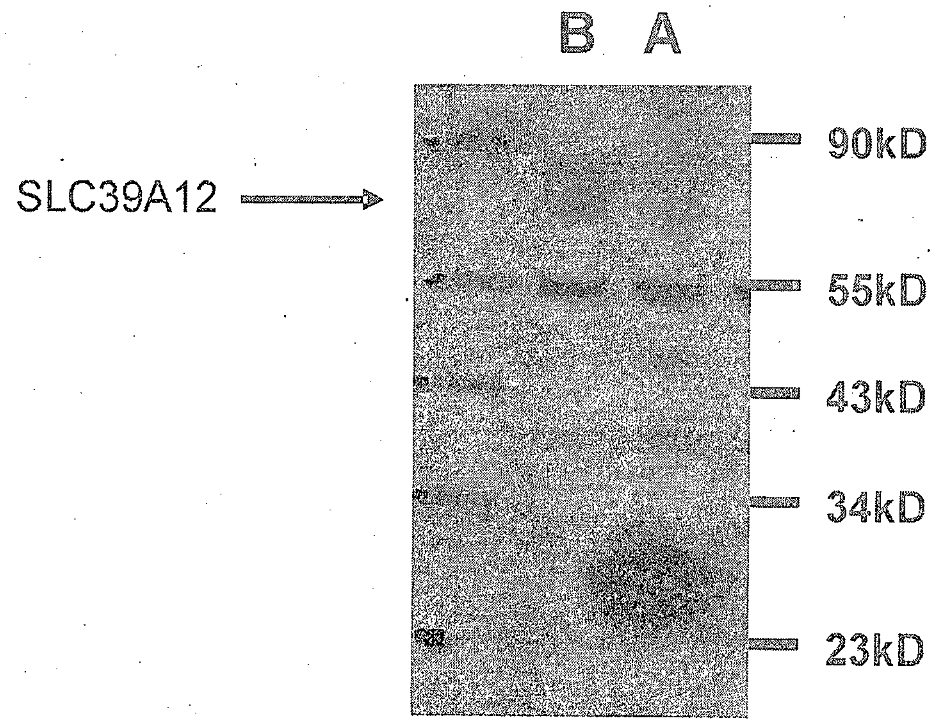


A: H4 APPsw-SLC39A12-myc
B: H4 APPsw (control)

**Fig. 16: Immunofluorescence of SLC39A12 expression in H4APPsw cells stably expressing SLC39A12**

overlay Myc/Cy DAPI

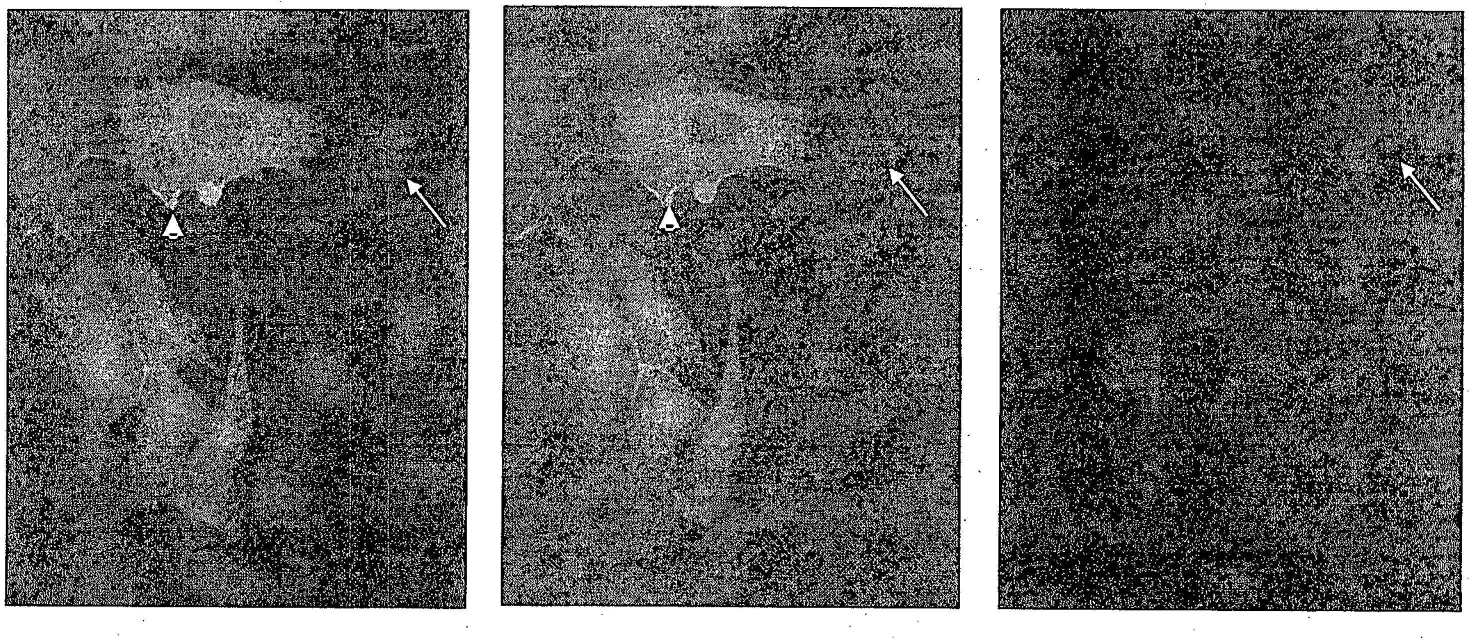

Arrow indicates nucleus of a cell without SLC39A12-overexpression as a negative control.
Arrowhead indicates expression at the plasmamembrane.

Fig. 17: Zinc uptake assay verified the function of SLC39A12 as a zinc transporter

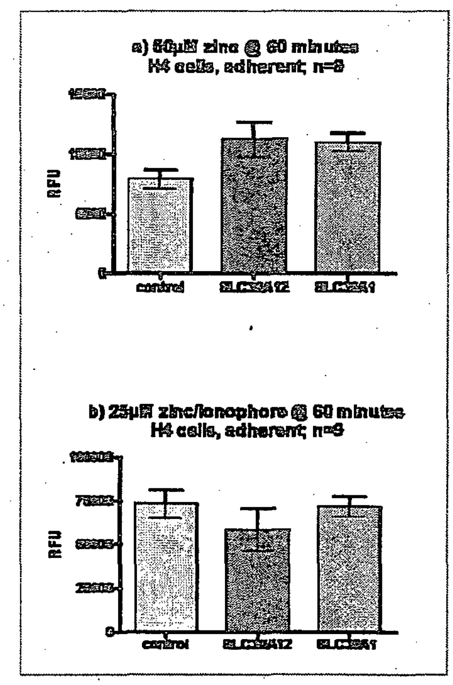

| Cell line | 50 µM zinc | | 25µM zinc/ionophore | |
|---|---|---|---|---|
| | P value | significant | P value | significant |
| H4 SLC39A12 | 0.0364 | * | 0.3350 | ns |
| H4 SLC39A1 | 0.0079 | ** | 0.8840 | ns |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004023973 A
- EP 1293569 A
- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- US 6150173 A


### Non-patent literature cited in the description


- **VICKERS et al.** *Progress in Neurobiology,* 2000, vol. 60, 139-165
- **WALSH ; SELKOE.** *Neuron,* 2004, vol. 44, 181-193
- **SELKOE ; KOPAN.** *Annu Rev Neurosci,* 2003, vol. 26, 565-597
- **LING et al.** *Int J Biochem Cell Biol,* 2003, vol. 35, 1505-1535
- **BRAAK ; BRAAK.** *J Neural Transm,* 1998, vol. 53, 127-140
- **JOHNSON ; JENKINS.** *J Alzheimers Dis,* 1996, vol. 1, 38-58
- **JOHNSON ; HARTIGAN.** *J Alzheimers Dis,* 1999, vol. 1, 329-351
- **SCHMITT et al.** *Neurology,* 2000, vol. 55, 370-376
- **TERRY et al.** Annals of Neurology. 1981, vol. 10, 184-92
- **STRITTMATTER et al.** *Proc Natl Acad Sci USA,* 1993, vol. 90, 1977-81
- **ROSES.** *Ann NYAcad Sci,* 1998, vol. 855, 738-43
- **PAVEL L. KATSEL et al.** Large-Scale Microarray Studies of Gene Expression in Multiple Regions of the Brain in Schizophrenia and Alzheimer's Disease. *International Review of Neurobiology,* 2005, vol. 63, 41-82
- **LAURA SHAUGHNESSY et al.** *Journal of Molecular Neuroscience,* 01 August 2004, vol. 24 (1), 23-32
- **FREDERICKSON et al.** *Nature Rev Neurosci.,* 2005
- **SLOMIANKA et al.** *Neuroscience,* 1990, vol. 38, 843-854
- **FREDERICKSON ; BUSH.** *BioMetals,* 2001, vol. 14, 353-366
- **LIUZZI ; COUSINS.** *Annu. Rev. Nutr.,* 2004, vol. 24, 151-172
- **HEDIGER et al.** *Plugers Arch.,* 2004, vol. 447, 465-468
- **EIDE.** *Plugers Arch.,* 2004, vol. 447, 796-800
- **TAYLOR ; NICHOLSON.** *Biochim. Biophys. Acta,* 2003, vol. 1611, 16-30
- **STRAUSBERG et al.** *Proc. Natl. Acad. Sci. U.S.A.,* 2002, vol. 99, 16899-16903
- **HUBBARD et al.** *Ensembl 2005, Nucleic Acids Res.,* 2005, vol. 33, D447-53
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **DEVEREUX et al.** *Nucleic Acids Res.,* 1984, vol. 12, 387
- **PEARSON ; LIPMAN.** *Proc. Natl. Acad. Sci. USA,* 1988, vol. 85, 2444-2448
- **WILBUR ; LIPMAN.** *SIAM J. Appl. Math.,* 1984, vol. 44, 557-567
- **NEEDLEMAN ; WUNSCH.** *J. Mol. Biol.,* 1970, vol. 48, 443-453
- **IQBAL ; SWAAB ; WINBLAD ; WISNIEWSKI.** Alzheimer's Disease and Related Disorders. Wiley & Sons, 1999
- **SCINTO ; DAFFNER.** Early Diagnosis of Alzheimer's Disease. Humana Press, 2000
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **BRAAK ; BRAAK.** *Acta Neuropathology,* 1991, vol. 82, 239-259
- **BANCHER et al.** *Neuroscience Letters,* 1993, vol. 162, 179-182
- **GOLD et al.** *Acta Neuropathol,* 2000, vol. 99, 579-582

- **RÖSSLER et al.** *Acta Neuropathol,* 2002, vol. 103, 363-369
- **GIANNAKOPOULOS et al.** *Neurology,* 2003, vol. 60, 1495-1500
- **BENNETT et al.** *Arch Neurol,* 2004, vol. 61, 378-384
- **METSAARS et al.** *Neurobiol Aging,* 2003, vol. 24, 563-572
- **TERRY et al.** Annals of Neurology. 1981, vol. 10, 184-192
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Antibodies: A Laboratory Manual. Cold Spring Harbor Laboratory Press, 1999
- **EDWARDS R.** Immunodiagnostics: A Practical Approach. Oxford University Press
- **BEHR.** *Acc Chem Res,* 1993, vol. 26, 274-278
- **MULLIGAN.** *Science,* 1993, vol. 260, 926-931
- **WOLFF.** *Curr Opin Neurobiol,* 1993, vol. 3, 743-748
- **GILLESPIE.** *DN&P,* 1992, vol. 5, 389-395
- **AGRAWAL ; AKHTAR.** *Trends Biotechno!,* 1995, vol. 13, 197-199
- **CROOKE.** *Biotechnology,* 1992, vol. 10, 882-6
- **BARINAGA.** *Science,* 1993, vol. 262, 1512-1514
- **WICKSTROM.** *Trends Biotechnol,* 1992, vol. 10, 281-287
- **HANNON.** *Nature,* 2002, vol. 418, 244-251
- **MC CELLAND ; PARDEE.** Expression Genetics: Accelerated and High-Throughput Methods. Eaton Publishing, 1999
- **LANZA et al.** *Nature Medicine,* 1999, vol. 9, 975-977
- **PETERSON DA.** *Curr. Opin. Pharmacol.,* 2002, vol. 2, 34-42
- **COLMAN A.** *Drug Discovery World,* 2001, vol. 7, 66-71
- **CAPECCHI.** *Science,* 1989, vol. 244, 1288-1292
- **HOGAN et al.** Manipulating the Mouse Embryo: A Laboratory Manual. Cold Spring Harbor Laboratory Press, 1994
- **JACKSON ; ABBOTT.** Mouse Genetics and Transgenics: A Practical Approach. Oxford University Press, 1999
- **SCHWARZ et al.** *Biochemistry,* 1999, vol. 38, 9456-9464
- **KRIVOSHEEV ; USANOV.** *Biochemistry-Moscow,* 1997, vol. 62, 1064-1073
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **SACHS L.** *Statistische Methoden: Planung und Auswertung,* 1988, 60
- **GREEVE et al.** *J. Neurosci.,* 2004, vol. 24, 3899-3906
- **BRAND ; PERRIMON.** *Development,* 1993, vol. 118, 401-15
- **JÜRGEN GOETZ, GÖTZ et al.** *Science,* 2001, vol. 293 (5534), 1491-1495
- **RUBIN ; SPRADLING.** *Science,* 1982, vol. 218, 348-53
- **SPRADLING ; RUBIN.** *Science,* 1982, vol. 218, 341-7
- **FOSSGREEN et al.** *Proc Natl Acad Sci USA,* 1998, vol. 95, 13703-8
- **YE ; FORTINI.** *J Cell Biol,* 1999, vol. 146, 1351-64